# EUROPÄISCHE PATENTANMELDUNG

(11) **EP 2 363 494 A1**
(43) Veröffentlichungstag der Anmeldung: **07.09.2011**
(21) Anmeldenummer: 11001260.6
(22) Anmeldetag: 24.11.2008
(51) Int. Cl.: C12N 15/87

(54) **Verbesserung von Transfektionsergebnissen nicht-viraler Genliefersysteme durch Beeinflussung des angeborenen Immunsystems**

(30) Priorität: 22.11.2007 DE 102007056488; 28.03.2008 DE 102008016275; 16.05.2008 DE 102008023913
(62) Teilanmeldung aus: 08851432.8
(71) Anmelder: Biontex Laboratories Gmbh, 82152 Martinsried/Planegg (DE)
(72) Erfinder: Klösel, Roland, 80995 München (DE); König, Stefan, 81745 München (DE)
(74) Vertreter: Forstmeyer, Dietmar

(57) **Zusammenfassung**

Das angeborene Immunsystem von Eukaryoten ist in der Lage, über Toll Like Rezeptoren fremdes genetisches Material zu erkennen und Signaltransduktionskaskaden anzustoßen, die über eine Interferonantwort einen antiviralen Zustand von Zellpopulationen auslösen. Dieser antivirale Zustand ist auch eine Barriere für nicht-virale Genliefersysteme. Wird die Signaltransduktionskaskade intrazellulär oder interzellulär unterbrochen lassen sich Transfektionseffizienzen von nicht-viralen Genliefersystemen steigern und unerwünschte Änderungen des Expressionsprofils vermeiden. Da die RNA Interferenz zum antiviralen Zustand zuzurechnen ist, ist die RNAi-Maschinerie nach einer Aktivierung des angeborenen Immunsystems ebenfalls aktiviert. Auf diese Weise lassen sich Knock-downeffizienzen bei der Transfektion mit siRNA erhöhen.

SEQUENCE LISTING

<110>  Biontex Laboratories GmbH

<120>  Verbesserung von Transfektionsergebnissen nicht-viraler
       Genliefersysteme durch Beeinflussung des angeborenen Immunsystems

<130>  18327-PCT

<140>  PCT
<141>  2008-11-22

<160>  5

<170>  PatentIn version 3.3

<210>  1
<211>  9
<212>  RNA
<213>  Homo sapiens

<400>  1
guccuucaa                                                              9


<210>  2
<211>  21
<212>  DNA
<213>  Photinus pyralis

<400>  2
cuuacgcuga guacuucgat t                                               21


<210>  3
<211>  21
<212>  DNA
<213>  Photinus pyralis

<400>  3
ucgaaguacu cagcguaagt t                                               21


<210>  4
<211>  21
<212>  DNA
<213>  unknwon organism

<400>  4
agguagugua aucgccuugt t                                               21


<210>  5
<211>  21
<212>  DNA
<213>  unknown organism

<400>  5
caaggcgauu acacuaccut t                                               21

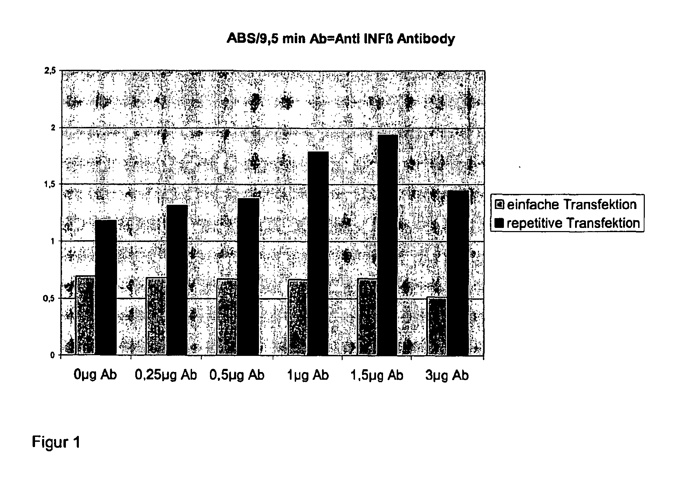

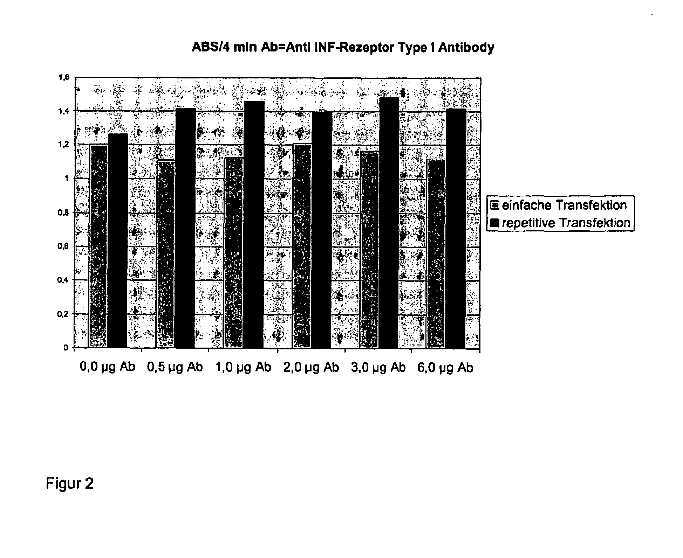

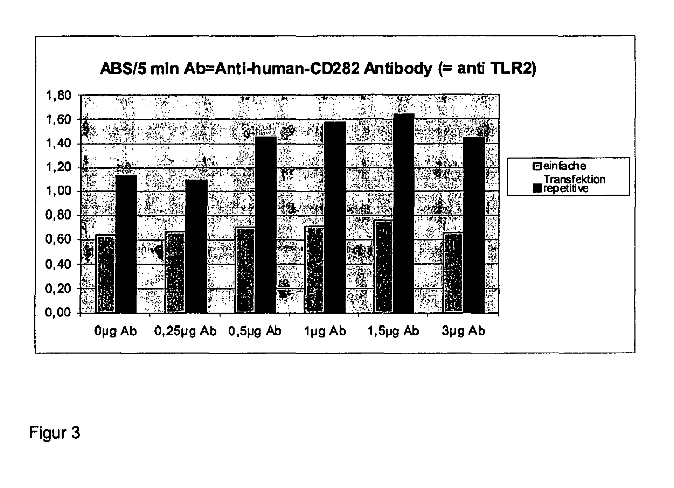

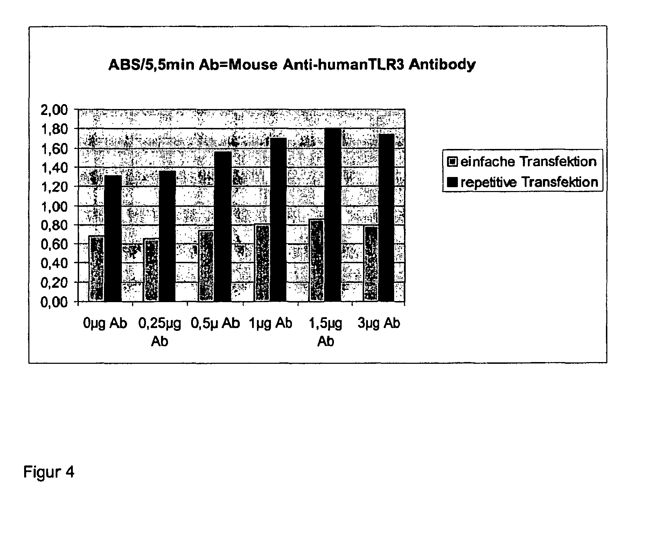

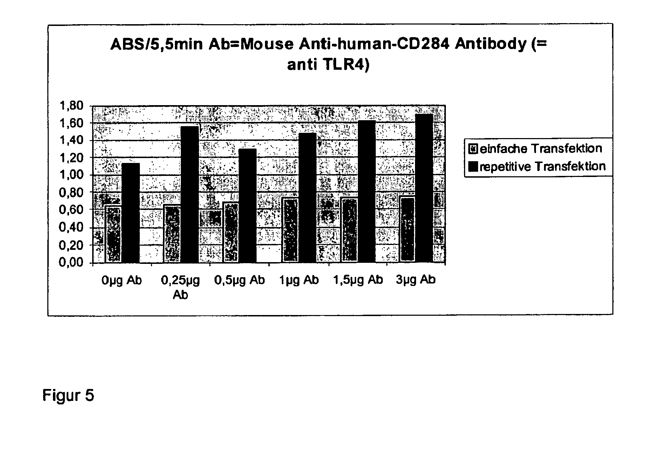

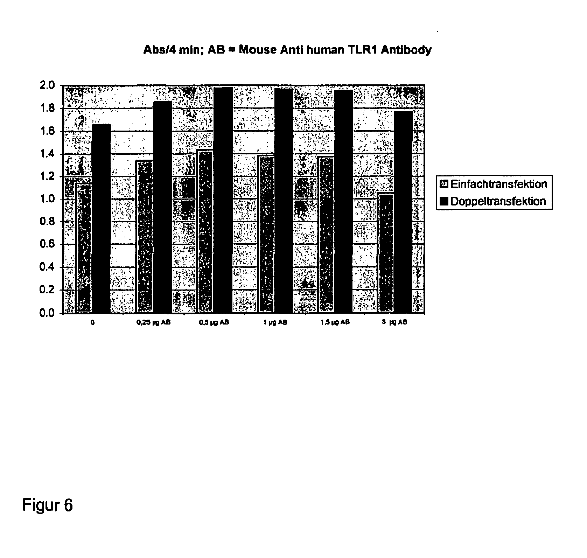

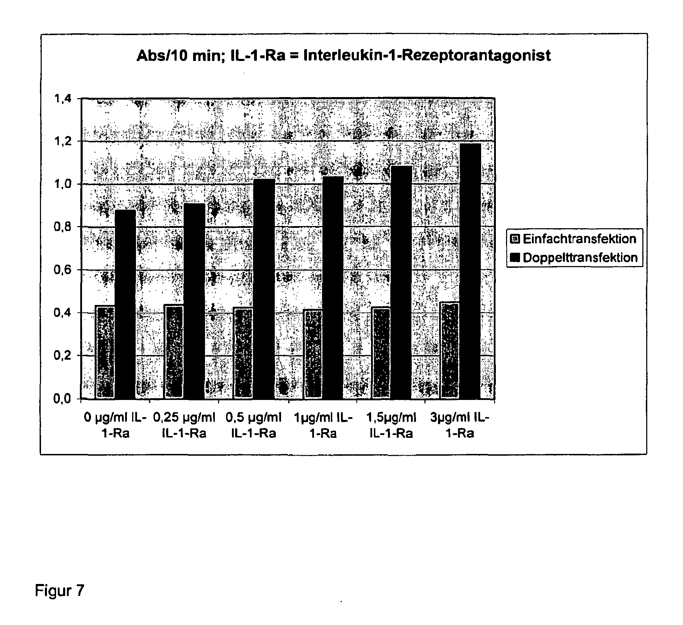

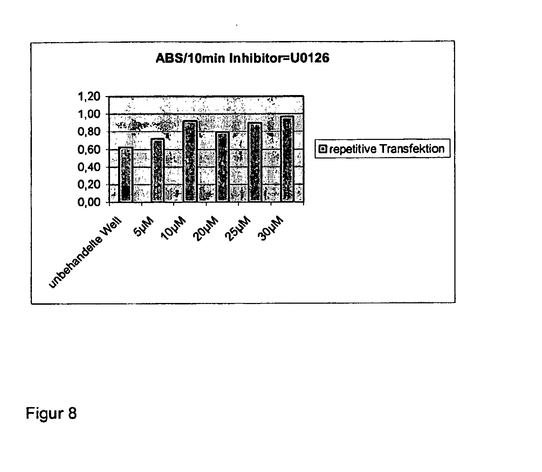

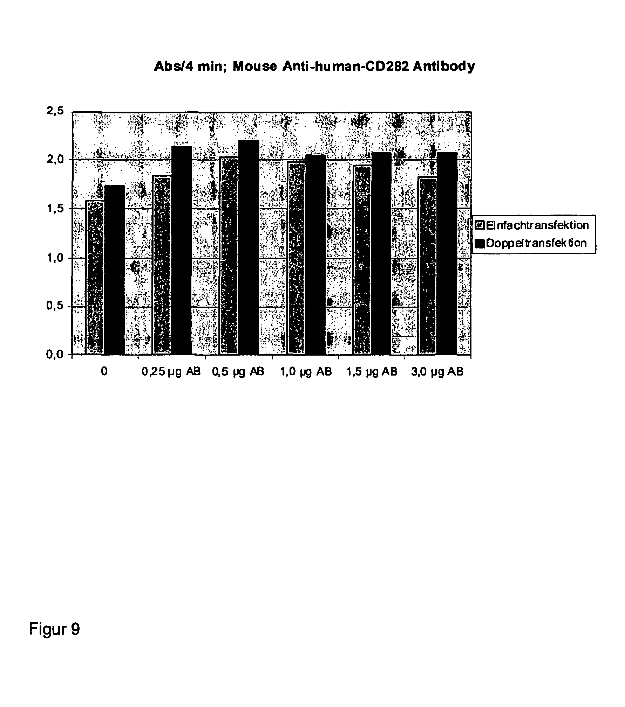

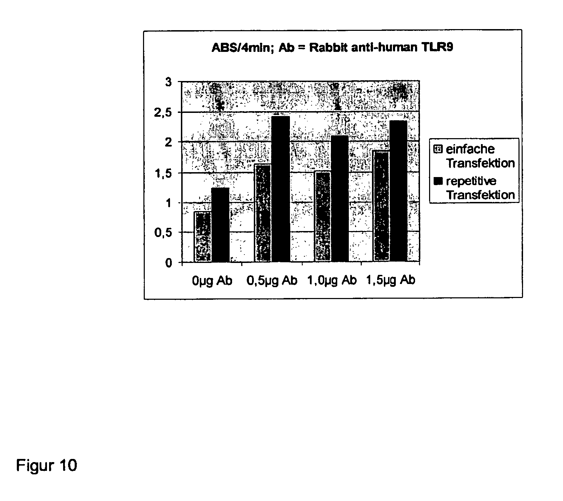

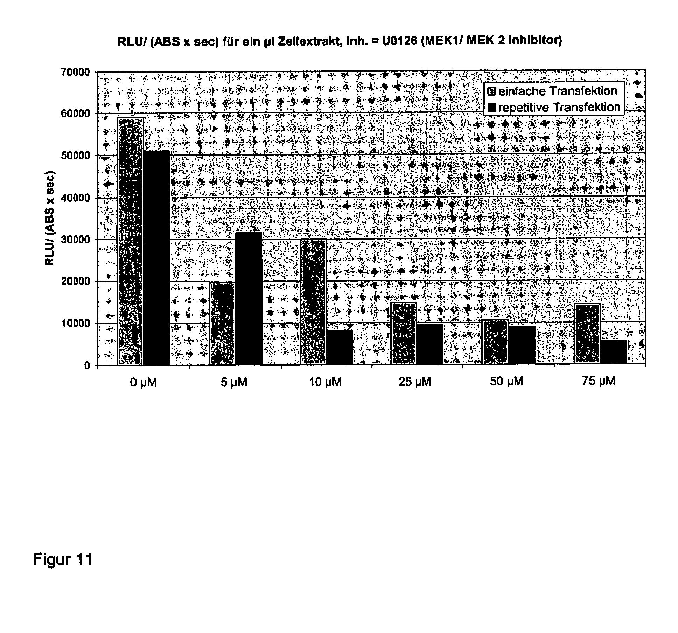

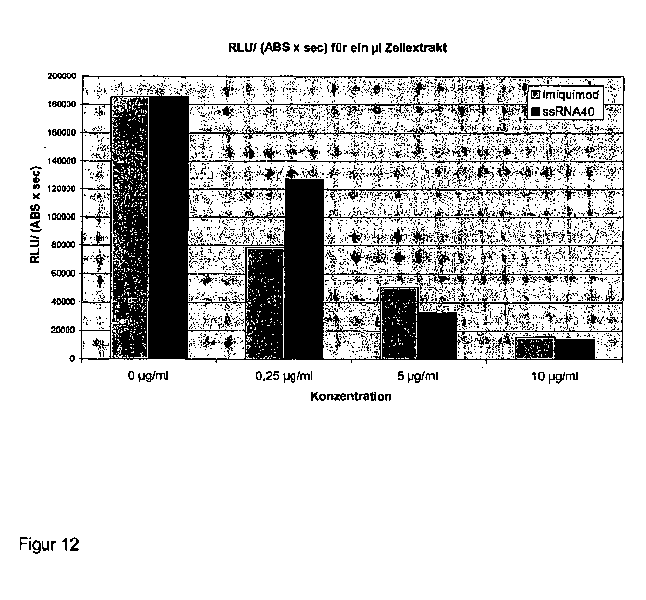

## Beschreibung

### Stand der Technik

Bei Immunantworten des Körpers (Luke A. et al.; Spektrum der Wissenschaft, August 2005, Seiten 68-75) gegen eine infektiöse oder immunologische Herausforderung unterscheidet man zwischen der angeborenen (innate immunity) und der erworbenen Immunantwort (antigen-specific acquired immunity).

Die erworbene Immunabwehr wird erst bei einer Infektion mit Krankheitserregern ausgebildet. Sie besitzt eine Art Gedächtnis, sodass es bei einer zweiten Infektion durch denselben Erreger in der Regel nicht mehr zum Ausbruch der Krankheit kommt. Auf diesem Prinzip beruhen Impfstoffe. Gäbe es nur die erworbene lmmunabwehr wäre der Organismus vor der ersten Infektion völlig ungeschützt. Das ist jedoch nicht der Fall, da es noch eine weitere sehr ursprüngliche Immunabwehr gibt, die als angeborene Immunabwehr bezeichnet wird und von der Fliege Drosophila bis zu Säugetieren, ja sogar bei Pflanzen gefunden wird.

Die angeborene Immunabwehr ist die erste Abwehrfront gegen Krankheitserreger und stellt ein evolutionär sehr altes System dar. Bei der angeborenen Immunabwehr werden über so genannte Toll-like Rezeptoren (TLRs) (Heine H. et al.; Int. Arch. Allergy Immunol. 2003; 130; 180-192 und Uematsu S. et al., J. Biol. Chem. 2007, May 25; 282 (21);15319 -23) und RIG-1-like Helikasen (RLHs) die krankheitsassozierten molekularen Muster, so genannte pathogenassociated molecular patterns (PAMPs), erkannt und entsprechende Entzündungs- sowie Immunreaktionen eingeleitet. Dadurch kann der Organismus zwischen "selbst" und "nicht selbst" unterscheiden. RLHs werden ubiquitär im Zytosol exprimiert, wo sie in der Lage sind, die bei viraler Infektion entstehende dsRNA zu erkennen. TLRs und RLHs gehören zu einer Gruppe von Rezeptoren, die auch als pattern recognition receptors (PRRs) bezeichnete werden.

### Toll-like Rezeptoren (TLRs)

Toll-like Rezeptoren wurden erstmals in der Mitte der 1990er Jahre entdeckt (Zimmer A. et al.; PNAS, 1999; 96(10), 5780-5785). Der Name ist abgeleitet von einem in Drosophila Melanogaster von Christiane Nüsslein-Volhard gefundenem Protein, das Sie "Toll" nannte. TLR-Proteine ähneln diesem Typus und werden damit als "Toll-like"-Proteine bezeichnet. Dabei handelt es sich um Transmembranproteine mit einer extrazellulären, "leucin-reichen repeat" Domäne (LRR) wie auch einer zytoplasmatischen Domäne, die derjenigen der IL-1R Familie homolog ist. Die verschiedenen TLRs reagieren selektiv auf verschiedene -molekulare virale und bakterielle Komponenten und steuern über eine Signaltransduktionskaskade eine entsprechende Aktivierung von Genen. Dies geschieht zunächst über so genannte Adaptermoleküle und darauf folgend über Kinasen, die schließlich Transkriptionsfaktoren (z.B. NF-kB und die IRF-Familien) durch Phosphorylierung derselben oder entsprechender intrazellulärer Inhibitoren dieser Transkriptionsfaktoren aktivieren. Letztendlich werden neben einer Vielzahl spezifischer Gene, die eine antimikrobielle Wirkung haben, so genannte Zytokine produziert. Zytokine sind wiederum notwendige Stimulatoren für die erworbene Immunabwehr und damit auch ein Bindeglied wischen angeborener und erworbener Immunabwehr. Die Prinzipien der Ligandenerkennung, Signaltransduktion und Signalweiterieitung sind allerdings erst in Ansätzen verstanden.

Bisher sind 13 verschiedene TLRs bekannt (davon 10 beim Menschen), deren Anzahl ausreichend ist für die Erkennung aller pathogenen Erreger, angefangen von Bakterien über Pilze bis zu den Viren. Die Rezeptoren erkennen dabei allen Erregem gemeinsame Strukturen, des Weiteren mitunter auch mehrere Bestandteile gleichzeitig, ohne dass diese sich strukturell ähneln. Beispielsweise erkennt das TLR4 Lipopolysaccharide, aber auch Taxol. Bisher ist nicht bekannt wie die TLRs das leisten können. Die TLRs unterscheiden sich von Spezies zu Spezies nur wenig.

Bisher sind folgende Gruppen von Molekülen als Liganden der TLRs bekannt, die zu einer Auslösung von Signaltransduktionskaskaden führen:
TLR1:
   bildet mit TLR2 ein Heterodimer, ist der Rezeptor von triacyliertem Lipoprotein und Zymosan aus Hefen.
TLR2: ist der Rezeptor für bestimmte Peptidoglykane, Lipopeptide, Glykolipide und verschiedener Bakterien.
TLR3:
   erkennt lange dsRNA, wie Sie bei einer Virusreplikation in infizierten Zellen vorkommt.
TLR4:
   ist der Rezeptor für Lipopolysaccharide (LPS, auch Endotoxine), verschiedene Hüllglykoproteine (auch von Viren) und Taxol. LPS sind Bestandteile von Bakterienzellwänden. Der TLR4 Rezeptor benötigt für seine Funktion ein zusätzliches membrangebundenes Protein (TLR assistierendes Protein). Dabei bindet CD14 zB. das LPS und führt es dem TLR4 Rezeptor zu. Die Bindung an CD14 alleine löst dabei keine Signaltransduktionskaskade aus.
TLR5:
   ist der Rezeptor von Flagellin, einem Hauptbestandteil der Geiseln (Flagellae), mit weichen sich Bakterien fortbewegen.
TLR6:
   bildet mit TLR2 ein Heterodimer, ist der Rezeptor von diacyliertem Lipoprotein und bestimmten Peptidoglykanen. Ein spezielles Lipoprotein (MALP-2 = macrophage-activating lipopeptide) wird mittels Unterstützung durch das membrangebundene Protein CD36 detektiert (TLR assistierendes Protein).
TLR7&TLR8:
   sind Rezeptoren für Imidazochinolinen und von ssRNA/dsRNA z.B. von RNA-Viren.
TLR9:
   ist der Rezeptor für bakterielle DNA, bzw. für nicht methylierte CpG Motive, die in bakterieller DNA gehäuft (20 x häufiger als in Säugerzellen) auftritt. Das CpG Motiv ist in Säugerzellen stark methyliert, wodurch es unterschieden werden kann. Ähnliches wie für bakterielle DNA gilt auch für virale DNA, die auch von TLR9 detektiert wird. Über die immunstimulatorische Eigenschaft von bakterieller DNA berichtete schon Anfang der 80er Jahre die Gruppe um Dr. Tokunaga. Als zugehöriger Rezeptor wurde von der Gruppe um Dr. Shizuo Akira der TLR9 Rezeptor identifiziert (Aufklärung der Rollen von Toll-Rezeptoren und ihrer Signaltransduktionskaskaden mittels Gen-Targeting, Robert-Koch-Vo_{d}esung von Dr. Shizuo Akira, Allgemeine Presseinformation 2002; www.robert-koch.stiftung.de).
TLR10:
   Ligand noch nicht bekannt
TLR11:
   Ist Rezeptor für das urpathogene Bakterium Escherichia coli und dem Profilin-ähnlichen Protein des Urtierchens Toxoplasma gondii
TLR12:
   Funktion und Ligand noch unbekannt
TLR13:
   Funktion und Ligand noch unbekannt
Lokalisation der TLR:
   TLR2, 4, 5 und 6 sitzen insbesondere in den Plasmamembranen von Monozyten, natürlichen Killerzellen, Mastzellen oder myaloiden dendritischen Zellen., 7, 8, und 9 befinden sich insbesondere in Endosomen von Immunzellen (Siegmund-Schultze N., www.aerzteblatt.de). Die Aktivierung der Immunantwort erfordert daher eine intrazelluläre Aufnahme über Endozytose und eine Reifung der Endosomen. Die Signalweiterleitung beginnt hier in einem endosomalen Kompartiment. Für TLR 3 gibt es Hinweise, dass er sich in den Plasmamembranen befindet, allerdings gibt es auch Darstellungen in der Literatur die von einer endosomalen Lokalisation ausgehen. TLRs agieren häufig paarweise und treten bei unterschiedlichen Zelltypen in unterschiedlichen Kombinationen auf.
Signaltransduktionskaskaden:
   Die Signaltransduktionswege der unterschiedlichen TLRs (Perry A.K. et al; Cell Research 2005; 15(6); 407-422 und Kawai T. et al.; J. Biochem; 2007; 141; 137-145) sind teilweise zwar ähnlich, weisen aber durchaus auch größere Unterschiede auf, sodass es am Ende zu einer unterschiedlichen Genexpression und damit unterschiedlichen biologischen Reaktionen kommt. Mit Ausnahme von TLR3 geben alle TLRs ihr Signal an das Adapterprotein MyD88 weiter. MyD88 spielt eine entscheidende Rolle bei der Signalübermittlung über den TLR/Interleukin-1 Rezeptor. Die zytosolische Domäne der TLRs zeigt hohe Ähnlichkeit zu der des Interleukin-1 Rezeptors und wird daher auch als Toll/IR-1 Rezeptor Domäne (TIR) bezeichnet. MyD88-defiziente Splenozyten zeigten z.B. keine Reaktionen auf Interleukin-1, LPS oder CpG-DNA. Außerdem wurde bei MyD88 defizienten Zellen in Reaktion auf TLR2, TLR7, TLR9-Liganden keine Aktivierung von Signalmolekülen, wie NF-kB oder MAP Kinasen beobachtet. Das ist ein deutlicher Hinweis auf die vollständige Abhängigkeit der TLRs (außer TLR3) von MyD88 für deren Signalweiterleitung. Andere Adaptermoleküle sind z.B. TIRAP (Toll-Interleukin-1-Rezeptor(TIR)-Domäne enthaltendes Adapterprotein (TLR1, TLR2, TLR4 und TLR6), Mal (MyD88-adapter-like), TRIF (TLR3 und TLR4) und TRAM (TLR4).

Welche Proteine neben den Adaptermoieküien noch mitwirken hängt vom jeweiligen TLR ab. Allgemein und vereinfacht dargestellt beginnt eine Signatransduktionskaskade in der Regel mit einem Rezeptor an der Zelloberfläche mit einer zytosolischen Domäne, der sein Signal bei Belegung mir einem passenden Liganden über zytosolische Adaptermoleküle an Kinasen weiterleitet, die über Kaskaden Transkriptionsfaktoren aktivieren. Die aktivierten Transkriptionsfaktoren lokalisieren im Kern und lösen die Expression von Proteinen, meistens Zytokinen aus.

### Signaltransduktionskaskade über TLR1ITLR2, TLR2frLR2, TlR2ITLR6

Die Rezeptoren, die in entsprechenden Paaren auftreten, lösen bei der Belegung mit passenden Liganden die gleichen Signaltransduktionskaskaden aus. Letztendlich werden unter anderem die Transkriptionsfaktoren NF-kB und AP-1 aktiviert, die insbesondere zur Expression von Zytokinen führen. Dabei sind bei der Signalübertragung die Adaptermoleküle RAC-1, TIRAP, MyD88 und TRAF6 beteiligt. Als Kinasen sind mindestens IRAK1, IRAK4, TAK1, Pl 3K, IKKalpha, IKKbeta, IKKgamma, JNK, p38 MAPK und MKKs beteiligt.

### Signaltransduktionskaskade über TLR4

Der Rezeptor benötigt das membrangebundene Protein CD14 für seine volle Funktion. CD14 bindet entsprechende Agonisten und führt Sie dem Rezeptor zu. Dieser löst bei der Belegung mit passenden Liganden letztendlich die Aktivierung der Transkriptionsfaktoren NF-kB, AP-1, IRF3 und IRF7 aus und führt wiederum insbesondere zur Expression von Zytokinen. Dabei sind bei der Signalübertragung die Adaptermoleküle TIRAP, MyD88, TRAM, TRIF, TRAF3, TRAF6, NAP1 und RIP1 beteiligt. Als Kinasen sind mindestens IRAK1, IRAK4, TAK1, IKKalpha, IKKbeta, IKKgamma, IKKepsilon, TBK1, ERK1, ERK2, JNK, p38 MAPK, MEK1, MEK2 und MKKs beteiligt.

### Signaltransduktionskaskade über TLR5

Der Rezeptor löst bei der Belegung mit passenden Liganden letztendlich die Aktivierung der Transkriptionsfaktoren NF-kB und AP-1, die insbesondere zur Expression von Zytokinen führen. Dabei sind bei der Signalübertragung die Adaptermoleküle MyD88 und TRAF6 beteiligt. Als Kinasen sind mindestens IRAK1, IRAK4, TAK1, IKKalpha, IKKbeta, IKKgamma, JNK, p38 MAPK und MKKs beteiligt.

### Signaltransduktionskaskade über TLR10,11,12,13

Die Rezeptoren lösen bei der Belegung mit passenden Liganden letztendlich die Aktivierung der Transkriptionsfaktoren NF-kB und AP-1, die insbesondere zur Expression von Cytokinen führen. Dabei sind bei der Signalübertragung die Adaptermoleküle MyD88 und TRAF6 beteiligt. Als Kinasen sind mindestens IRAK1, IRAK4, TAK1, IKKalpha, IKKbeta, IKKgamma und MKKs beteiligt. '

### Signaltransduktionskaskade über TLR3:

Der Rezeptor löst bei der Belegung mit passenden Liganden letztendlich die Aktivierung der Transkriptionsfaktoren NF-KB, AP-1, IRF3 und IRF7 aus, wobei wiederum insbesondere Zytokine verstärkt exprimiert werden. Dabei sind bei der Signalübertragung die Adaptermoleküle TRIF, TRAF6, TRAF3, NAP1 und RIP1 beteiligt. Als Kinasen sind mindestens IRAK1, IRAK4, TAK1, IKKalpha, IKKbeta, IKKgamma, IKKepsilon, TBK1, PKR, Pl K3, JNK, p38 MAPK und MKKs beteiligt.

### Signaltransduktionskaskaden über TLR7, TLR8 und TLR9

Die Rezeptoren, lösen bei der Belegung mit passenden Liganden letztendlich die Aktivierung der Transkriptionsfaktoren NF-kB, AP-1, IRF1, IRF5 und IRF7 aus, wobei wiederum insbesondere Zytokine verstärkt exprimiert werden.. Dabei sind bei der Signalübertragung die Adaptermoleküle MyD88, TRAF6 und TRAF3 beteiligt. Als Kinasen sind IRAK1, IRAK4, TAK1, IKKalpha, IKKbeta, IKKgamma, JNK, p38 MAPK und MKKs beteiligt.

TLRs sind von großem therapeutischem Interesse. TLR Agonisten werden z.B. als Adjuvantien in Impfstrategien oder in der Krebstherapie eingesetzt. Beispiele sind die Behandlung von Basalzellkarzinom durch die TLR7/8 Agonisten Imiquimod/Resiquimod oder von Blasenkrebs durch einen TLR2 Agonisten. Der TLR9 Rezeptor wird durch ein synthetisches CpG-haltiges Oligonukleotid (CpG 7909 und CpG 10101) für die Therapie von Autoimmunerkrankungen, Krebs und Infektionskrankheiten angesteuert. Kommerzialisiert werden TLR9 basierende Therapiestrategien von der Firma Coley Pharmaceuticals.

### TLR7 und TLR8 Agonisten

Bekannte kommerzielle erhältliche Agonisten für den TLR7 und TLR8 Rezeptor sind allgemein Immidazochinoline (Schön et al., Oncogene, 2008, 27, 190-199), wie Imiquimod (R837, 1-(2-methylpropyl)-1H-imidazol[4,5-c]quinolin-4-amine), Resiquimod (R848, 4-amino-2-(ethoxymethyl)-a,a-dimethyl-1H-imidazo[4,5-c]quinoline-1-ethanol) und Gardiquimod (1-(4-amino-2-ethylamino methylimidazo-[4,5-c]quinolin-1-yl)-2-methylpropan-2-ol), Guanosinanaloga, wie zB. Loxoribine (7-allyl-7,8-dihydro-8-oxo-guanosin) und andere wie zB. Bropirimine (2-amino-5-bromo-6-phenyl-4-pyrimidinone). Weitere Immidazochinoline wurden in der Literatur beschrieben (Miller et al. Drug News & Perspectives, 2008, 21(2), 69-87; Gorden et al., J. Immunol., 2005, 174, 1259-1268; Jurk et al., Eur. J. Immunol., 36, 1815-1826; 2006; Gorden et al., J. Immunol., 2006, 177, 8164-8170) und sollen durch Referenz mitaufgenommen sein. Weitere teilweise ebenfalls kommerziell erhältliche Agonisten sind Thiazolochinoline wie CL075 (Gordon KB et al., J. Immunol., 2005, 174(3), 1259-68) und CL097 (Schindler U. et al., Mol. Cell Biol., 1994, 14(9), 5820-5831) und das Guanosinanalogon Isatoribine (7-Thia-8-oxoguanosin) (Horsmans Y. et al., Hepatology, 42(3), 724-731 ).

Bekannte Agonisten für den TLR8 sind ganz allgemein ssRNA, insbesonders einzelsträngiges polyUridin und ssRNA mit U oder GU reichen Sequenzen (Diebold et al, Science, 2004, 303, 1529-1531; Heil et al., Science, 2004, 303, 1526-1529; Lund et al; Proc. Natl. Acad. Sci. USA, 2004, 101, 5598-5603). Dabei kann die ssRNA auch als Phosphothioat vorliegen. Besonders zu nennen sind die Sequenzmotive UGUGU und GUCCUUCAA (Hornung et al., Nat. Med., 2005, 11, 263-270; Judge et al., Nat. Biotechnol., 2005, 23, 457-462) die besonders in ssRNA ab einer Länge von 16bp stimulierend sind. Die ssRNAs müssen natürlich in die Endosomen transportiert werden, um den TLR8 zu erreichen. In der Regel geschieht dies mit den üblichen Transfektionsreagentien. Die Firma InvivoGen bietet eine ssRNA40 komplexiert mit einem kationischen Lipid (Lyovec) als TLR8 Agonisten käuflich an.

Aber auch durch eine Überreaktion der angeborenen Immunabwehr können zahlreiche Krankheiten ausgelöst werden, beispielsweise Autoimmunerkrankungen wie rheumatische Arthritis und systemischer Lupus erythematodes. Hier reagieren TLRs mit Zerfallsprodukten körpereigener Zellen und leiten damit die Immunabwehr fehl.

Die TLRs stehen sogar im Verdacht, ursächlich mit Herz-Kreislauf-Erkrankungen im Zusammenhang zu stehen. Entzündungsreaktionen am Herz können zur Bildung von arteriosklerotischen Plaques beitragen, die letztendlich durch Gefäßverschluss zum Infarkt führen können.

### Zytokine/Cytokines:

Zytokine sind multifunktionale Signalstoffe. Es handelt sich dabei um zuckerhaltige Proteine, die eine regulierende Funktion für das Wachstum und die Differerenzierung von Körperzellen haben. Einige von ihnen werden daher auch als Wachstumsfaktoren bezeichnet. Viele Zytokine spielen zudem eine wichtige Rolle bei immunologischen Reaktionen und werden daher auch Mediatoren genannt. Zytokine werden von den Zellen durch Sekretion in das umgebende Medium abgegeben und stimulieren andere Zellen, wenn diese einen passenden Rezeptor besitzen. Man unterscheidet 5 Hauptgruppen von Zytokinen:

### 1. Interferone (IFN)

Interferone weisen Zellen an, Proteine zu bilden, die eine virale Infektion erschweren oder unterbinden. Auch können Interferone antitumorale Wirkung haben.

### 2. Interleukine (IL)

Interleukine dienen insbesondere der Kommunikation von Immunabwehrzellen untereinander und erhöhen dadurch die Koordination bei der Abwehr von Krankheitserregern und der Tumorbekämpfung

### 3. Koloniestimulierende Faktoren z o

Koloniestimulierende Faktoren werden in der Niere gebildet. Es handelt sich um Wachstumsfaktoren für Blutkörperchen

### 4. Tumornekrosefaktoren (TNF)

Die wichtigste Funktion von TNFs ist, die Aktivität verschiedener Immunzellen zu regeln. Sie werden hauptsächlich von Makrophagen ausgeschüttet. TNFs können den Zelltod (Apoptose), Zellproliferation, Zelldifferenzierung und Ausschüttung anderer Zytokine anregen.

### 5. Chemokine

Chemokine sind Chemoattraktoren, die Zellen mit passenden Rezeptoren veranlassen durch Chemotaxis zur Quelle der Chemokine zu wandern

Von besonderer Bedeutung als Mediatoren für immunologische Prozesse sind die Interferone (IFN), insbesondere die Interferone vom Typ 1. Das erste Interferon dieser Art wurde 1957 von lsaacs und Lindemann gefunden (isaacs, A. et al.; J. Proc. R. Soc. Lond. B. Biol. Sci. 147, 258-267). Der Name rührt daher, dass dieses Protein mit der Replikation von Viren interferiert. Typ I Interferone sind Schlüsselzytokine, die eine antivirale Antwort von Zellen auslösen, einen "antiviralen Status" etablieren und Zellen des Immunsystems zu einer antiviralen Antwort stimulieren. Diese Gruppe bindet an einen Rezeptor, den so genannten IFN-alpha Rezeptor (IFNAR), der aus zwei Proteinketten (IFNAR1 und IFNAR2) besteht. Man unterscheidet verschiedene Untertypen. Diese werden als IFN-alpha, IFN-beta, IFNkappa, IFN-delta, IFN-epsilon, IFN-tau, IFN-omega und IFN-zeta bezeichnet. Wiederum von besonderer Bedeutung sind hier die Interferone des Typs alpha und beta, die von vielen Zellen sekregiert werden, z.B. von Lymphozyten, Makrophagen, Fibroblasten Endothelzellen, Osteoplasten und anderen.

Die IFN-alpha Proteine kommen in 13 Subtypen vor, die als IFNAX (x **₌** 1,2,4,5,6,7,8,10,13,14,16,17 und 21 ) bezeichnet werden. Alle ihre Gene liegen clusterartig auf dem Chromosom 9.

Von den IFN-beta Proteinen sind zwei beschrieben. Es handelt sich um IFNB1 und IFNB3. Ein als IFNB2 beschriebenes Protein konnte später als ein bekanntes Interleukin identifiziert werden.

Über eine Signaltransduktionskaskade, den sogenannten JAK/STAT Pathway wird nach der Bindung an den in der äußeren Zellmembran liegenden Interferon-Typ-1-Rezeptor der Transkriptionsfaktor "interferone stimulated gene factor 3" (ISGF3), ein Heterotrimer der Transkriptionsfaktoren STAT1, STAT2 und ₙIFN regulatory factor 9 (IRF9), welche in den Zellkern wandern und dort die Transkription von Hunderten von Effektormolekülen (über so genannte IFN induzierbare Gene) induziert. Diese Effektormoleküle beeinflussen direkt die Proteinsynthese, das Zellwachstum und das Überleben im Prozess der Etablierung des so genannten antiviralen Status" (antiviral state). In diesem Status wird die Infektiösität der Viren z.B. durch verringerte Replikationsraten abgewehrt oder zumindest verringert.

Zusätzlich wird das adaptive Immunsystem aktiviert, indem die Reifung von dendritischen Zellen ausgelöst, die Antikörperantwort der B Zellen und die T Zellantwort aktiviert wird. Es werden Lymphozyten und Monozyten durch induzierte Chemokine zum Ort der Infektion rekrutiert.

Auch Stress kann Signaltransduktionswege anstossen, die in einen antiviralen Status münden. Diese Signaltransduktionkaskaden kreuzen die Signaltransduktionkaskaden der TLR.

Stress-Signaltransduktionswege:
Zellulärer Stress kann ausgelöst werden, durch:
   - Hitze/Kälte
   - UV
   - mechanische Beanspruchung /Scherkräfte
   - Sauerstoffmangel
   - Nährstoffmangel
   - Osmotischer Stress
   - Oxidativer Stress/Freie Radikale
   - Entzündungen
   - biologische und chemische Agentien (zB. TNFalpha, Chemotherapeutika)

Die Zellen reagieren auf Stress mit komplexen Veränderungen in der Aktivität von Signalketten, an welchen spezifische MEK, MSK und MAP Kinasen und verschiedene Transkriptionsfaktoren (zB. NF-kB), Apoptoseregulatoren und Zellzyklus-regulatoren beteiligt sind. GTP-bindende Proteine (Ras/Rho-Familie) die an der Membran gebunden sind spielen bei der Reaktion der Zellen auf zellulären Stress eine besondere Rolle.

Die angeborene Immunantwort erfolgt sowohl intrazellulär als auch interzellulär. Bei der intrazellulären Antwort löst eine durch einen Kontakt mit einem Pathogen betroffene Zelle über PRRs, wie beispielsweise TLRs und RLHs, Signaltransduktionskaskaden aus, wodurch sich der physiologische Status und das Expressionsprofil der Zelle ändern. Daneben gibt es eine interzelluläre Antwort, bei der die durch einen Kontakt mit einem Pathogen betroffene Zelle andere Zellen, die keinem direkten Kontakt mit dem entsprechenden Pathogen ausgesetzt waren, von der "Infektion" mit dem Pathogen "informiert". Dabei werden von der durch einen Kontakt mit einem Pathogen betroffenen Zelle Zytokine freigesetzt, die von Zytokinrezeptoren, welche sich auf den anderen nicht mit dem Pathogen in Kontakt gekommen Zellen befinden, detektiert werden. Durch die Bindung der Zytokine an die Zytokinrezeptoren wird eine Signaltransduktionskaskade in den Zellen, die keinem direkten Kontakt mit dem Pathogen ausgesetzt waren, ausgelöst, mit der Folge, dass sich auch deren physiologischer Status und Expressionsprofil ändert, obwohl sie mit dem Pathogen nicht direkt in Berührung gekommen sind. Die Änderung des physiologischen Status und des Expressionsprofils der Zellen soll dabei den pathogenen Angriff abwehren und das Überleben der Zellen sichern.

Die interzelluläre Antwort grenzt sich von der intrazellulären Antwort durch die unterschiedlichen Rezeptoren und Agonisten ab. Als Rezeptoren fungieren bei der interzellulären Antwort Zytokinrezeptoren und bei der intrazellulären Antwort PRRs. Die Agonisten der interzellulären Antwort sind Zytokine und bei der intrazellulären Antwort sind die Agonisten pathogene Muster.

### Genliefermethoden

Die Transfektion, d.h. das Einbringen von genetischem Material in eukariotische Zellen, insbesondere Säugerzellen, ist heute eine Methode, die aus der modernen Forschung nicht mehr wegzudenken ist (Domb A. J.; Review in Molecules; 2005; 10; 34 und Xiang G; Keun-Sik K.; Dexi L.; Review in The AAPS Journal; 2007; 9(1) Article 9; http://www.aapsj.org). Ohne diese Methode wäre eine Aufklärung der Funktion verschiedener Gene wesentlich erschwert. Nicht zu vergessen ist die Möglichkeit, auf diesem Wege Proteine eukariotischen Ursprungs originalgetreu herzustellen, da die korrekte posttranslationale Modifikation durch die eukariotischen Zellen, im Gegensatz zu früher häufig verwendeten prokariotischen Zellen, sichergestellt wird. Des Weiteren wird für die nahe Zukunft erwartet, dass insbesondere das Einbringen von genetischem Material in humane Zellen, also die Gentherapie, Einzug in die moderne Medizin in Form klinisch getesteter Verfahren und Therapien halten wird. Das Einbringen von genetischem Material ermöglicht es z.B. in eukariotischen Zellen zerstörte DNA Bereiche zu ersetzen und somit Fehlfunktionen zu beheben. Des Weiteren können Suizidgene eingeschleust werden, die beispielsweise Krebszellen zum "Selbstmord" zwingen. Aber auch das Stilllegen (Knock-down) von Genen kann erreicht werden, indem beispielsweise siRNA (small interfering RNA), Ribozyme oder Antisense Moleküle zum Einsatz kommen. Mit der Möglichkeit, auf den genetischen Steuerungsapparat der Zelle zugreifen zu können, steht dem Menschen daher ein wertvolles Mittel zur Verfügung, sein Verständnis aber auch seinen Einfluss auf die natürlich ablaufenden Prozesse in einer Zelle zu vermehren.

In den vergangenen Jahren erlangte die Erforschung so genannter Genliefermethoden (Gene Delivery Systems), die sowohl in vitro als auch in vivo eingesetzt werden können, enorme Bedeutung, da jenen große Chancen eingeräumt werden, der Gentherapie zum Durchbruch zu verhelfen. Ein Schwerpunkt der Gentherapieforschung besteht darin, Viren als Carriersysteme zu nutzen. Da das Einbringen von DNA oder RNA in Fremdzellen ein integraler Bestandteil des Vermehrungszyklus der Viren ist, wurde diese Fähigkeit durch einen natürlichen, evolutiven Prozess in der Entwicklungsgeschichte der Viren soweit verfeinert, dass es bis heute keine effektiveren Gen-Carrier gibt. Die natürlich vorkommenden Viren werden gentechnisch so manipuliert, dass sie Ihre Fähigkeit zur Reproduktion und ihre Pathogenität verlieren, jedoch eine Zelle mit rekombinant eingebrachtem genetischem Material infizieren können. Da Viren außer aus genetischem Material im Wesentlichen aus Proteinen bestehen, bieten Sie dem Immunsystem allerdings eine große Angriffsfläche. Dabei hat das Immunsystem in einem ebenso evolutionären Anpassungsprozess Strategien entwickelt, sich diesen Eindringlingen zur Wehr zu setzen. Daher wird die Immunantwort des Körpers als ein besonders bedeutender Faktor bezüglich gescheiterter Gentherapiestudien genannt.

Die gegenwärtig zur Verfügung stehenden Genliefermethoden können in die zwei Hauptgruppen virale Systeme und nicht-virale Systeme unterteilt werden. Die nicht-viralen Systeme können wiederum in chemische und physikalische Methoden unterschieden werden.

Von den nicht-viralen Systemen, die auf chemischen Methoden beruhen, sind insbesondere solche erwähnenswert, die auf kationischen Lipiden (sog. Lipofektion) oder kationischen Polymeren (sog. Polyfektion) beruhen. Deren Effizienz liegt in der Regel weit hinter den viralen Systemen.
Allseits bekannte kationische Polymere sind beispielsweise Poly-L-Lysin (PLL), (EP 388758) Polyethylenimin (PEI), (J.P. Behr et al.; Proc. Natl. Acad. Sci. USA; 1995;92; 7297; WO 9602655), Diethylaminoethyldextran (DEAE), (S. C. De Smedt et al.; Phar. Res.; 2000; 17; 113), Starburst Dendrimere (PAMAM), (F.C. Szoka et al.; Bioconjug. Chem.; 1996; 7; 703; WO 9502397), Chitosanderivate (W. Guang Liu et al.; J. Control. Release; 2002; 83; 1) oder auch Polydimethylaminoethylmethacrylate (P. van de Wetering et al.; J. Gene Med.; 1999; 1; 156; WO 9715680). Auch die weit verbreitete Ca-Phosphat-Präzipitationsmethode nutzt in weiterem Sinne ein "kationisches Polymer-" und kann daher zu dieser Gruppe gezählt werden.

Kommerziell erhältliche Produkte solcher kationischer Polymere sind z.B. Superfect, Polyfect (Qiagen), ExGen500 (Biomol) und jetPEI (Qbiogene).
Ebenso bekannte kationische Lipide (J. P. Behr; Bioconjugate Chem.; 1994; 5; 382) sind beispielsweise DOTMA (US 4946787), DOTAP (Leventis et al.; Biochim. Biophys. Acta; 1990; 1023; 124), DOGS (EP 394111 ), DOSPA (WO 9405624), DOSPER (WO 97002419), DMRIE (US 5264618) oder DC-Chol (Huang et al; Biochem. Biophys. Res. Commun.; 1991; 179; 280; WO 9640067). Solche oder ähnliche Lipide werden als solche oder in Kombination mit so genannten Colipiden (z.B. DOPE) in der Regel in ethanolischen, wässrigen Pufferlösungen als Micellen oder Liposomen formuliert. Als solche, oder auch als Öl oder Festsubstanz zur Eigenformulierung sind sie als kommerziell erhältliche Reagenzien, wie Lipofectin, Lipofectamin, Lipofectamine 2000 (Invitrogen), Fugene (Roche), Effectene (Qiagen), Transfectam (Promega), Metafectene (Biontex) etc. erhältlich.

Kationische Lipide und kationische Polymere bilden in Anwesenheit von DNA oder RNA aufgrund der gegenläufigen Ladungsverhältnisse spontan so genannte Lipoplexe oder Polyplexe. Die DNA wird dabei durch die Kompensation der negativen Ladung am Phosphatrest kondensiert, also in ihrer Größe minimiert. Im Allgemeinen hängt die Transfektionseffizienz von Lipoplexen oder Polyplexen von einer Vielzahl von Parametern ab. Die wichtigsten sind das Mengenverhältnis von genetischem Material zu kationischer Komponente bei der Herstellung der Lipo/Polyplexe, lonenstärke während der Herstellung der Lipo/Polyplexe, Absolutmenge von Lipo/Polyplexen pro Zelle, Zelltyp, Proliferationszustand der Zellen, physiologischer Status der Zellen, Zellteilungsrate, Inkubationszeit etc. Diese Einflussparameter sind Ausdruck eines komplizierten Transfektionsgeschehens, bei der die Lipo/Polyplexe bzw. die enthaltenen genetischen Materialien eine Vielzahl von zellulären Barrieren überwinden müssen.

Die erste Barriere stellt die äußere negativ geladene Zellmembran dar. Es wird angenommen, dass transfektionsaktive Lipoplexe, die eine positive Nettoladung haben müssen, durch adsorptive Endocytose oder Flüssigphasenendocytose in das Innere der Zelle gelangen. Durch die Endocytose, die einen aktiven Transportprozess der Zelle darstellt, wird Material auf der Zelloberfläche mit Zellmembran ummantelt und als Vesikel (Endosom) internalisiert. Durch Verschmelzung mit so genannten Lysosomen, die ein komplexes Gemisch von Enzymen beinhalten, werden die in den Endosomen enthaltenen Stoffe abgebaut. Da zu diesem Abbau ein niedriger pH-Wert nötig ist, besitzen Endosomen Protonenpumpen, die solange Protonen in die Endosomen pumpen, bis ein entsprechender pH-Wert erreicht wird. Um Ladungsneutralität zu wahren, strömen im gleichen Ausmaß Chloridionen in die Endosomen.

Viele moderne kationische Lipide oder Polymere besitzen aus diesem Grund Puffereigenschaften. Auf diese Weise wird der niedrige pH-Wert nicht erreicht und es kommt zu einem Eintrag an Ionen in die Endosomen, der die Endosomen durch den entstehenden osmotischen Druck zum Platzen bringt. Auf diese Weise gelangen diese Lipo/Polyplexe in das Cytosol. Da auch eine Reihe transfektionsaktiver Lipide und Polymere ohne Puffereigenschaften bekannt sind, muss ein weiterer Mechanismus existieren, der die Lipo/Polyplexe in das Cytosol gelangen lässt. Man vermutet zumindest im Falle der Lipide eine Fusion der beteiligten Membranen und damit einhergehend eine Destabilisierung. Ob dabei vorwiegend der Lipoplex oder die enthaltende DNAIRNA als solches in das Cytosol gelangt ist unklar. Es wird jedoch vermutet, dass die DNA im Cytosol aus dem Lipoplex freigesetzt wird, da Versuche scheiterten, durch Mikroinjektion von Lipoplexen direkt in den Zellkern eine Proteinexpression zu erreichen. Es scheint, als ob die in den Lipoplexen gebundene DNA dem Transkriptionsapparat nicht zugänglich ist.

Handelt es sich um gegen mRNA gerichtete Antisense Moleküle oder siRNA, ist der biologische Wirkort erreicht und die Dauer der Wirkung hängt im wesentlichen von der Konzentration cytosolischer RNasen und der Rate der Freisetzung aus den Lipo/Polyplexen ab. DNA kann als solche nicht in den Zellkern eindringen, was als "Nuclear Barrier" bezeichnet wird. Sie gelangt allerdings während der Zellteilung an ihren Wirkort und führt so zur Expression von Proteinen.

Als weitere nicht-virale Methoden, die auf chemischen Methoden basieren, seien Systeme genannt, die ein DNA-bindenden Molekülteil sowie einen Liganden tragen, der rezeptorvermittelte Endozytose auszulösen vermag (Beispiel Transferinfektion; Wagner et al.; Proc. Natl. Acad. Sci.; 1990; 87; 3410).

Andere Verbindungen bestehen aus einer DNA und/oder RNA-bindenden Domäne, sowie einem Liganden, der einen Membrantransfer auslösen kann; z.B. Penetratin, Derossi et al.; Trends in Cell Biology; 1998; 8 ; 84 oder HIV Tat Petid, Gratton et al.; Nature Medicine; 2003; 9(3); 357. Unter Membrantransfer versteht man, daß ein Molekül von einer Seite der Membran auf die andere Seite gelangen kann. Als DNA und/oder RNA-bindende Domäne kommen alle Strukturelemente einer Verbindung in Frage, die DNA und/oder RNA über elektrostatische Wechselwirkungen (zB. Kationen) oder Wasserstoffbrückenbindungen (zB. Peptid Nucleic Acids, PNAs) binden können. Auch interkalierende Verbindungen (zB. Acridin) können zur Bindung von RNA/DNA verwendet werden. Die Verbindungen, die rezeptorvermittelte Endozytose oder Membrantransfer auslösen können, können aber auch kovalent an das genetische Material gebunden werden, wenn dadurch die biologische Wirkung nicht oder nur wenig beeinträchtigt wird.

Das bedeutendste Beispiel einer nicht-viralen Methode, die auf einem physikalischen Verfahren beruht, stellt die Elektroporation dar. Dabei werden die zu transfizierenden Zellen zwischen zwei Elektroden verbracht, an die ein typischer Spannungsverlauf angelegt wird. Auf diese Weise werden die Zellen einem intensiven elektrischen Stromstoß (Puls) ausgesetzt, der zur einer reversiblen Öffnung (Poren) der Zellmembran führt. Durch diese Poren können Substanzen, wie z.B. genetisches Material, die sich in unmittelbarer Umgebung der Poren befinden in die Zelle eindringen. Der Puls (also Spannungsverlauf) als einer der wichtigsten Erfolgsparameter muss für jeden Zelltyp optimiert werden. Es gibt inzwischen einige kommerzielle Anbieter für Elektroporatoren (z.B. Eppendorf/Multiporator, US 6008038, Biorad/Genpulser, US 4750100, Genetronics Inc., US 5869326, BTX/ECM Serie), die speziell für eukariotische Zellen entwickelt wurden und eine Anpassung der Pulsparameter an den jeweiligen Zelltyp erlauben. Tatsächlich gibt es inzwischen auch Vorrichtungen die eine in vivo Applikation möglich machen. Bei der in vitro Anwendung werden die Zellen in einem Elektroporationspuffer suspendiert, zusammen mit der zu transfizierenden DNA/RNA in eine mit Elektroden versehene Elektroporationsküvette verbracht und einem oder mehreren Pulsen ausgesetzt. Neben dem Spannungsverlauf sind weitere wichtige Parameter die Beschaffenheit des Puffers, die Temperatur, die Zellkonzentration und die DNA-Konzentration. Nach dem die Zellen dem Puls ausgesetzt wurden, lässt man ihnen eine kurze Zeit zur Regeneration der Zellmembran. Anschließend werden die Zellen in ein Kulturgefäß ausgesäht und wie üblich kultiviert.

Als weitere physikalische Methoden seien Mikroinjektion, Hydrodynamische Methoden, ballistische Methoden (Genegun) oder Methoden, die Ultraschall benutzen genannt oder auch die Injektion nackter DNA in verschiedene Organe, die zu geringer Expression der entsprechenden Gene führt.
Zu den Verfahren die physikalische Methoden als auch chemische Methoden vereinen, zählt insbesondere auch die Magnetofektion, die DNA-bindende Moleküle auf magnetischen Nanoteilchen nutzt um über eine magnetischen Feldgradienten DNA auch der Oberfläche von Zellen anzureichern und Endozytose auszulösen

Den enormen Möglichkeiten, die das Einbringen von genetischem Material in eukariotische Zellen mit sich bringt, steht ein Arsenal von Methoden gegenüber, die nur unbefriedigende Leistungsfähigkeit aufweisen. Die jeweils spezifisch auftretenden Mängel bis dato vorhandener Methoden betreffen im Wesentlichen die wichtigen Parameter Effizienz, Toxizität, Immunogenität, Targeting, Restriktion bzgl. der Größe des genetischen Materials, Möglichkeiten der in vivo/in vitro Anwendung, Möglichkeit von High-Throughput-Anwendungen, Gefahrenpotential, Einfachheit der Methode und Kosten der Methode. Kein Verfahren ist in der Lage, alle diese Parameter ausreichend zu erfüllen. Dem Fehlen eines geeigneten Gen-Carriersystems wird zugeschrieben, dass sich bis heute trotz erheblicher Forschungsaufwendungen keine auf Gentherapie beruhende medizinische Therapie etablieren konnte.

Insbesondere kann das angeborene Immunsystem von Eukaryoten eine erhebliche Barriere für nicht-virale Genliefersysteme darstellen. Der Grund dafür ist, dass das angeborene Immunsystem von Eukaryoten in der Lage ist, über Toll Like Rezeptoren fremdes genetisches Material zu erkennen und Signaltransduktionskaskaden anzustoßen, die einen antiviralen Zustand von Zellpopulationen auslösen. Ein derartiger antiviraler Zustand einer Zelle stellt auch eine Barriere für die Transfektion mit einem nicht-viralen Genliefersystem dar, die kaum bzw. nicht überwunden werden kann.

So zeigten beispielsweise repetitive Lipofektionsversuche, bei denen zunächst eine Transfektion mit einer spezifisch gegen ein bestimmtes Protein gerichteten siRNA durchgeführt wurde und anschließend eine Plasmidtransfektion mit einem Reportergen folgte, dass der Transfektionserfolg der Plasmidtransfektion ausblieb, obwohl die Zellen einen gesunden Eindruck machten. Nur bei sehr geringen siRNA Mengen konnte eine geringe Menge des Reporterproteins detektiert werden.

Um auszuschließen, dass es sich um einen OFF-Target-Effekt der spezifischen siRNA handelt, wurde der Versuch mit einer unspezifischen siRNA wiederholt, die gegen das humane Erbgut "geblastet" wurde. Das Ergebnis blieb jedoch dasselbe.

Da bekannt ist, dass auch die Proliferation der Zellen einen Einfluss bei der Lipofektion hat, wurde untersucht, ob die Zellen in Ihrem Proliferationsverhalten durch die Vortransfektion mit siRNA beeinträchtigt wurden. Dabei konnte festgestellt werden, dass die Proliferationsraten bei höheren siRNA Mengen sinken, jedoch eine ausreichende Proliferation bei den Experimenten gegeben, war, als die der Vortransfektion folgende Plasmidtransfektion fehlschlug. Es schien, als ob die Zellen sich überraschenderweise gegen die zweite Transfektion wehren können.

Mit repetitiven Transfektionsversuchen, bei denen zwei Plasmidtransfektionen hintereinander geschaltet wurden, konnte ein ähnliches Ergebnis erhalten werden, wenn auch nicht mit der oben genannten Deutlichkeit. Der zweite Transfektionschritt war häufig entweder sehr ineffizient oder kontraproduktiv. Auch hier wurden ähnliche Untersuchungen, wie oben genannt, durchgeführt, um sicherzustellen, dass es sich nicht um toxische Effekte handelt. Weitere Untersuchungen zeigten, dass es zur Aussschüttung von lnterferonen kam.

Transfektion von siRNA zum Auslösen von RNA-Interferenz
Durch das Einbringen von dsRNA in Zellen können Gene gezielt ausgeschaltet werden, wenn die mRNA eine Sequenzhomologie zu der eingeführten dsRNA aufweist.
Der Prozeß wird als RNA-Interferenz bezeichnet (Fire et al., Nature, 1998, 391, 806-811) und läuft meist folgendermaßen ab. Eine in die Zelle eingeschleuste dsRNA mit homologer Sequenz einer zelleigenen mRNA wird durch das Enzym Dicer in viele kleine dsRNA-Fragmente von 21-25 Nukleotiden zerschnitten (Bernstein et al., Nature, 2001, 409, 363-366).

Dicer ist eine ATP- abhängige Ribonuclease. Die entstehenden Nukleotidfragmente besitzen am 3'-Ende 2-3 Nukleotide, welche überstehen. Die kleinen RNA-Stücke werden "small interfering RNA" (siRNA) genannt (Elbashir et al., Nature, 2001, 411, 494-498).

Die doppelsträngigen siRNAs werden unter ATP-Verbrauch vermutlich mit Hilfe einer Helicase (Dalmay et al., EMBO, 2001, J20, 2069-2078) entwunden. Danach wird ein Einzelstrang in den Proteinkomplex RISC (RNA induced silencing complex) überführt (Kuhlmann et al., Biol. unserer Zeit, 2004, 3, 142-150). Der am RISC verbleibende Strang kann mit einer komplementären RNA (target RNA) hybridisieren. Anschließend wird die target-RNA von einer integralen Endoribonuklease zerschnitten. Da Gene nur über den Umweg von einsträngiger mRNA wirken können, wird dieses Gen dadurch faktisch ausgeschaltet. Es wird zwar noch transkribiert, aber die RNA-Interferenz (RNAi) baut diese mRNA genau so schnell wieder ab. Aus diesem Grund wird die RNA-Interferenz auch als ₙPosttranscriptional Gene Silencing" (PTGS) bezeichnet.

Die RNA-Interferenz findet man bei Protozoen, Pilzen, Pflanzen und Tieren wenn sich auch die einzelnen Mechnismen leicht unterscheiden. Archaebakterien und Prokaryonten verfügen nicht über diese Fähigkeit.

Die Frage nach der Funktion ist noch nicht genau geklärt. Man vermutet, dass es zur Abwehr von RNA-Viren dient. Mit Viren infizierte Pflanzen können sich beispielsweise erholen und bei neu entwickelten Blättern schwächen sich die Symptome ab. Symptomfreie Blätter können nicht mehr mit artverwandten Viren infiziert werden. Von den eingedrungenen Viren bzw. von derer RNA werden komplementäre Kopien erstellt, die als Matrize für die Synthese der ursprünglichen RNA dienen. Es bildet sich virusspezifische dsRNA, welche den PTGS Mechanismus auslöst. Da am Anfang die Konzentration an dsRNA zu gering ist, kann sich die Pflanze erst allmählich erholen und die Herrschaft über die Viren erlangen. Es wird vermutet, dass eine zelleigene RNA-abhängige RNA-Polymerase (RdRP) das einsträngige Viren-Genom erkennt, in dsRNA umwandelt und dann den RNAi- Vorgang startet. Die These des PTGS zur Abwehr der Viren wurde unterstützt durch den Fund von Inhibitoren gegen das PTGS in Viren. Wie genau diese wirken und ob Pflanzen wiederum Mechanismen gegen diese Inhibitoren entwickelt haben, ist noch nicht bekannt.

Die RNA-Interferenz hat in den letzten Jahren eine immense Bedeutung erlangt, da sie die Möglichkeit bietet unerwünschte Gene bzw. Proteine auszuschalten und damit Virus- und andere Krankheiten zu bekämpfen. Desweiteren ist sie auch in der Forschung zur Aufdeckung von Gen-Funktionsbeziehungen zu einem unverzichtbaren Hilfmittel geworden.

Die am häufigsten verwendete Variante RNA-Interferenz zu nutzen, besteht in der Transfektion von synthetisch hergestellten siRNA-Molekülen, also doppelsträngiger RNA mit einem 3'-Überhang von 2-3 Nukleotiden. In Säugerzellen bewirkt eine eingeschleuste dsRNA mit mehr als 30bp eine enzymatische Zerstörung aller mRNAs und den Stopp der Proteinsynthese (Kaufmann, Proc. Natl. Acad. Sci USA, 1999, 96, 11693-11695). Nach der Injektion von längerer dsRNA können einige höhere Eukaryonten mit der Produktion von Interferonen reagieren, welches die Expression viraler Gene hemmen und die Zelle in Apoptose lenken können. Will man dies verhindern, so muss für Säugerzellen die Länge der siRNA unter 30 bp liegen (Tuschl et al., Genes Dev., 2001, 15, 188-200). Als Transfektionsmethoden stehen die aus der DNA-Transfektion bekannten Methoden zur Verfügung. Der Unterschied zur DNA Transfektion besteht lediglich im Wirkort des eingeschleusten genetischen Materials. Bei der DNA Transfektion ist dies der Kern. Bei der siRNA Transfektion ist es das Cytosol.

Die am häufigsten verwendeten Methoden sind die Elektroporation, die Transfektion durch kationische Polymere und insbesonders die Transfektion durch kationische Lipide. Dabei sind nicht unbedingt die besten Reagentien bei der Plasmidtransfektion auch die besten Reagentien bei der siRNA Transfektion und umgekehrt. Daher werden spezielle für die siRNA Transfektion geeignete Reagentien kommerziell angeboten. Beispiele sind Interferrin (Polyplus), X-treme Gene siRNA (Roche), siPort (Ambion), Silentfect (Biorad), Dharmafect (Dharmacon) und Lipofectamin RNAiMax (Invitrogen).

Als Maß für den Transfektionserfolg wird bei der siRNA-Transfektion der relative Knock-down eines Proteins bzw. eines Gens im Vergleich zu einer unbehandelten Probe oder einer mit unspezifischer siRNA (siRNA ohne Target) transfizierten Probe verstanden. Ein Problem bei der siRNA-Transfektion stellen sogenannte OFF-Target-Effekte dar. Darunter versteht man beispielsweise die unbeachsichtigte Beeinträchtigung der Expression eines Gens, das nicht Ziel des Knockdowns ist. Dazu kann es zB. bei Sequenzähnlichkeiten kommen. Um diese OFF-Target-Effekte möglichst gering zu halten und aus Gründen der Toxizität, besteht eine Anforderung an potentielle siRNA-Transfektionsysteme darin, einen möglichst hohen Knockdown bei einer möglichst geringen Menge eingesetzter siRNA zu erreichen. Eine weitere bevorzugte Anforderung insbesondere für in vivo Anwendungen besteht darin, das Expressionsprofil der Zellen, abgesehen von der Expression des Targetproteins, so wenig wie möglich zu ändern.

### Beschreibung der Erfindung

Aufgabe der Erfindung ist es, ein Verfahren bereitzustellen, das eine effizientere Transfektion ermöglicht. Dies gilt sowohl für eine einfache Transfektion als auch für eine wiederholte, d.h. mindestens zwei- oder mehrmalige Transfektion. Weiter stellt sich die Aufgabe den physiologischen Status der Zellpopulation so wenig wie möglich zu beeinflussen, d.h. das Protein-Expressionsprofil der Zellpopulation sollte sich idealerweise nur bezüglich der Proteine ändern, deren Gene in die Zelle eingeschleust wurden oder deren Expression durch das eingeschleuste genetische Material herabgesetzt oder blockiert werden. Im Falle der Transfektion von siRNA kann es jedoch auch vorteilhaft sein den physiologischen Status der Zelle absichtlich zu Verändern, um die Zellen in einen "antiviralen Status" zu bringen, da hier die RNA-Interferenz besonders effizient verläuft. Weiterhin wird eine Zusammensetzung und ein Kit of parts bereitgestellt, die die Komponenten zur Durchführung einer effizienteren Transfektion von eukariotischen Zellen mit nicht-viralen Genliefersystemen enthalten.

Diese Aufgabe wird erfindungsgemäß gelöst durch ein Verfahren zur Verbesserung des Transfektionsergebnisses von nicht-viralen Genliefersystemen, dadurch gekennzeichnet, dass man
a) die Zellen vor und/oder bei der Transfektion mit mindestens einem Mittel zur zumindest teilweisen Unterdrückung der angeborenen intrazellulären und/oder interzellulären Immunabwehr behandelt und bei der Transfektion genetisches Material, insbesondere modifizierte und/oder unmodifizierte ssDNA, modifizierte und/oder unmodifizierte dsDNA, modifizierte und/oder unmodifizierte ssRNA, modifizierte und/oder unmodifizierte dsRNA und/oder modifizierte und/oder unmodifizierte siRNA, in die Zellen einbringt; oder
b) die Zellen vor und/oder bei und/oder nach der Transfektion mit mindestens einem Mittel zur zumindest teilweisen Aktivierung der angeborenen intrazellulären und/oder interzellulären Immunabwehr behandelt und bei der Transfektion modifizierte und/oder unmodifizierte siRNA in die Zellen einbringt.

Das erfindungsgemäße Verfahren zur Verbesserung des Transfektionsergebnisses kann in vitro und/oder in vivo durchgeführt werden.

Durch die zumindest teilweise Unterdrückung der angeborenen intrazellulären und/oder interzellulären lmmunabwehr, d.h. eine der intra- und/oder inter-zellulären Signaltransduktionskaskaden der angeborenen Immunabwehr wird unterbrochen, lässt sich das Transfektionsergebnis verbessern und/oder unerwünschte Änderungen des Expressionsprofils einer transfizierten Zelle vermeiden.

Das heißt, dass insbesondere die intrazelluläre Signaltransduktionskaskade ausgehend von den TLRs, über die Adaptermoleküle, über die entsprechenden Kinasen, welche wiederum über die Aktivierung von Transkriptionsfaktoren Zytokine, insbesondere die Interferone induzieren und Membrantransportvorgänge herunterregulieren, unterbrochen oder geschwächt werden kann. Weiter kann die Signalübertragung durch Botenstoffe zwischen den Zellen unterbrochen werden. Da es sich dabei allesamt um Proteine handelt, werden erfindungsgemäß vorzugsweise aktivitätssteigernde oder aktivitätsmindernde Effektoren wie Antikörper, Aptamere, Antagonisten oder Inhibitoren gegen diese Proteine eingesetzt. Dabei können die entsprechenden Wirkstoffe als solches oder mit entsprechenden Hilfsmolekülen an oder in die Zellen verbracht werden, je nach Zellgängigkeit oder Zielort. So können z.B. Wirkstoffe über liposomale Carrier in die Endosomen verbracht werden. Ist der Zielort das Zytosol, bieten sich insbesondere Elektroporation oder spezielle Peptidsequenzen an, die in der Lage sind, die Zellwände zu permeabilisieren. Handelt es sich bei den Wirkstoffen um Peptide oder Proteine, so können diese erfindungsgemäß auch durch Transfektion geeigneten genetischen Materials intrazellulär gebildet werden und falls nötig mit Lokalisationssequenzen zu den entsprechenden Zellkompartimenten dirigiert werden. Will man über siRNA Gene stilllegen, die für entscheidende Proteine des Signatransduktionapparates kodieren, stehen erfindungsgemäß die bekannten Transfektionssysteme zur Verfügung.

Das erfindungsgemäße Verfahren bzw. die erfindungsgemäße Verwendung kann sowohl in vitro als auch in vivo eingesetzt werden bzw. erfolgen. Es kann dazu angewendet werden die Ausbildung des "antiviralen Status" von Zellen während der Transfektion zu verhindern oder auch schon vorher. Da die Zellen bei der Kultivierung auch mit biologischem Material zB. Serum oder Trypsin in Verbindung kommen können, das Stoffe beinhalten kann, auf die ein oder mehrere TLR ansprechen (zB. DNA, RNA, LPS etc.), kann es dazu kommen, dass sich Zellen schon in einem antiviralen Status befinden, bevor mit der Transfektion begonnen wurde. Unter Beachtung dieses Hintergrundes wird auch verständlich, warum es als schwierig gilt, Transfektionsergebnisse zu repoduzieren. Die Qualität von Transfektionsergebnissen hängt stark vom immunologischen Ausgangszustand der Zellen ab, der wiederum von der Vorbehandlung (zB. Subkultivierung) abhängt.

Bei dem erfindungsgemäßen Verfahren kann das nicht-virale Genliefersystem ein kationisches Lipid, ein kationisches Polymer, oder ein kationisches Protein umfassen; und/oder eine Verbindung umfassen, die eine DNA und/oder RNA-bindende Domäne aufweist und rezeptorvermittelte Endozytose oder einen Membrantransfer auslösen kann; und/oder eine Verbindung umfassen, die kovalent an DNA und/oder RNA gebunden ist und rezeptorvermittelte Endozytose oder einen Membrantransfer auslösen kann; und/oder auf einer physikalischen Methode wie Elektroporation, Mikroinjektion, Magnetofektion, Ultraschall oder einer ballistischen oder hydrodynamischen Methode beruhen.

Ferner kann bei dem erfindungsgemäßen Verfahren die Transfektion mindestens zweimal,, d.h. zwei- oder mehrfach (3, 4, 5, 6, etc.), ausgeführt werden.

Weiterhin kann bei dem erfindungsgemäßen Verfahren bevorzugt ein kationisches Lipid in dem nicht-viralen Genliefersystem enthalten sein, insbesondere ein kationisches Lipid gemäß Formel (1): 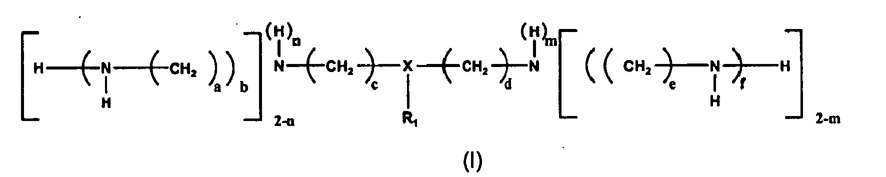 wobei 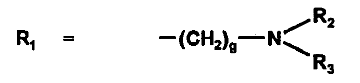 sein kann, wobei
R₂ und R₃ unabhängig voneinander Dodecyl, Dodecenyl, Tetradecyl, Tetradecenyl, Hexadecyl, Hexadecenyl, Octadecyl, Octadecenyl oder andere Alkylreste, die in allen möglichen Kombinationen gesättigt, ungesättigt, verzweigt, unverzweigt, fluoriert oder nicht fluoriert sein können, und aus 5 bis 30 Kohlenstoffatomen aufgebaut sein können; 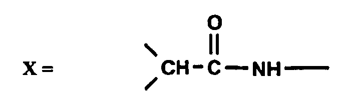 sein kann; und
wobei m = 0 und n = ₀; oder m = 0 und n = 1; oder m = 0 und n=2;oderm=1 1 und n = ₁; oder m = 1 und n = 2; oder m = 2 und n = 2 sein kann; und
g = 1,2,3,4,5,6,7 oder 8 sein kann; a = 0,1,2,3,4,5 oder 6 sein kann; b = 0,1,2,3,4,5 oder 6 sein kann; c = 0,1,2,3,4,5 oder 6 sein kann; d = 0,1,2,3,4;5 oder 6 sein kann; e = 0,1,2,3,4,5 oder 6 sein kann, und f = 0,1,2,3,4,5 oder 6 sein kann.

Noch mehr bevorzugt kann bei dem erfindunsgemäßen Verfahren ein nicht-virales Genliefersystem, umfassend ein kationisches Lipid gemäß der Formel (1) eingesetzt werden, wobei R₂ und R₃ unabhängig voneinander Dodecyl, Dodecenyl, Tetradecyl, Tetradecenyl, Hexadecyl, Hexadecenyl, Octadecyl, Octadecenyl sein können; m = 1 und n = 1 sein kann; und g = 1,2,3,4,5,6,7 oder 8 sein kann; a = 0,1,2,3,4,5 oder 6 sein kann; b = 0,1,2,3,4,5 oder 6 sein kann; c = 0,1,2,3,4,5 oder 6 sein kann; d = 0,1,2,3,4,5 oder 6 sein kann; e = 0,1,2,3,4,5 oder 6 sein kann; und f = 0,1,2,3,4,5 oder 6 sein kann.

Bei dem erfindungsgemäßen Verfahren kann die angeborene intrazelluläre und/oder interzelluläre Immunabwehr durch mindestens einen Antikörper, Intrabody, Aptamer, Antagonisten, Inhibitor und/oder eine siRNA, der/die die Weiterleitung eines intra- und/oder interzellulären Signals der angeborenen Immunabwehr blockiert(en), zumindest teilweise unterdrückt werden. Entsprechende Antikörper, Intrabodies, Aptamere, Antagonisten und Inhibitoren sind kommerziell erhältlich.

Weiterhin kann bei dem erfindungsgemäßen Verfahren durch Knock-down mit siRNA zumindest ein Gen ausgeschaltet werden, das für ein zur Signaltransduktion notwendiges Protein codiert. Entsprechende siRNA oder Plasmide, die shRNA (short hairpin RNA) z.B. gerichtet gegen TLR_{S}, Kinasen und Transkriptionsfaktoren sind teilweise kommerziell erhältlich (Imgenex/Invivogen).

Bei dem erfindungsgemäßen Verfahren kann zur zumindest teilweisen Unterdrückung der angeborenen intrazellulären und/oder interzellulären Immunabwehr mindestens einer aus der Gruppe TLR 1, TLR 2, TLR 3, TLR 4, TLR 5, TLR 6, TLR 7, TLR 8, TLR 9, TLR 10, TLR 11,TLR 12, TLR 13, CD14, CD38, RIG-1 Helikase und/oder RIG-I-like Helikase blockiert werden. Insbesondere ist es bevorzugt, TLR 1, TLR 2, TLR 4 und/oder TLR 9 zu blockieren. Weiterhin ist es bevorzugt, mehrere der vorstehend genannten Rezeptoren bzw. Proteine zu blockieren. Erfindungsgemäß sind Antikörper geeignet, die in der Lage sind, die TL-Rezeptoren zu blockieren. So sind Antikörper gegen die Toll Like Rezeptoren TLR1, TLR2, TLR3, TLR4, TLR5, TLR6, TLR7, TLR8, TLR9 und CD14 bekannt und kommerzielle erhältlich. Beipiele für Antikörper, die in der Lage sind, humane TLR zu blockieren sind: Mouse Anti-human-CD282 Antibody (= anti TLR2), monoclonal, AbD Serotec, Kat-nr.: MCA2484EL, Mouse Anti-humanTLR3 Antibody, monoclonal, Lifespan Biosciences Kat-nr.: LS-C18685, Mouse Anti-human-CD284 Antibody (= anti TLR4), monoclonal, AbD Serotec, Kat-nr.: MCA2061EL, Antikörper: Mouse Anti human TLR1 Antibody (monoclonal, 0.05% Natriumazid, 100 µg Lyophilisat); Invivogen, No. Mab-htlrl , Rabbit Anti-human-TLR9 Antibody (= anti TLR9), polyclonal, 0,5 µg/µl in PBS, 0,2% Gelatine, 0,05% Natriumazid, Lifespan Biosciences, Kat-nr.: MCA2484EL. Sind unerwünschte Stoffe wie Natriumazid in den kommerziell erhältlichen Antikörpern enthalten, so müssen diese unerwünschten Stoffe vor dem Einsatz des Antikörpers in dem erfindungsgemäßen Verfahren abgetrennt werden, zB. durch Dialyse.

Antikörper, die in der Lage sind, TLR zu blockieren können zusammen mit Endozytose auslösenden Lipo- oder Polyplexen als solche oder verpackt in Liposomen in die Endosomen geschleust werden und so die Rezeptoren blockieren. In der Regel reicht aber auch eine Zugabe zum extrazellulären Raum. Die Wahl des zu blockierenden Rezeptors hängt erfindungsgemäß natürlich auch von der Art des zu transfizierenden genetischen Materials ab. Desweiteren kann auch das transfizierende Agens die Wahl des zu blockierenden Rezeptors bestimmen. Die TLR, die genetisches Material detektieren, d.h. TLR 3, TLR 7, TLR 8 und TLR 9, sitzen in der Regel in den Endosomen. Die übrigen TLR sitzen auf der Plasmamembran. Will man beispielsweise DNA transfizieren, kann bevorzugt TLR 9 blockiert werden. Ist die DNA bakteriellen Ursprungs, ist es bevorzugt, zusätzlich zu TLR 9, TLR 4 und/oder TLR 5 zu blockieren.

In einer bevorzugten Ausführungsform kommt dabei eine Antikörperkonzentration von 0,01-100_{N}g/ml durch Zugabe zum Kulturmedium zur Anwendung. In einer noch mehr bevorzugten Ausführungsform beträgt die Konzentration des Antikörpers 0,01-1 Oug/mi Kulturmedium und in der am meisten bevorzugten Ausführungsform 0,01-5µg/ml.

Der Antikörper kann auch in den transfektionsaktiven Komplex, z.B. Lipoplex, integriert werden. In einer bevorzugten Ausführungsform beträgt das Verhältnis Antikörper : genetisches Material von 0,01 : 1 (µg/µg) bis 10 : 1 (µg/µg). In einer noch mehr bevorzugten Ausführungsform beträgt die Menge 0,01-1µg/µg und in der am meisten bevorzugten Ausführungsform 0,01-0,3µg/µg genetischem Material.

Für alle Antikörper, die erfindungsgemäß als Mittel zur zumindest teilweisen Unterdrückung der angeborenen intrazellulären und/oder interzellulären Immunabwehr eingesetzt werden können, gilt: Der oder die eingesetzten Antikörper muss/müssen in der Regel gegen das zu blockierende Zielmolekül, zB. einen Rezeptor wie einen TLR, Zytokinrezeptor, Interferonrezeptor, usw. der Zellen gerichtet sein, die transfiziert werden sollen. Handelt es sich beispielsweise um einen TLR einer humanen Zelle, so muss der Antikörper sich in der Regel gegen den humanen TLR Rezeptor richten, der blockiert werden soll. In manchen Fällen sind die Antikörper wegen der großen Ähnlichkeit der Zielmoleküle aus verschiedenen Spezies, zB. TLR_{S}, auch kreuzreaktiv; d.h. obwohl ein Antikörper gegen ein Zielmolekül von einer Spezies entwickelt wurde, zeigt er seine Eigenschaften auch gegen ein ähnliches Zielmolekül aus einer anderen Spezies. Weiterhin werden bevorzugt Antikörper aus Zellen derselben Spezies, die transfiziert werden soll, eingesetzt, da sie in diesem Falle wenig oder nicht immunogen sind. Die Antikörper können auch rekombinant hergestellt worden sein. Sollen sie an menschlichen Zellen eingesetzt werden, können sie "humanisiert" sein. Vorzugsweise bindet der eingesetzte Antikörper hochaffin an sein Zielmolekül. Der verwendete Antikörper kann polyklonal oder monoklonal sein, wobei monoklonale Antikörper bevorzugt sind. Es kann auch ein modifizierter Antikörper eingesetzt werden.beispielsweise kann bei einem modifizierten Antikörper das Fc-Fragment fehlen, da die Bindung an das Zielmolekül/Antigen ausschliesslich durch die Fab-Fragmente zustande kommt. Ein modifizierter Antikörper kann auch mit hydrophoben Gruppen, wie z.B. Lipiden, modifiziert sein, um seine Verankerung in Membranen und/oder Liposomen zu erleichtern. Will man den eingesetzten Antikörper leichter detektieren, kann er auch mit einem Fluoreszenzfarbstoff gelabelt sein. Es können auch mehrere Modifikationen an einem Antikörper vorgenommen werden. Weiter muss der eingesetzte Antikörper frei von Zusatzstoffen sein, die eine Anwendung an lebenden Zellen verbieten, wie zB. bestimmte Konservierungstoffe.

Bei dem erfindungsgemäßen Verfahren kann ferner zumindest eine der Kinasen IRF-Kinase TBK1, MAP Kinase, MAPK Kinase, MAPK Kinase, MAPKK Kinase, MAPKKK Kinase, MEK1, MEK2, MEK5, MKK4/SEK, MKK5, MKK6, MKK7, ERK1, ERK2, ERK3, ERK4, ERK5, ERK6, ERK7, ERK8, JAK, JNK1, JNK2, p38 MAP Kinase, RK, p38/RK MAP Kinase, p30/RK MAP Kinase, Phosphatidylinositol 3-Kinase, IRAK-1, IRAK-4, IKK-alpha, IKK-beta, IKKgamma, IKK delta, IKK epsilon, TAK1, PKB Kinase, PKD1, PKD2, MSK1 oder PKR blockiert werden. Bevorzugt wird mindestens eine Kinase, ausgewählt aus MEK1 und MEK2, blockiert. Weiterhin ist es bevorzugt, mehrere der vorstehend genannten Kinasen zu blockieren.

Weiterhin kann erfindungsgemäß die Kinase MEK1 und/oder MEK2 durch eine Verbindung inhibiert werden, die einen 1C50 Wert kleiner 100 nM aufweist. Weiterhin kann erfindungsgemäß die Kinase MEK1 und/oder MEK2 durch 1,4-Diamino-2,3-dicyano-1,4-bis(o-amionophenylmercapto)butadien (U0126) blockiert werden. In einer bevorzugten Ausführungsform kommt dabei eine Konzentration von 1-500 _{N}M zur Anwendung. In einer noch mehr bevorzugten Ausführungsform beträgt die Konzentration 1-100pM und in der am meisten bevorzugten Ausführungsform 1-30µM.

Bei dem erfindungsgemäßen Verfahren kann auch zumindest ein Zytokin, zumindest ein Tumomekrosefaktor (TNF), zumindest ein Interleukin und/oder zumindest ein Interferon blockiert werden, die bei der angeborenen interzellulären Immunabwehr beteiligt sind.

Als zu blockierendes Interferon kommt beispielsweise ein Interferon des Typs 1, insbesondere zumindest eines der Interferone, ausgewählt aus Interferon-alpha, Interferon-beta, Interferon-gamma oder Interferon-omega in Betracht. Als zu blockierendes Interleukin kommt insbesondere Interleukin-1 in Betracht.

Auch kann zumindest ein Rezeptor für Zytokine blockiert werden, insbesondere zumindest ein Rezeptor für Interferone, insbesondere des Typs 1, zumindest ein Rezeptor für Interleukine, und/oder zumindest ein Rezeptor für Tumornekrosefaktoren. Ein Beispiel für einen geeigneten Antikörper der gegen den Interferon-Typ-1-Rezeptor gerichtet ist, ist der Mouse monoclonal Antibody against Human Interferone Alpha/Beta Receptor Chain 2 (CD118), clone MMHAR-2, Isotype 1g2a, C= 0,5 mg/ml in PBS (Phosphat buffered saline) containing 0,1 % bovine serum albumin (BSA), PBL Biomedical Laboratories, Product-No: 21385. Insbesondere kann der Rezeptor für Interleukin-1 durch einen Antikörper oder einen Antagonisten wie IL-ra (Human Interleukin-1 Receptor Antagonist, Biomol, Kat.-Nr.: 54592) blockiert werden. Ein weiteres bevorzugtes Ziel ist der Interferon-gamma-Rezeptor.

Für alle Antagonisten, die erfindungsgemäß als Mittel zur zumindest teilweisen Unterdrückung der angeborenen intrazellulären und/oder interzellulären Immunabwehr eingesetzt werden können, gilt: Der oder die eingesetzten Antagonist(en) muss/müssen in der Regel gegen das zu blockierende Ziel molekül, zB. einen Rezeptor wie einen Interleukinrezeptor, Zytokinrezeptor, lnterferonrezeptor, usw., der Zellen gerichtet sein, die transfiziert werden sollen. Handelt es sich beispielsweise um einen Rezeptor einer humanen Zelle, so muss der Antagonist sich in der Regel gegen den humanen Rezeptor richten, der blockiert werden soll. In manchen Fällen sind die Antagonisten wegen der großen Ähnlichkeit der Zielmoleküle aus verschiedenen Spezies, zB. TLR_{S}, auch kreuzreaktiv; d.h. obwohl ein Antagonist gegen ein Zielmolekül von einer Spezies entwickelt wurde, zeigt er seine Eigenschaften auch gegen ein ähnliches Zielmolekül aus einer anderen Spezies. Weiterhin werden bevorzugt Antagonisten aus Zellen derselben Spezies, die transfiziert werden soll, eingesetzt, da sie in diesem Falle wenig oder nicht immunogen sind. Die Antagonisten können auch rekombinant oder synthetisch hergestellt worden sein. Vorzugsweise bindet der eingesetzte Antagonist hochaffin an sein Zielmolekül. Erfindungsgemäß eingesetzte Antagonisten können auch modifiziert sein; analog zu den modifizierten Antikörpern.

In einer bevorzugten Ausführung der Erfindung werden mehrere der vorstehend erwähnten Rezeptoren und/oder Proteine, die an der Signaltransduktionskaskade zur Auslösung der angeborenen intra- und/oder interzellulären Immunabwehr beteiligt sind, blockiert. So können beispielsweise mehrere Antikörper gegen TLR-Rezeptoren kombiniert werden, um additive Effekte und/oder Synergieeffekte zu nutzen. Ebenso können beispielsweise auch Inhibitoren und/oder Antikörper, und/oder Intrabodys, und/oder Aptamere, und/oder Antagonisten, und/oder siRNA gegen TLR-Rezeptoren und/oder TLR assistierende Proteine und/oder Adaptermoleküle und/oder Kinasen und/oder Transkriptionsfaktoren und/oder Zytokine und/oder Zytokinrezeptoren kombiniert werden, um die Signaltransduktionskaskade der angeborenen Immunabwehr zumindest teilweise zu unterbrechen.

Das erfindungsgemäße Verfahren kann ferner dazu benutzt werden, die Transfektionsergebnisse von siRNA Transfektionen mit nicht-viralen Genliefersystemen auf mehrerlei Weise zu verbessern. Dabei kann die siRNA modifiziert oder unmodifiziert sein.

Bei dem erfindungsgemäßen Verfahren kann als Mittel zur Aktivierung der angeborenen Immunabwehr ein Agonist eingesetzt werden.

Zum Einen kann das erfindungsgemäße Verfahren dazu genutzt werden durch eine Anregung des intrazellulären Teils des angeborenen Immunsystems die RNA-Interferenzmaschinerie zu aktivieren. Die Aktivierung gelingt durch die Belegung verschiedener TLR Rezeptoren mit entsprechenden Agonisten, insbesonders aber durch Belegung (und damit Aktivierung) der Rezeptoren TLR7 und TLR8. Die Rezeptoren TLR7 und TLR8 befinden sich in den Endosomen und detektieren ssRNA, wie sie bei einer Infektion eines RNA-Virus zu erwarten ist. Die Aktivierung dieser Rezeptoren führt zu einem besonders starken "antiviralen Status" zu dem eine aktive RNA-Interferenzmaschinerie zugeordnet werden kann. Die Annahme, dass es sich bei der RNA-Interferenz um einen Mechanismus zur Abwehr von Viren handelt fügt sich zwanglos in das Gesamtbild. Die Verbesserung der Transfektionsergebnisse von siRNA Transfektionen weicht insofern von den Transfektionen mit anderem genetischen Material ab, dass für einen guten Transfektionserfolg eine aktive RNAi-Maschinerie zur Verfügung stehen muss, die Bestandteil des angeborenen Immunsystems ist. Dabei überkompensiert die aktive RNAi-Maschinerie den negativen Einfluss des angeborenen Immunsystems auf die Aufnahme der siRNA.

Bevorzugt kann bei dem erfindungsgemäßen Verfahren die angeborene intrazelluläre und/oder interzelluläre Immunabwehr durch mindestens einen Agonisten für einen TL-Rezeptor, insbesondere durch mindestens einen Agonisten für TLR7 und/oder TLR8, zumindest teilweise aktiviert werden.

Erfindungsgemäß kann der mindestens eine Agonist für TLR7 und/oder TLR8 ausgewählt werden aus der Gruppe, umfassend Bropirimine (2-amino-5-bromo-6-phenyl-4-pyrimidinone), Imidazochinoline, Thiazolochinoline, Guanosinanaloga und ssRNA.

Bevorzugt kann bei dem erfindungsgemäßen Verfahren der mindestens eine Agonist Imiquimod (R837, 1-(2-methylpropyl)-1H-imidazol[4,5-c]quinolin-4-amine), Resiquimod (R848, 4-amino-2-(ethoxymethyl)-a,a-dimethyl-1H-imidazo[4,5-c]quinoline-1-ethanol) oder Gardiquimod (1-(4-amino-2-ethylamino methylimidazo-[4,5-c]quinolin-1-yl)-2-methylpropan-2-ol); oder CL075 oder CL097; oder Loxoribine (7-allyl-7,8-dihydro-8-oxo-guanosin) oder Isatoribine (7-Thia-8-oxoguanosin); oder ssRNA mit U und/oder GU reichen Sequenzen, insbesondere ssRNA mit den Sequenzmotiven UGUGU und/oder GUCCUUCAA, sein. Imiquimod kann in einer Konzentration von 0,1-100 µg/ml, bevorzugt in einer Konzentration von 0,1-20 µg/ml, und am meisten bevorzugt in einer Konzentration von 0,1-10 µg/ml eingesetzt werden. Die Zugabe findet bevorzugt zur gleichen Zeit wie die Transfektion und/oder später statt. Vorzugsweise hat die ssRNA eine Länge von mindestens 15 Basen. Ferner kann die ssRNA ein Phosphothioatrückgrat haben. Beispielsweise kann eine ssRNA mit 20 Nukleotiden Länge in einer Konzentration von 0,1-100 µg/ml, bevorzugt 0,1-20 µg/ml, und am meisten bevorzugt 0,1-10 µg/ml eingesetzt werden. Die Zugabe der ssRNA findet bevorzugt zur gleichen Zeit wie die Transfektion und/oder nach der Transfektion statt. Bevorzugt kann die immunstimulierende ssRNA in dem transfektionsaktiven Komplex aus nicht-viralem Genliefersystem und siRNA oder Plasmid-DNA, auf der shRNA codiert ist, enthalten sein.

Die zellgängigen Agonisten können vor, während oder nach dem eigentlichen siRNA-Transfektionsschritt direkt zum Medium der Zellen gegeben werden. Die ssRNA und deren Analoga müssen dabei mit geeigneten Transfektionsreagentien komplexiert werden, da sie von Haus aus nicht zellgängig sind und damit die in den Endosomen liegenden TLRs nicht erreichen. Es ist auch möglich die Agonisten in Lipo- oder Polyplexe mitsamt der siRNA miteinzubauen oder durch Derivatisierung mit Alkylketten die Agonisten in die Lipidmembranen der Liposomen bzw. Lipoplexe miteinzubauen, die aus den eigentlichen transfektionsaktiven kationischen Lipiden und möglicher Colipide wie zB. DOPE bestehen. Auch können die Agonisten kovalent an kationische Polymere gebunden werden.

Zum Zweiten kann das erfindungsgemäße Verfahren dazu genutzt werden durch eine Anregung des interzellulären Teils des angeborenen Immunsystems die RNA-Interferenzmaschinerie zu aktivieren. Die Aktivierung gelingt über die Belegung von Rezeptoren von antiviralen Zytokinen mit geeigneten Agonisten. In einer bevorzugten Ausführung kommt dabei Interferon beta und/oder Interferon gamma in einer Konzentration von 1-10000 U/ml zur Anwendung. In einer noch mehr bevorzugten Ausführungsform beträgt die Konzentration 1-5000 und in der am meisten bevorzugten Ausführungsform 1-2000 U/ml. Die Zugabe findet bevorzugt zur gleichen Zeit wie die Transfektion und/oder später statt.

Zum Anderen kann das erfindungsgemäße Verfahren dazu genutzt werden, Teile des adaptiven Immunsystems zu stimulieren, die für die Aktivierung der siRNA-Maschinerie geeignet sind und gleichzeitig andere Teile zu blockieren, die zB. durch eine Herunterregelung der Endozytose Einfluss auf die Menge der eingetragenen siRNA haben. Dabei können Synergieffekte genutzt werden.

Bei dem erfindungsgemäßen Verfahren können die Zellen bis zu 4 Tage, vorzugsweise bis zu 18 Stunden, insbesondere bis zu 6 Stunden vor der Transfektion mit dem mindestens einen Mittel zur zumindest teilweisen Aktivierung der angeborenen intrazellulären und/oder interzellulären lmmunabwehr behandelt werden.

Ferner können bei dem erfindungsgemäßen Verfahren die Zellen bis zu 2 Tage, vorzugsweise bis zu 12 Stunden, insbesondere bis zu 6 Stunden nach der Transfektion mit dem mindestens einen Mittel zur zumindest teilweisen Aktivierung der angeborenen intrazellulären und/oder interzellulären Immunabwehr behandelt werden.

Weiterhin können bei dem erfindungsgemäßen Verfahren die Zellen gleichzeitig mit dem mindestens einen Mittel zur zumindest teilweisen Unterdrückung oder Aktivierung der angeborenen intrazellulären und/oder interzellulären lmmunabwehr behandelt werden und mit dem nicht-viralen Genliefersystem in Kontakt gebracht werden.

Das erfindungsgemäße Verfahren kann weiters dazu genutzt werden eine unerwünschte Reaktion des angeborenen Immunsystems zu verhindern und damit eine veränderte Genexpression der Zelle. Dies ist insbesondere bei in vivo Anwendungen und bei der Aufklärung von Proteinfunktionen durch siRNA Transfektionen in komplexen Signal-Pathways von besonderer Bedeutung. Häufig sind verschiedene Signaltransduktionswege der Zelle auch mit den Signaltransduktionswegen des angeborenen Immunsystems gekoppelt. Bei einem Knockdown eines Proteins, der gleichzeitig den physiologischen Status der Zelle massiv beeinflusst, ist die Zuordung der Funktion zum Protein erschwert. Um dies zu vermeiden kann das erfindungsgemäße Verfahren dazu genutzt werden, die Antwort des angeborenen Immunsystems teilweise oder vollständig zu blockieren.

Das erfindungsgemäße Verfahren eignet sich zur Transfektion von eukariotischen Zellen. Werden die eukariotischen Zellen *in vitro* transfiziert, können die Zellen adhärent oder in Suspension in einem geeigneten Kulturmedium vorliegen. Bevorzugt befinden sich die Zellen zum Zeitpunkt der Transfektion in der logarithmischen Phase der Proliferation, falls es sich um eine Transfektion mit DNA handelt. Soll RNA transfiziert werden, befinden sich die Zellen zum Zeitpunkt der Transfektion bevorzugt in der lag oder log-Phase. Soll eine Transfektion mit dem Ziel durchgeführt werden, ein Protein zu exprimieren, kommt als genetisches Material dsDNA mit einem als RNA oder Peptid/Protein exprimierbaren Anteil oder ssRNA mit einem als Peptid/Protein exprimierbaren Anteil (jeweils modifiziert oder unmodifiziert) in Betracht.

Soll eine Transfektion mit dem Ziel durchgeführt werden, durch RNA-Interferenz einen Knock-down eines Gens zu erreichen, kann modifizierte oder unmodifizierte dsDNA mit einem als small hairpin-RNA (shRNA) exprimierbaren Anteil oder modifizierte oder unmodifizierte siRNA eingesetzt werden; nur in diesem Falle ist eine Aktivierung der TLR 7 und/oder TLR 8 sinnvoll. Jedoch kann eine zusätzliche Blockierung von TLR und/oder Zytokinrezeptoren und/oder die Unterbrechung einer Signaltransduktion durch Blockierung von MEK1 und/oder MEK2 vorteilhaft sein, insbesondere um das Expressionsprofil der Zellen möglichst nicht zu beeinflussen.

Ein erfindungsgemäßes Verfahren kann bevorzugt folgende Verfahrensschritte aufweisen:
(a) Bereitstellen einer ersten Lösung mit einem Antikörper, Antagonisten, Inhibitor oder Agonisten gegen ein Zielmolekül; bereitstellen einer zweiten Lösung mit einem nicht-viralen Genliefersystem; und bereitstellen einer dritten Lösung mit dem zu transfizierenden genetischen Material;
(b) Zugabe der ersten Lösung, enthaltend einen Antikörper, Antagonisten oder Inhibitor gegen das Zielmolekül, zu den zu transfizierenden Zellen im Kulturmedium;
(c) Mischen der zweiten Lösung mit dem nicht-viralen Genliefersystem und der dritten Lösung mit dem zu transfizierenden genetischen Material
(d) Inkubation des Gemisches aus Schritt (c);
(e) Zugabe des inkubierten Gemisches aus Schritt (d) zu den mit der ersten Lösung vorbehandelten zu transfizierenden Zellen aus Schritt (b);

Erfindungsgemäß kann die erste Lösung einen Antagonisten oder Antikörper gegen einen Zytokinrezeptor oder TLR 1, TLR 2, TLR 3, TLR 4, TLR 5, TLR 6, TLR 7, TLR 8, oder TLR 9 als Zielmolekül; einen Inhibitor für das Zielmolekül MEK 1 und/oder MEK 2; oder einen Agonisten für TLR 7 und/oder TLR 8 enthalten; insbesondere einen oben genannten derselben. Ein Antagonist oder Antikörper kann bevorzugt in einer gepufferten Salzlösung, z.B. PBS, Basalmedium, etc., mit einem physiologischen pH-Wert 6,8-7,45 und einer physiologischen Osmolalität 270-310 mosmol/kgH₂O, oder auch in Wasser oder in ungepufferter Salzlösung mit einem pH-Wert von 5-9 gelöst werden. Der Inhibitor kann bei Löslichkeitsproblemen auch in einem geeigneten physiologisch unbedenklichen Solvent, z.B. Ethanol/DMSO verdünnt werden. Die Konzentration des Antagonisten, Antikörpers oder Inhibitors in der ersten Lösung kann 0,01-1 µg/µl betragen.

Erfindungsgemäß kann die Zugabe der ersten Lösung zu den zu transfizierenden Zellen im Kulturmedium im Schritt (b) im Zeitraum 24 h bis 1 s vor der Behandlung der Zellen mit dem Transfektionskomplex (Schritt (e)) erfolgen, bevorzugt im Zeitraum 0,5-5 h. Dies gilt insbesondere, wenn die erste Lösung einen MEK 1- und/oder MEK 2-Inhibitor, oder einen gegen TLR 1, TLR 2, TLR 3, TLR 4, TLR 5, oder TLR 6 oder Zytokinrezeptor gerichteten Antikörper oder Antagonisten enthält. Die Zugabe erfolgt, indem eine entsprechende Menge der ersten Lösung zum Kulturmedium der zu transfizierenden Zellen hinzugegeben wird. Die resultierende Konzentration des Antikörpers oder Antagonisten im Kulturmedium der zu transfizierenden Zellen kann von 0,01 _{N}g/ml bis 50 pg/ml, bevorzugt 0,05-12 µg/ml, insbesondere 0,1-5 _{N}g/ml betragen. Die resultierende Konzentration des Inhibitors im Kulturmedium der zu transfizierenden Zellen kann 1 nM bis 500 _{N}M, bevorzugt 5-100 _{N}M, insbesondere 10-50 _{N}M betragen.

Bei einem erfindungsgemäßen Verfahren kann das Mischen der zweiten Lösung mit dem nicht-viralen Genliefersystem und der dritten Lösung mit dem zu transfizierenden genetischen Material (Schritt (c)) durch pipettieren erfolgen. Pro zu transfizierende Zelle kann an zu transfizierendem genetischen Material 0,1 Picogramm/Zelle bis 300 Picogramm/Zelle, bevorzugt 0,2-50 Picogramm/Zelle, insbesondere 1-10 Picogramm/Zelle zu transfizierendes genetisches Material eingesetzt werden. Die Menge des nicht-viralen Genliefersystems richtet sich nach der Menge des genetischen Materials. Im Falle einer elektrostatischen Bindung des nicht-viralen Genliefersystems an das genetische Material definiert sich die Menge durch das Ladungsverhältnis (+/-) zwischen Genliefersystem und genetischem Material. Das Ladungsverhältnis (+/-) Genliefersystem : genetisches Material kann 0,1:1 bis 100:1, bevorzugt 1:1 bis 20:1, insebsondere 4:1 bis 10:1 betragen. Im Falle einer nicht-elektrostatischen Bindung definiert sich die Menge des nicht-viralen Genliefersystems über das stöchiometrische Verhältnis zum genetischen Material. Das stöchiometrische Verhältnis nicht-virales Genliefersystem : genetisches Material kann von 0,1:1 bis 1000:1, bevorzugt 1:1 betragen.

Die Mischung der zweiten Lösung mit dem nicht-viralen Genliefersystem und der dritten Lösung mit dem zu transfizierenden genetischen Material kann für einen Zeitraum von 1 min bis 30 min, bevorzugt 10-15 min inkubiert werden.
Vorzugsweise kann bei dem erfindungsgemäßen Verfahren ein nicht-virales Genliefersystem eingesetzt werden, das ein kationisches Lipid umfasst, insbesondere ein vorstehend erwähntes kationisches Lipid gemäß der Formel (1).

Erfindungsgemäß können nach 24 h die Schritte (a) bis (e) wiederholt werden, um die Zellen ein weiteres Mal mit genetischem Material zu transfizieren. Vorzugsweise sollte vor einer zweiten oder weiteren Transfektion das Kulturmedium mit Additiven, in dem sich die zu transfizierenden Zellen befinden, durch frisches Kulturmedium ersetzt werden.

Bei einem erfindungsgemäßen Verfahren kann eine erste Lösung mit einem Antagonisten oder Antikörper gegen TLR 3, TLR 7, TLR 8 oder TLR 9 mit einer zweiten Lösung mit einem nicht-viralen Genliefersystem und einer dritten Lösung mit dem zu transfizierenden genetischen Material gemischt werden. Vorzugsweise wird die erste Lösung mit einem Antikörper/Antagonisten zur zweiten Lösung mit einem nicht-viralen Genliefersystem gegeben und gemischt. Die eingesetzte Antikörpermenge beträgt 0,1 Gewichts-% bis 50 Gewichts-% bezogen auf die Menge des nicht-viralen Genliefersystems, bevorzugt 0,1 bis 10 Gewichts-%. Die Menge des nicht-viralen Genliefersystems richtet sich nach der Menge des genetischen Materials das verwendet werden soll. Im Falle einer elektrostatischen Bindung des nicht-viralen Genliefersystems an das genetische Material definiert sich die Menge durch das Ladungsverhältnis (+/-) zwischen Genliefersystem und genetischem Material. Das Ladungsverhältnis (+/-) Genliefersystem : genetisches Material kann 0.1:1 bis 100:1, bevorzugt 1:1 bis 20:1, insebsondere 4:1 bis 10:1 betragen. Im Falle einer nicht-elektrostatischen Bindung definiert sich die Menge des nicht-viralen Genliefersystems über das stöchiometrische Verhältnis zum genetischen Material. Das stöchiometrische Verhältnis nicht-virales Genliefersystem : genetisches Material kann von 0,1:1 bis 1000:1, bevorzugt 1:1 1 betragen. Die Mischung aus erster und zweiter Lösung wird bevorzugt mindestens 5 min inkubiert. Anschließend wird die dritte Lösung mit zu transfizierendem genetischen Material zu der inkubierten Mischung aus erster und zweiter Lösung zugegeben und es wird gemischt. Pro zu transfizierende Zelle kann an zu transfizierendem genetischen Material 0,1 Picogramm/Zelle bis 300 Picogramm/Zelle, bevorzugt 0,2-50 Picogramm/Zelle, insbesondere 1-10 Picogramm/Zelle zu transfizierendes genetisches Material eingesetzt werden. Dabei wird der Antikörper/Antagonist in den transfektionsaktiven Komplex aus nicht-viralem Genliefersystem und genetischem Material eingebaut. Bevorzugt werden Antikörper/Antagonisten eingesetzt, die mit einem lipophilen Molekülteil modifiziert sind, um die Bindung des Antikörpers/Antagonisten in den Lipoplex zu erleichtern. Es können auch Antikörper eingesetzt werden, die mit dem nicht-viralen Genliefersystem über Avidin/Streptavidin und Biotin verknüpft sind. In diesem Fall müssen das Genliefersystem und der Antikörper entsprechend vorher verändert werden. Auch kann ein gegen TLR 3, TLR 7, TLR 8 oder TLR 9 gerichteter Antikörper kovalent mit dem nicht-viralen Genliefersystem verknüpft sein.

Methoden, kationische lmmunoliposomen aus Liposomen, die kationische Lipide umfassen oder Lipoplexe, d.h. DNA und kationische Lipide enthaltende Komplexe, kovalent mit Antikörpern oder Antikörperfragmenten zu versehen, sind bekannt. Die Verfahren wurden entwickelt, um ein Targeting der Genliefersysteme in Richtung der Zielzellen im Körper zu ermöglichen. Die meisten Methoden nutzen sogenannte Cross-Linker. Cross-Linker sind bifunktionale Moleküle, die eine kovalente Verknüpfung zwischen zwei Molekülen mit entsprechenden funtionellen Gruppen erstellen. Beispielsweise bietet die Firma Pierce eine breite Auswahl von wasserlöslichen und wasserunlöslichen heterobifunktionalen (also für die Verknüpfung zweier unterschiedllicher funktioneller Gruppen geeigneter) und homobifunktionalen (für die Verknüpfung zweier gleicher funktioneller Gruppen geeigneter) Cross-Linker an. Für die Verknüpfung der Antikörper können Carboxyl- und Aminogruppen genutzt werden; bei Fab-Fragmenten kann auch die Thiolgruppe für eine Verknüpfung genutzt werden. Kationische Lipide und Polymere enthalten Aminofunktionen zur Verknüpfung. Bei kationischen Peptiden können Carboxyl- und Aminogruppen für die Verknüpfung genutzt werden.

Kationische Immunoliposomen können wie herkömmliche Liposomen formuliert werden, indem man Antikörper mit Lipiden derivatisiert und zu den kationischen Lipiden (ev. mit Colipiden) vor der Formulierung zugibt. Für die kovalente Verknüpfung durch homobifunktionale Cross-linker, wie zB. DSP (dithiobis[succinimidylpropionat]), können Aminofunktionen genutzt werden, um ein Lipid mit einer Aminofunktion mit einem Antikörper kovalent zu verknüpfen. Zur kovalenten Verknüpfung eines Fab-Fragments mit einem Lipid kann auch eine Thiolfunktion und der Cross-linker N-succinimidyl-4-(p-maleimidophenyl)butyrat (Martin et al.; J. Biol. Chem.; 1982 257(1), 286) oder SPDP (N - Succinimidyl 3-(2-pyridyldithio)-propionat) genutzt werdendar. Sofern die Crosslinker in Wasser nicht löslich sind, müssen diese Kupplungsreaktionen zwischen Antikörper und Lipid in organischen Lösungsmitteln durchgeführt werden und vor einer Transfektion eine Reinigung, d.h. Entfernung des organischen Lösungsmittels erfolgen. Zur Reinigung kann Dialyse eingesetzt werden. Ist der Crosslinker in Wasser löslich, z.B. DSP, kann eine kovalente Verknüpfung zwischen dem Lipid und dem Antikörper, z.B. Verknüpfung über Aminofunktionen, in wässriger Lösung erfolgen und eine Reinigung vor der Transfektion ist nicht zwingend nötig.

Verfahren zur Darstellung solcher Lipoplexe mit modifizierten Antikörpern, die kovalent an ein Lipid gebunden sind, sind dem Fachmann bekannt. Die Lipoplexe nehmen dann die modifizierten Antikörper auf (Lee et al.; J. Biomed. Sci.; 2003; 10; 337). Ebenso bekannt sind Methoden um kationische Polymere, wie zB. PEI oder Dendrimere oder kationische Proteine, wie zB. Polylysin (Chen et al.; FEBS Letters; 1994; 338; 167; Suh et al.; J. Controlled Release; 2001; 72; 171) kovalent mit Antikörpern oder Antikörperfragmenten zu verknüpfen. PEI wird dabei zB. mit DPS in DMSO zur Reaktion gebracht und zu einer Antikörperlösung in PBS zugegeben. Nach einer Dialyse steht das mit Antikörper gekuppelte nicht-virale Genliefersystem zur Verfügung (Chiu et al.; J. Controlled Release; 2004); 97; 357).

Bei einem erfindungsgemäßen Verfahren kann die erste Lösung einen Agonisten zur Aktivierung des TLR 7 und/oder TLR 8 enthalten, insbesondere einen der vorstehend angeführten. Der Agonist kann in einer gepufferten Salzlösung, z.B. PBS, Basalmedium, etc, mit einem physiologischen pH-Wert 6,8-7,45 und einer physiologischen Osmolalität 270-310 mosmol/kgH₂O, in Wasser oder in einer ungepufferten Salzlösung mit einem pH-Wert von 5-9 gelöst sein. Die Konzentration des Agonisten kann 0,01-1 µg/µl betragen. Diese erste Lösung mit einem TLR 7 und/oder TLR 8 Agonisten kann vor, während oder nach der Transfektion zu den zu transfizierenden Zellen im Kulturmedium zugegeben werden. Der Zeitpunkt der Agonistenbehandlung sollte in Abhängigkeit der Natur des Agonisten so gewählt werden, daß ein möglichst aktives angeborenes Immunsystem auf eine möglichst hohe Anzahl an siRNA trifft. Bevorzugt erfolgt die Zugabe der ersten Lösung mit dem Agonisten gleichzeitig mit der Zugabe des Transfektionskomplexes zu den zu transfizierenden Zellen, indem eine entsprechende Menge an erster Lösung zum Kulturmedium hinzugegeben wird. Die Agonistenmenge richtet sich nach der Zellmenge. Bevorzugt werden 0,1 pg Agonist /Zelle bis 25 ng Agonist/Zelle, bevorzugt 1-500 pg Agonist/Zelle, insbesondere 10 bis 250 pg Agonist/Zelle eingesetzt.

Erfindungsgemäß kann ein efindunsgsgemäßes Verfahren, d.h. eine Transfektion, auch *in* vivo erfolgen, wobei die Verfahrensschritte den jeweiligen Verfahrensschritten einer *in vitro* Transfektion entsprechen, mit der Ausnahme, dass die Zugabe der jeweiligen Lösungen oder Mischungen peroral (p.o.), percutan, sublingual (s.1.), nasal, intravenös (i.v.), intraartikulär, intraarteriell (i.a.), intralymphatisch, intramuskulär (i.m.), intraossär (i.o.), subkutan (s.c.), intrakutan (i.c.), transdermal, rektal, vaginal, inhalativ (p.i. = per inhalation), intrapulmonal, endobronchial (e.b.), intraperitoneal (i.p.), intrakardial, intraneural, perineural, peridural, intrathekal, intrapleural, intravitreal, parenteral, enteral oder buccal erfolgt. Die Menge des genetischen Materials richtet sich nach der Art des genetischem Materials, nach dem Zielkompartiment, z.B. Blut, extrazelluläre Flüssigkeit der Gewebe, Zellgewebe, etc., der Applikationsform und der Art der Zugabe, z.B. Infusion, Injektion, oder Inhalation, und sie beträgt 0,01-500 mg/kg Körpergewicht. Die Menge an Antikörper, Antagonist oder Agonist bei einer direkten Verabreichung einer ersten Lösung an ein Individuum kann 0,1 mg/kg bis 500 mg/kg Körpergewicht, bevorzugt 1-100 mg/kg, insbesondere 5-10 mg/kg betragen. Die Menge an Inhibitor bei einer direkten Verabreichung einer ersten Lösung an ein Individuum kann 0,1 mg/kg bis 500 mg/kg Körpergewicht, bevorzugt 10-300 mg/kg, insbesondere 100-200 mg/kg betragen. Der Zeitpunkt und die Dauer der Zugabe einer ersten Lösung richtet sich nach dem Zielkompartiment, z.B. Blut, extrazelluläre Flüssigkeit der Gewebe, Zellgewebe, etc., der Applikationsform und der Verabreichungsart, z.B. Infusion, Injektion, Inhalation.

Bei in vivo Anwednungen kann das Zeitintervall zwischen zwei Transfektionen bis zu 6 Wochen betragen.

Erfindungsgemäß wird ferner eine Zusammensetzung bereitgestellt, die zumindest zwei der folgenden Komponenten umfasst:
a) ein nicht-virales Genliefersystem,
c) genetisches Material, und
b) ein Mittel zur zumindest teilweisen Unterdrückung oder Aktivierung der angeborenen intrazellulären und/oder interzellulären Immunabwehr.

Auch wird erfindungsgemäß ein Kit of parts vorgesehen, der zumindest zwei der folgenden Komponenten umfasst:
a) ein nicht-virales Genliefersystem,
c) genetisches Material, und
b) ein Mittel zur zumindest teilweisen Unterdrückung oder Aktivierung der angeborenen intrazellulären und/oder interzellulären Immunabwehr.

Erfindungsgemäß umfasst das nicht-virale Genliefersystem insbesondere ein kationisches Lipid, ein kationisches Polymer, ein kationisches Protein; und/oder eine Verbindung, die eine DNA und/oder RNA-bindende Domäne aufweist und rezeptorvermittelte Endozytose oder einen Membrantransfer auslösen kann; und/oder eine Verbindung, die kovalent an DNA und/oder RNA gebunden ist und rezeptorvermittelte Endozytose oder einen Membrantransfer auslösen kann.

Erfindungsgemäß kann als genetisches Material beispielsweise genetisches Material zur Reparatur eines Gendefekts, z.B. genetisches Material, insbesondere modifizierte oder unmodifizierte ssDNA, modifizierte oder unmodifizierte dsDNA, modifizierte oder unmodifizierte ssRNA, modifizierte oder unmodifizierte dsRNA und/oder modifizierte oder unmodifizierte siRNA eingesetzt werden.

Erfindungsgemäß kann als Mittel zur zumindest teilweisen Unterdrückung und/oder Aktivierung der angeborenen intrazellulären und/oder interzellulären Immunabwehr zumindest ein aktivitätsmindernder oder aktivitätssteigernder Effektor eingesetzt werden.

Weiterhin kann erfindungsgemäß als Mittel zur zumindest teilweisen Unterdrückung der angeborenen intrazellulären und/oder interzellulären Immunabwehr ein Antikörper, Intrabody, Aptamer, Antagonist, Inhibitor und/oder eine siRNA eingesetzt werden.

Bevorzugt kann der/die als Mittel zur zumindest teilweisen Unterdrückung der angeborenen intrazellulären und/oder interzellulären Immunabwehr verwendete Antikörper, Intrabody, Aptamer, Antagonisten, Inhibitor und/oder siRNA zumindest eine Signaltransduktionskaskade des angeborenen Immunsystems blockieren.

Weiterhin kann erfindungsgemäß als Mittel zur zumindest teilweisen Aktivierung der angeborenen intrazellulären und/oder interzellulären Immunabwehr ein Agonist eingesetzt werden. '

Bevorzugt kann der/die als Mittel zur zumindest teilweisen Aktivierung der angeborenen intrazellulären und/oder interzellulären Immunabwehr verwendete Agonist zumindest eine Signaltransduktionskaskade des angeborenen Immunsystems aktivieren.

Auch kann das erfindungsgemäße Mittel zur zumindest teilweisen Unterdrückung der angeborenen intrazellulären und/oder interzellulären Immunabwehr genetisches Material umfassen, das einen Knock-down eines Proteins einer Signaltransduktionskaskade des angeborenen Immunsystems bewirken kann.

Weiterhin kann das erfindungsgemäße Mittel zur zumindest teilweisen Unterdrückung der angeborenen intrazellulären und/oder interzellulären Immunabwehr genetisches Material umfassen, das zur Expression eines Proteins führen kann, welches die Aktivität eines Proteins in einer Signaltransduktionskaskade des angeborenen Immunsystems blockieren kann.

Bevorzugt kann eine erfindungsgemäße Zusammensetzung für eine Transfektion (a) ein nicht-virales Genliefersystem und (b) ein Mittel zur zumindest teilweisen Unterdrückung und/oder Aktivierung der angeborenen intrazellulären und/oder interzellulären Immunabwehr umfassen, wobei das nicht-virale Genliefersystem:
(i) ein kationisches Lipid, ein kationisches Polymer, oder ein kationisches Protein umfassen kann; und/oder
(ii) eine Verbindung umfassen kann, die eine DNA und/oder RNA-bindende Domäne aufweist und rezeptorvermittelte Endozytose oder einen Membrantransfer auslösen kann; und/oder
(iii) eine Verbindung umfassen kann, die kovalent an DNA und/oder RNA gebunden ist und rezeptorvermittelte Endozytose oder einen Membrantransfer auslösen kann; und
das Mittel zur zumindest teilweisen Unterdrückung und/oder Aktivierung der angeborenen intrazellulären und/oder interzellulären lmmunabwehr, ausgewählt sein kann aus:
(i) einem Antikörper gegen TLR 1, TLR 2, TLR 3, TLR 4, TLR 5, TLR 6, TLR 7, TLR 8, TLR 9, TLR 10, TLR 11,TLR 12, oder TLR 13;
(ii) einem Antikörper gegen einen Zytokinrezeptor oder einem Zytokinrezeptor-Antagonisten;
(iii) einem Inhibitor der Kinase MEK1 und/oder MEK2;
(iv) einem Agonisten für TLR7 und/oder TLR8, ausgewählt aus der Gruppe, umfassend Bropirimine (2-amino-5-bromo-6-phenyl-4-pyrimidinone), Imidazochinoline, Thiazolochinoline, und Guanosinanaloga; und
(v) einer Kombination derselben.

Ein erfindungsgemäßer Kit of parts zur Transfektion kann bevorzugt (a) ein nicht-virales Genliefersystem und (b) ein Mittel zur zumindest teilweisen Unterdrückung und/oder Aktivierung der angeborenen intrazellulären und/oder interzellulären lmmunabwehr umfassen, wobei das nicht-virale Genliefersystem:
(i) ein kationisches Lipid, ein kationisches Polymer, oder ein kationisches Protein umfassen kann; und/oder
(ii) eine Verbindung umfassen kann, die eine DNA und/oder RNA-bindende Domäne aufweist und rezeptorvermittelte Endozytose oder einen Membrantransfer auslösen kann; und/oder
(iii) eine Verbindung umfassen kann, die kovalent an DNA und/oder RNA gebunden ist und rezeptorvermittelte Endozytose oder einen Membrantransfer auslösen kann; und
das Mittel zur zumindest teilweisen Unterdrückung und/oder Aktivierung der angeborenen intrazellulären und/oder interzellulären lmmunabwehr, ausgewählt sein kann aus:
(i) einem Antikörper gegen TLR 1, TLR 2, TLR 3, TLR 4, TLR 5, TLR 6, TLR 7, TLR 8, TLR 9, TLR 10, TLR 11,TLR 12, oder TLR 13;
(ii) einem Antikörper gegen einen Zytokinrezeptor oder einem Zytokinrezeptor-Antagonisten;
(iii) einem Inhibitor der Kinase MEK1 und/oder MEK2;
(iv) einem Agonisten für TLR7 und/oder TLR8, ausgewählt aus der Gruppe, umfassend Bropirimine (2-amino-5-bromo-6-phenyl-4-pyrimidinone), Imidazochinoline, Thiazolochinoline, und Guanosinanaloga; und
(v) einer Kombination derselben.

Weiterhin bevorzugt umfasst eine erfindungsgemäße Zusammensetzung oder ein erfindungsgemäßer Kit of parts ein nicht-virales Genliefersystem mit einem kationischen Lipid.

Ferner kann eine erfindungsgemäße Zusammensetzung oder ein erfindungsgemäßer Kit of parts ein nicht-virales Genliefersystem mit einem kationischen Lipid gemäß folgender Formel umfassen: 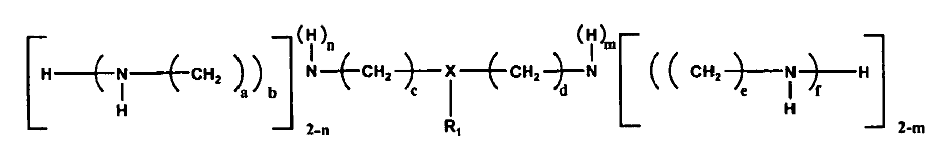 wobei 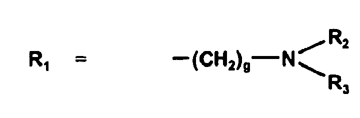 sein kann, wobei
R₂ und R₃ unabhängig voneinander Dodecyl, Dodecenyl, Tetradecyl, Tetradecenyl, Hexadecyl, Hexadecenyl, Octadecyl, Octadecenyl oder andere Alkylreste sind, die in allen möglichen Kombinationen gesättigt, ungesättigt, verzweigt, unverzweigt, fluoriert oder nicht fluoriert sein können, und aus 5 bis 30 Kohlenstoffatomen aufgebaut sein können; 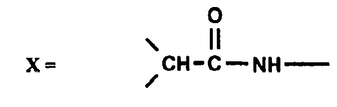 sein kann; und wobei
m = 0 und n = 0; oder m = 0 und n = 1; oder m = 0 und n = 2; oder m = 1 und n = 1; oder m = 1 und n = 2; oder m = 2 und n = 2 sein können; und g = 1,2,3,4,5,6,7 oder 8 sein kann; a = 0,1,2,3,4,5 oder 6 sein kann; b = 0,1,2,3,4,5 oder 6 sein kann; c = 0,1,2,3,4,5 oder 6 sein kann; d = 0,1,2,3,4,5 oder 6 sein kann; e = 0,1,2,3,4,5 oder 6 sein kann; und f = 0,1,2,3,4,5 oder 6 sein kann.

Bevorzugt können in dem kationischen Lipid R₂ und R₃ unabhängig voneinander Dodecyl, Dodecenyl, Tetradecyl, Tetradecenyl, Hexadecyl, Hexadecenyl, Octadecyl, Octadecenyl sein; m und n = 1 sein; g = 1,2,3,4,5,6,7 oder 8 sein; a = 0,1,2,3,4,5 oder 6 sein; b = 0,1,2,3,4,5 oder 6 sein; c = 0,1,2,3,4,5 oder 6 sein; d = 0,1,2,3,4,5 oder 6 sein; e = 0,1,2,3,4,5 oder 6 sein; und f = 0,1,2,3,4,5 oder 6 sein.

Erfindungsgemäß kann die Zusammensetzung oder der Kit of parts modifiziertes oder unmodifiziertes genetisches Material enthalten, insbesondere modifizierte oder unmodifizierte ssDNA, modifizierte oder unmodifizierte dsDNA, modifizierte oder unmodifizierte ssRNA, modifizierte oder unmodifizierte dsRNS und/oder modifizierte oder unmodifizierte siRNA.

Bevorzugt kann die Zusammensetzung oder der Kit of parts als Mittel zur zumindest teilweisen Unterdrückung und/oder Aktivierung der angeborenen intrazellulären und/oder interzellulären Immunabwehr 1,4-Diamino-2,3-dicyano-1,4-bis(o-amionophenylmercapto)-butadien (U0126); Imiquimod (R837, 1-(2-methylpropyl)-1H-imidazol[4,5-c]quinolin-4-amine); Resiquimod (R848, 4-amino-2-(ethoxymethyl)-a,a-dimethyl-1 H-imidazo[4,5-c]quinoline-1-ethanol); Gardiquimod (1-(4-amino-2-ethylamino methylimidazo-[4,5-c]quinolin-1-yl)-2-methylpropan-2-ol); CL075; CL097; Loxoribine (7-allyl-7,8-dihydro-8-oxo-guanosin); lsatoribine (7-Thia-8-oxoguanosin); Bropirimine (2-amino-5-bromo-6-phenyl-4-pyrimidinone); oder irgendeine Kombination derselben enthalten.

Ferner kann die Zusammensetzung oder der Kit of parts als Mittel zur zumindest teilweisen Unterdrückung und/oder Aktivierung der angeborenen intrazellulären und/oder interzellulären Immunabwehr einen Antikörper gegen TLR 3, TLR 7, TLR 8, oder TLR 9 enthalten.

Weiterhin kann die Zusammensetzung oder der Kit of parts erfindungsgemäß als Mittel zur zumindest teilweisen Unterdrückung und/oder Aktivierung der angeborenen intrazellulären und/oder interzellulären Immunabwehr einen Antikörper oder Antagonisten gegen Interleukin-1-Rezeptor, insbesondere IL-ra; Interferon-Typ-I-Rezeptor; Interferon-gamma-Rezeptor; oder Tumornekrosefaktor-Rezeptor enthalten.

In einer bevorzugten Ausführungsform können mehrere der vorstehend genannten Mittel zur zumindest teilweisen Unterdrückung und/oder Aktivierung der lmmunabwehr miteinander kombiniert werden, d.h. es können zwei, drei oder mehrere Komponenten in dem erfindungsgemäßen Mittel zur zumindest teilweisen Unterdrückung und/oder Aktivierung der angeborenen intrazellulären und/oder interzellulären Immunabwehr eingesetzt werden. Ebenso ist es möglich mehrere der vorstehend genannten Komponenten a) und/oder b) und/oder c) einzusetzen.

Bei dem erfindungsgemäßen Kit of parts können:
- alle Komponenten getrennt voneinander vorliegen, oder
- die Komponenten a) und b) gemeinsam, oder
- die Komponenten a) und c) gemeinsam, oder
- die Komponenten b) und c) gemeinsam vorliegen.

Bevorzugt können in einem Kit of parts:
- alle Komponenten vollständig getrennt voneinander vorliegen;
- die Komponenten a) und b) getrennt voneinander vorliegen; oder
- die Komponenten a) und b) gemeinsam vorliegen.

So können die Komponenten entweder getrennt voneinander z.B. in Glas- oder Plastikbehälter vorliegen, die gemeinsam verpackt sind, oder die Komponenten können zu zweit oder zu mehreren in entsprechenden Behältern bereitgestellt werden. Erfindungsgemäß kann der Kit of parts das nicht-virale Genliefersystem in Form von Salzen, insbesondere formuliert als Liposomen/Micellen; als Lösung, insbesondere in Form einer wässrigen Lösung, Salzlösung, gepufferten Salzlösung, z.B. Salzlösung mit HEPES Puffer, oder Lösung in einem physiologisch unbedenklichen Solvent, z.B. Ethanal/DMSO; in lyophilisierter Form; als Feststoff oder als Film enthalten. Lösungen des nicht-viralen Genliefersystems haben vorzugsweise einen pH Wert von 5 bis 9. Die Endkonzentration des nicht-viralen Genliefersystems im Kulturmedium beträgt vorzugsweise 0,01-10 mg/ml.

Das Mittel zur zumindest teilweisen Unterdrückung und/oder Aktivierung der angeborenen intrazellulären und/oder interzellulären Immunabwehr, wie beispielsweise ein Antikörper, Antagonist oder Inhibitor, kann in einem erfindungsgemäßen Kit of parts lyophilisiert; gelöst in Wasser, Salzlösung, gepufferter Salzlösung, z.B. Salzlösung mit PBS Puffer, oder einem geeigneten organischen Solvent, z.B. Ethanol, DMSO, etc, enthalten sein. Lösungen haben vorzugsweise einen pH Wert von 5 bis 9. Optional können Stabilisatoren enthalten sein. Das Mittel zur zumindest teilweisen Unterdrückung und/oder Aktivierung der angeborenen intrazellulären und/oder interzellulären Immunabwehr hat liegt vorzugsweise in einer Konzentration von 0,01 bis 10 mg/ml vor.

Kits mit genetischem Material können dieses genetische Material lyophilisiert, gelöst in Wasser, Salzlösung oder gepufferter Salzlösung, z.B. Salzlösung mit TE Puffer enthalten. Lösungen des genetischen Materials haben vorzugsweise einen pH Wert von 5 bis 9. Die Endkonzentration des genetischen Materials im Kulturmedium beträgt vorzugsweise 0,01-10 mg/ml.

Die erfindungsgemäße Zusammensetzung und/oder der erfindungsgemäße Kit of parts können zur Durchführung eines erfindungsgemäßen Verfahrens eingesetzt werden.

Ferner kann die erfindungsgemäße Zusammensetzung als pharmazeutische Zusammensetzung und der erfindungsgemäße Kit of parts als pharmazeutischer Kit of parts vorliegen.

Weiterhin kann eine erfindungsgemäße Zusammensetzung oder Kit of parts zur Behandlung einer Krankheit durch Gentherapie verwendet werden.

Bei der Krankheit kann es sich beispielsweise um cystische Fibrose, Muskeldystrophie, Phenylketonurie, Ahornsirupkrankheit, Propionazidämie, Methylmalonazidämie, Adenosindeaminasemangel, Hypercholesterinämie, Hämophilie, β-Thalassämie, Krebs, eine Viruserkrankung, eine Degeneration der Macula, Amyotropische Lateral-Sklerose und/oder eine Entzündungserkrankung handeln.

Die vorliegende Erfindung kann auch dazu genutzt werden Transfektionen durchzuführen, ohne das Expressionprofil der Zellen in einem ungewünschten Ausmaß zu verändern. Von besonderem Interesse ist dies bei in vivo Anwendungen, da hier die Aktivierung des Immunsystems häufig ein Problem darstellt.

Besonders auch bei der Untersuchung von Signaltransduktionswegen durch den Knockdown eines beteiligten Proteins durch siRNA ist es unerwünscht, dass übrige Expressionsprofil der Zelle unnötig zu verändern, insbesondere da bekannt ist, dass die Signaltransduktionskaskaden sich gegenseitig häufig beeinflussen.

### Beispiele

Allgemeines Material:
1. Hela Zellen
2. Metafectene Pro, T040-1.0, Biontex Laboratories
3. 24-Well-Platte, TPP, Produktnummer 92024
4. 48-Well-Platten, Corning Inc., Costar, Produktnummer 3548
5. DMEM, PAA, Kat-Nr. E15-883
6. FCS Mycoplex, PAA, Kat-Nr. E15-773
7. pCMV-lacZ, Produktnr.: PF462-060207, Plasmidfactory, c= 1mg/ml in WFI
8. β-Galaktosidase Assay Kit, Stratagene
9. Heia Luc-Zellen (stabil mit Luciferase transfizierte Zellen)
10. Luciferase Assay Kit, Promega
11. BCA Protein Assay Kit, Thermo Scientific, Prod-Nr.: 23227
12. Dimethylsulfoxid (DMSO für die Molekularbiologie), Fluka; No. 41639
13. siRNA, entsalzt, 30pMol/µl in Universal Buffer (siMAX von MWG)
   gerichtet gegen Luciferase GL3 (anti-Luciferase-siRNA):
   Sense Sequence: CUUACGCUGAGUACUUCGAtt
   Antisense Sequence: UCGAAGUACUCAGCGUAAGtt
   Non-specific control:
   Sense Sequence: AGGUAGUGUAAUCGCCUUGtt
   Antisense Sequence: CAAGGCGAUUACACUACCUtt

### Beispiel 1

### Material:

1. Sheep Polyclonal Antibody against human IFNβ, PBL Biomedical Laboratories, Product Number 31400-1, 0,25 mg/ml (estimate), 2 x 10⁴ Units/ml.

### Detaillierte Versuchsbeschreibung:

**1. Ta4:** Es werden HeLa Zellen in eine 24 Weil Platte ausgesät. Dabei wird eine Zellzahl von 2,3 * 105 Zellen in ein Weil in 500 µl komplettem Medium (10% FCS) ausplattiert. Anschließend wird 24 h in einem CO₂-Inkubator (10%) inkubiert.

### 2.Tag:

Zunächst wird der Antikörper (Sheep Polyclonal Antibody against human IFNβ) aufgetaut. Anschließend wird er mit 400pl PBS (Phosphate Buffered Saline) versetzt und sanft gemischt. Er hat jetzt eine Konzentration von 0,05pg/pl. Anschließend werden die Wells der 24 Weil Platte mit folgenden Mengen Antikörper versorgt:

| | 1 | 2 | 3 | 4 | 5 | 6 |
|---|---|---|---|---|---|---|
| A | 0µg (0µl) | 0,25µg | 0,5µg | 1µg (20µl) | 1,5µg | 3µg (60µl) |
| | | (5µl) | (10µl) | | (30µl) | |
| B | 0µg (0µl) | 0,25µg | 0,5µg | 1µg (20µl) | 1,5µg | 3µg (60µl) |
| | | (5µl) | (10µl) | | (30µl) | |
| C | 0µg (0µl) | 0,25µg | 0,5µg | 1µg (20µl) | 1,5µg | 3µg (60µl) |
| | | (5µl) | (10µl) | | (30µl) | |
| D | 0µg (0µl) | 0,25µl | 0,5µg | 1µg (20µl) | 1,5Ng | 3µg (60µl) |
| | | (5µl) | (10µl) | | (30µl) | |

Anschließend wird im Inkubator für 0,5 h inkubiert. Derweilen werden die Lipoplexe hergestellt. Dazu werden 26 µl DNA (pCMV-lacZ) in 1300 µl PBS einpipettiert und durch sanftes Auf- und Abpipettieren gemischt. 104 µl Metafectene Pro werden ebenfalls in 1300 µl

PBS einpipettiert und durch sanftes Auf und Abpipettieren gemischt. Jetzt werden beide Lösungen gemischt und 15 min inkubiert.
Am Ende werden jeweils 100 µl der Lipoplexlösung in jedes Well gegeben. Anschließend wird 24 h in einem CO₂-)Inkubator (10%) inkubiert.

### 3. Tag:

Am dritten Tag wird das Medium der Zeilen C und D erneuert. Die Schritte Lipoplexherstellung und Transfektion werden für diese Zeilen wiederholt. Anschließend wird 24 h in einem CO₂-Inkubator (10%) inkubiert.

### 4. Tag

### Reporter₄enassav:

Die Effizienz der Transfektion wird mit dem β-Galaktosidase Assay Kit nach Vorgaben des Herstellers durchgeführt. Die Platten werden so lange entwickelt bis im Mikroplate-Reader eine Gelbfärbung mit einer Absorption von 1-2 gemessen wird und anschließend sofort gestoppt. Die Inkubationszeit wird anschließend notiert. Die Werte werden mit dem Mikroplate-Reader ausgelesen, und der Mittelwert gebildet.

### Ergebnis:

Inkubationszeit 9,5 min

### Mittelwert von 3 Messungen:

| | **1** | **2** | **3** | **4** | **5** | **6** |
|---|---|---|---|---|---|---|
| A | 0,717 | 0,689 | 0,650 | 0,652 | 0,635 | 0,509 |
| B | 0,668 | 0,672 | 0,690 | 0,679 | 0,714 | 0,525 |
| C | 0,858 | 0,823 | 1,228 | 1,690 | 1,852 | 0,975 |
| D | 1,501 | 1,805 | 1,518 | 1,886 | 2,017 | 1,909 |

Die Zeilen A und B stellen Duplikate der Ergebnisse für eine einfache Transfektion dar. Die Zeilen C und D stellen Duplikate der Ergebnisse für eine repetitive Transfektion dar. Es wird daher der Mittelwert gebildet:

| | **1** | **2** | **3** | **4** | **5** | **6** |
|---|---|---|---|---|---|---|
| **A&B/2** | 0,6925 | 0,6805 | 0,6700 | 0,6655 | 0,6745 | 0,5170 |
| **C&D/2** | 1,1810 | 1,3140 | 1,3730 | 1,7880 | 1,9345 | 1,4420 |

### Figur 1

### Beispiel 2

### Material:

1. Mouse monoclonal Antibody against Human Interferone Alpha/Beta Receptor Chain 2 (CD118), clone MMHAR-2, Isotype 1g2a, C= 0,5 mg/ml in PBS (Phosphat buffered saline) containing 0,1% bovine serum albumin (BSA), PBL Biomedical Laboratories, Product-No: 21385

### Detaillierte Versuchsbeschreibung:

### Analog Beispiel 1

### Abweichungen:

Zunächst wird der Antikörper (Mouse monoclonal Antibody against Human Interferone Alpha/Beta Receptor Chain) mit 400pl PBS (Phosphate Buffered Saline) versetzt und sanft gemischt. Er hat jetzt eine Konzentration von 0,1 µg/µl. Die Wells der 24 Well Platte werden mit folgenden Mengen Antikörper versorgt:

| | 1 | 2 | 3 | 4 | 5 | 6 |
|---|---|---|---|---|---|---|
| A | 0µg(0µl) | 0,5µg(5µl) | 1µg(10µl) | 2µg(20µl) | 3µg(30µl) | 6µg(60µl) |
| B | 0µg(0µl) | 0,5µg(5µl) | 1µg(10µl) | 2µg(20µl) | 3µg(30µl) | 6µg(60µl) |
| C | 0µg(0µl) | 0,5µg(5µl) | 1µg(10µl) | 2µg(20µl) | 3µg(30µl) | 6µg(60µl) |
| D | 0µg(0µl) | 0,5µg(5µl) | 1µg(10µl) | 2µg(20µl) | 3µg(30µl) | 6pg(60µl) |

Die Lipoplexzugabe erfolgt nach 5 h Inkubationszeit mit dem Antikörper

Ergebnis:
Inkubationszeit: 4min

### Mittelwert von 3 Messungen:

| | **1** | **2** | **3** | **4** | **5** | **6** |
|---|---|---|---|---|---|---|
| **A** | 1,269 | 1,113 | 1,163 | 1,185 | 1,214 | 1,084 |
| **B** | 1,132 | 1,102 | 1,088 | 1,229 | 1,113 | 1,154 |
| **C** | 1,127 | 1,438 | 1,533 | 1,378 | 1,504 | 1,367 |
| **D** | 1,395 | 1,393 | 1,377 | 1,417 | 1,459 | 1,467 |

Die Zeilen A und B stellen Duplikate der Ergebnisse für eine einfache Transfektion dar. Die Zeilen C und D stellen Duplikate der Ergebnisse für eine repetitive Transfektion dar. Es wird daher der Mittelwert gebildet:

| | **1** | **2** | **3** | **4** | **5** | **6** |
|---|---|---|---|---|---|---|
| **A&B/2** | 1,2005 | 1,1075 | 1,1255 | 1,2070 | 1,1635 | 1,1190 |
| **C&D/2** | 1,2610 | 1,4155 | 1,4550 | 1,3975 | 1,4815 | 1,4170 |

### Figur 2

### Beispiel 3

Weitere Versuche mit Antikörpern gegen Rezeptoren & Zytokine

### Detaillierte Versuchsbeschreibung:

**1**. **Tag:** Es werden HeLa Zellen in eine 48 Well Platte ausgesät. Dabei wird eine Zellzahl von 1,2 * 10⁵ Zellen in ein Well in 250 µl komplettem Medium (10% FCS) ausplattiert. Anschließend wird 24 h in einem CO₂-lnkubator (10%) inkubiert.

### 2.Tag:

Der Antikörper wird mit PBS auf eine Konzentration von 0,05µg/µl eingestellt. Anschließend werden die Wells der 48 Well Platte mit folgenden Mengen Antikörper versorgt:

| | 1 | 2 | 3 | 4 |
|---|---|---|---|---|
| A | 0µg (0µl) | 0µg (0µl) | 0µg (0µl) | 0µg (0µl) |
| B | 0,25µg (5µl) | 0,25µg (5µl) | 0,25µg (5µl) | 0,25µg (5µl) |
| C | 0,5µg (10µl) | 0,5µg (10µl) | 0,5µg (10µl) | 0,5µg (10µl) |
| D | 1µg (20µl) | 1µg (20µl) | 1µg (20µl) | 1µg (20µl) |
| E | 1,5µg (30µl) | 1,5µg (30µl) | 1,5µg (30µl) | 1,5µg (30µl) |
| F | 3µg (60µl) | 3µg (60µl) | 3µg (60µl) | 3µg (60µl) |

Anschließend wird im Inkubator je nach Antikörper für 5 oder 0,5 h inkubiert. Anschließend werden die Lipoplexe hergestellt. Dazu werden 13 µl DNA (pCMV-lacZ) in 650 µl PBS einpipettiert und durch sanftes Auf- und Abpipettieren gemischt. 52 µl Metafectene Pro werden ebenfalls in 650 µl PBS einpipettiert und durch sanftes Auf und Abpipettieren gemischt. Jetzt werden beide Lösungen gemischt und 15 min inkubiert. Am Ende werden jeweils 50 µl der Lipoplexlösung in jedes Well gegeben. Anschließend wird 24 h in einem CO₂-Inkubator (10%) inkubiert.

### 3. Tag:

Am dritten Tag wird das Medium der Spalten 3 und 4 erneuert. Die Schritte Lipoplexherstellung und Transfektion werden für diese Zeilen wiederholt. Anschließend wird 24 h in einem CO₂-Inkubator (10%) inkubiert.

### 4. Tag

### Reportergenassay:

Analog Beispiel 1 (mit halben Mengen)

### Ergebnisse:

### Antikörper:

Mouse Anti-human-CD282 Antibody (= anti TLR2), monoclonal, AbD Serotec, Kat-nr.: MCA2484EL
Vorinkubationszeit mit dem Antikörper vor der Transfektion: 5h
Entwicklungszeit Reportergenassay [min]: 5

### Mittelwert von 3 Messungen:

| | **1** | **2** | **3** | **4** |
|---|---|---|---|---|
| **A** | 0,65 | 0,66 | 1,08 | 1,18 |
| **B** | 0,61 | 0,68 | 1,12 | 1,07 |
| **C** | 0,72 | 0,70 | 1,70 | 1,21 |
| **D** | 0,67 | 0,76 | 1,82 | 1,34 |
| **E** | 0,67 | 0,86 | 1,62 | 1,66 |
| **F** | 0,64 | 0,67 | 1,56 | 1,34 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 0,65 | 0,64 | 0,71 | 0,71 | 0,76 | 0,65 |
| **3&4/2** | 1,13 | 1,09 | 1,45 | 1,58 | 1,65 | 1,45 |

### Figur 3

### Antikörper:

Mouse Anti-human TLR3 Antibody, monoclonal, Lifespan Biosciences Kat-nr.: LS-C18685
Vorinkubationszeit mit dem Antikörper vor der Transfektion: 5 h
Entwicklungszeit Reportergenassay [min]: 5,5

### Mittelwert von 3

### Messungen:

| | **1** | **2** | **3** | **4** |
|---|---|---|---|---|
| **A** | 0,72 | 0,64 | 1,30 | 1,32 |
| **B** | 0,64 | 0,67 | 1,42 | 1,30 |
| **C** | 0,75 | 0,73 | 1,54 | 1,59 |
| **D** | 0,75 | 0,86 | 1,71 | 1,69 |
| **E** | 0,89 | 0,82 | 1,65 | 1,95 |
| **F** | 0,76 | 0,82 | 1,83 | 1,65 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 0,68 | 0,65 | 0,74 | 0,80 | 0,85 | 0,79 |
| **3&4/2** | 1,31 | 1,36 | 1,56 | 1,70 | 1,80 | 1,74 |

### Figur 4

### Antikörper:

Mouse Anti-human-CD284 Antibody (= anti TLR4), monoclonal, AbD Serotec, Kat-nr.: MCA2061 EL
Vorinkubationszeit mit dem Antikörper vor der Transfektion: 5 h
Entwicklungszeit Reportergenassay [min]: 5,5

### Mittelwert von 3

### Messungen:

| | **1** | **2** | **3** | **4** |
|---|---|---|---|---|
| **A** | 0,65 | 0,65 | 1,03 | 1,22 |
| **B** | 0,67 | 0,65 | 1,44 | 1,67 |
| **C** | 0,74 | 0,64 | 1,42 | 1,17 |
| **D** | 0,78 | 0,69 | 1,57 | 1,38 |
| **E** | 0,74 | 0,73 | 1,59 | 1,63 |
| **F** | 0,78 | 0,71 | 1,82 | 1,56 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 0,65 | 0,66 | 0,69 | 0,73 | 0,73 | 0,74 |
| **3&4/2** | 1,12 | 1,55 | 1,29 | 1,47 | 1,61 | 1,69 |

### Figur 5

### Antikörper:

Antikörper: Mouse Anti human TLR1 Antibody (monoclonal, 0.05% Natriumazid, 100 µg Lyophilisat); Invivogen, No. Mab-htlr1.

Zur Entfernung des Natriumazides wurde der Antikörper gegen PBS dialysiert:
Dialysemembran: Spectra/Por DispoDialyser (500µl, Celluloseestermembran, 25000 Da
Molecular Weight Cut-Off); Spectrum Laboratories; No. 135492, Lot 3224004.
Vorinkubationszeit mit dem Antikörper vor der Transfektion: 5 h
Entwicklungszeit Reportergenassay [min]: 4

### Mittelwert von 3 Messungen:

| | **1** | **2** | **3** | **4** |
|---|---|---|---|---|
| **A** | 1,055 | 1,225 | 1,683 | 1,627 |
| **B** | 1,354 | 1,328 | 1,905 | 1,798 |
| **C** | 1,346 | 1,516 | 2,033 | 1,914 |
| **D** | 1,401 | 1,367 | 2,106 | 1,827 |
| **E** | 1,260 | 1,488 | 2,063 | 1,841 |
| **F** | 1,085 | 1,020 | 1,816 | 1,712 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 1,140 | 1,341 | 1,431 | 1,384 | 1,374 | 1,053 |
| **3&4/2** | 1,655 | 1,852 | 1,974 | 1,966 | 1,952 | 1,764 |

### Figur 6

### Beispiel 4

Weitere Versuche mit Antagonisten

### Detaillierte Versuchsbeschreibung:

### 1. Tag: Analog Beispiel 3

### 2.Tag:

Der Antagonist wird mit PBS auf eine Konzentration von 0,025µg/µl eingestellt. Anschließend werden die Wells der 48 Well Platte mit folgenden Mengen Antikörper versorgt:

| | **1** | **2** | **3** | **4** |
|---|---|---|---|---|
| **A** | 0µg (0µl) | 0µg (0µl) | 0µg (0µl) | 0µg (0µl) |
| **B** | 0,0625µg (2.5µl) | 0,0625µg (2.5µl) | 0,0625µg (2.5µl) | 0,0625µg (2.5µl) |
| **C** | 0,125µg (5µl) | 0,125µg (5µl) | 0,125µg (5µl) | 0,125µg (5µl) |
| **D** | 0,250µg (10µl) | 0,250µg (10µl) | 0,250µg (10µl) | 0,250µg (10µl) |
| **E** | 0,375µg (15µl) | 0,375µg (15µl) | 0,375µg (15µl) | 0,375µg (15µl) |
| **F** | 0,75µg (30µl) | 0,75µg (30µl) | 0,75µg (30µl) | 0,75µg (30µl) |

Anschließend wird im Inkubator für 7 inkubiert. Anschließend werden die Lipoplexe hergestellt. Dazu werden 13 µl DNA (pCMV-lacZ) in 650 µl PBS einpipettiert und durch sanftes Auf- und Abpipettieren gemischt. 52 µl Metafectene Pro werden ebenfalls in 650 µl PBS einpipettiert und durch sanftes Auf und Abpipettieren gemischt. Jetzt werden beide Lösungen gemischt und 15 min inkubiert. Am Ende werden jeweils 50 µl der Lipoplexlösung in jedes Well gegeben. Anschließend wird 24 h in einem CO₂-lnkubator (10%) inkubiert.

### 3. Tag:

Analog Beispiel 3

### 4. Tag

### Reportergenassay :

Analog Beispiel 1 (mit halben Mengen)

### Ergebnisse:

### Antagonist:

Human Interleukin-1 Receptor Antagonist (IL1-ra Human), Biomol, Kat.-Nr.: 54592
Vorinkubationszeit mit dem Antikörper vor der Transfektion: 7h
Entwicklungszeit Reportergenassay [min]: 10

### Mittelwert von 3 Messungen:

| | **1** | **2** | **3** | **4** |
|---|---|---|---|---|
| **A** | 0,436 | 0,433 | 0,932 | 0,820 |
| **B** | 0,427 | 0,448 | 0,791 | 1,021 |
| **C** | 0,415 | 0,435 | 0,856 | 1,185 |
| **D** | 0,393 | 0,439 | 1,035 | 1,026 |
| **E** | 0,392 | 0,460 | 1,076 | 1,081 |
| **F** | 0,443 | 0,455 | 1,161 | 1,207 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 0,435 | 0,437 | 0,425 | 0,416 | 0,426 | 0,449 |
| **3&4/2** | 0,876 | 0,906 | 1,020 | 1.030 | 1,078 | 1,184 |

### Figur 7

### Beispiel 5

Weiterer Versuch mit einem Kinase-Inhibitor:
MEK1 und MEK2 Inhibitor:
   U0126, 1,4-Diamino-2,3-dicyano-1,4-bis(o-amionophenylmercapto)butadiene, MW = 380,5, Invivogen, Kat.-Nr.: tlr-u0126.

### Detaillierte Versuchsbeschreibung:

**1. Tag:** Es werden HeLa Zellen in eine 48 Well Platte ausgesät. Dabei wird eine Zellzahl von 0,8 * 10⁵ Zellen in ein Well in 250 µl komplettem Medium (10% FCS) ausplattiert. Anschließend wird 24 h in einem CO₂-lnkubator (10%) inkubiert.

### 2.Tag:

1 mg Inhibitor wird in 100 µl DMSO gelöst (Stocklösung). Anschließend wird mit PBS 1:20 verdünnt (Arbeitslösung). Anschließend werden die Wells der 48 Well Platte mit folgenden Mengen Inhibitor versorgt:

| | F | E | D | C | B | A |
|---|---|---|---|---|---|---|
| 1 | 0µM(0µl) | 5µM(0,95µl) | 10µM(1,9µl) | 25µM(4,75µl) | 50µM(9,5µl) | 75µM(14,25µl) |

Anschließend wird im Inkubator für 2 h inkubiert. Anschließend werden die Lipoplexe hergestellt. Dazu werden 5 µl DNA (pCMV-lacZ) in 250 µl PBS einpipettiert und durch sanftes Auf- und Abpipettieren gemischt. 20 µl Metafectene Pro werden ebenfalls in 250 µl PBS einpipettiert und durch sanftes Auf und Abpipettieren gemischt. Jetzt werden beide Lösungen gemischt und 15 min inkubiert.
Am Ende werden jeweils 50 µl der Lipoplexlösung in jedes Well gegeben. Anschließend wird 48 h in einem CO₂-Inkubator (10%) inkubiert.

### 3. Tag:

Am dritten Tag wird das Medium erneuert und die in der Tabelle angegebenen Inhibitormengen ersetzt. Die Schritte Lipoplexherstellung und Transfektion werden ohne Inkubationszeit wiederholt. Anschließend wird 24 h in einem CO₂-Inkubator (10%) inkubiert.

### 4. Tag

### Reportergenassay :

Analog Beispiel 1 (mit halben Mengen)

### Ergebnisse:

Vorinkubationszeit mit dem Inhibitor vor der Transfektion: 2h
Entwicklungszeit Reportergenassay [min]: 10

### Mittelwert von 3 Messungen:

| | **F** | **E** | **D** | **C** | **B** | **A** |
|---|---|---|---|---|---|---|
| **1** | 0,63 | 0,72 | 0,92 | 0,79 | 0,90 | 0,97 |

### Figur 8

### Beispiel 6

Weiterer Versuch mit HepG2 Zellen

### Durchführung anlalog Beispiel 3

Abweichungen: HepG2 Zellen, Antikörperkonzentration 0,1 µg/µl statt 0,05 µg/µl in PBS, Lipoplexbildung in serumfreien DMEM statt in PBS

### Antikörper:

Mouse Anti-human-CD282 Antibody (= anti TLR2), monoclonal, AbD Serotec, Kat-nr.: MCA2484EL
Vorinkubationszeit mit dem Antikörper vor der Transfektion: 5h
Entwicklungszeit Reportergenassay [min]: 4

### Mittelwert von 3 Messungen:

| | **1** | **2** | **3** | **4** |
|---|---|---|---|---|
| **A** | 1,680 | 1,486 | 1,649 | 1,815 |
| **B** | 1,884 | 1,792 | 1,999 | 2,267 |
| **C** | 2,095 | 1,960 | 2,111 | 2,297 |
| **D** | 2,036 | 1,936 | 1,911 | 2,184 |
| **E** | 1,964 | 1,930 | 1,987 | 2,164 |
| **F** | 1,719 | 1,935 | 1,930 | 2,224 |

Die Spalten 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **A** | **B** | **C** | **D** | **E** | **F** |
|---|---|---|---|---|---|---|
| **1&2/2** | 1,583 | 1,838 | 2,028 | 1,986 | 1,947 | 1,827 |
| **3&412** | 1,732 | 2,133 | 2,204 | 2,048 | 2,076 | 2,077 |

### Figur 9

### Beispiel 7

Weiterer Versuch mit einem Antikörper gegen TLR9 mit Einbau in einen Lipoplex

### Antikörper:

Rabbit Anti-human-TLR9 Antibody (= anti TLR9), polyclonal, 0,5 µg/µl in PBS, 0,2% Gelatine, 0,05% Natriumazid, Lifespan Biosciences, Kat-nr.: MCA2484EL.

Zur Entfernung des Natriumazides wurde der Antikörper gegen PBS dialysiert: Dialysemembran: Spectra/Por DispoDialyser (500µl, Celluloseestermembran, 25000 Da Molecular Weight Cut-Off); Spectrum Laboratories; No. 135492, Lot 3224004.

### Detaillierte Versuchsbeschreibung:

**1. Tag:** Es werden HeLa Zellen in eine 48 Well Platte ausgesät. Dabei wird eine Zellzahl von 1,0 * 10⁵ Zellen in ein Well in 250 µl komplettem Medium (10% FCS) ausplattiert. Anschließend wird 24 h in einem CO₂-lnkubator (10%) inkubiert.

### 2.Tag:

Der Antikörper wird mit PBS auf eine Konzentration von 0,1 µg/µl eingestellt. 15 µg DNA (pCMVßGal) und 60 µl Metafectene Pro werden jeweils in 300 µl serumfreien DMEM gelöst. Durch sanftes Auf- und Abpipettieren werden die einzelnen Lösungen gemischt. Anschließend werden Lipoplexe folgender Zusammensetzung hergestellt.

| **Lösung** | **1** | **2** | **3** | **4** | **6** |
|---|---|---|---|---|---|
| Antikörperlösung [µl] | 0 | 11 | 22 | 44 | 66 |
| Metafectene Pro-Lösung [µl] | 44 | 44 | 44 | 44 | 44 |
| DNA-Lösung [µl] | 44 | 44 | 44 | 44 | 44 |
| Serumfreies DMEM [µl] | 132 | 121 | 110 | 88 | 66 |

Dabei wird zunächst die Metafectene Pro-Lösung mit der Antikörperlösung vereint und für fünf Minuten bei Raumtemperatur (RT) inkubiert. Anschließend wird die DNA-Lösung und das serumfreie DMEM hinzugegeben und weitere 15 Minuten bei RT inkubiert.

Jeweils 50 µl der DNA-Antikörper-Lipidkomplexe werden in die Wells pipettiert. Dabei wird die Lösung 1 in vier Wells der Spalte 1 pipettiert. Lösung 2 in vier Wells der Spalte 2 und so weiter. Anschließend wird 24 h in einem CO₂-lnkubator (10%) inkubiert.

### 3. Tag

Das Medium aller Zeilen wird erneuert. Für die Zeilen C und D wird eine zweite Transfektion analog der Transfektion am ersten Tag durchgeführt.

### 4. Tag

### Reportergenassay :

Analog Beispiel 1 (mit halben Mengen)

### Ergebnisse:

### Mittelwert von 3 Messungen:

| | **1** | **2** | **3** | **4** |
|---|---|---|---|---|
| **A** | 0,819 | 1,671 | 1,419 | 1,845 |
| **B** | 0,860 | 1,591 | 1,604 | 1,858 |
| **C** | 1,005 | 2,185 | 1,940 | 2,267 |
| **D** | 1,472 | 2,650 | 2,264 | 2,401 |

Die Zeien 1 und 2 stellen Duplikate der Ergebnisse für eine einfache Transfektion dar. Die Spalten 3 und 4 stellen Duplikate der Ergebnisse für eine repetitive Transfektion dar. Es wird daher der Mittelwert gebildet:

| | **1** | **2** | **3** | **4** |
|---|---|---|---|---|
| **A&B** | 0,840 | 1,631 | 1,512 | 1,852 |
| **C&D** | 1,239 | 2,417 | 2,102 | 2,334 |

Die zugegebene Antikörpermenge enspricht folgenden Mengen Antikörper pro Well:

| | **1** | **2** | **3** | **4** |
|---|---|---|---|---|
| **µg** | 0 | 0,5 | 1,0 | 1,5 |

### Figur 10

### Beispiel 8

siRNA Transfektion in Anwesenheit der Kinase Inhibitoren:
- p38/RK MAPK-Inhibitor: SB203580, Invivogen; No. tlrl-sb20
- MEK-Inhibitor: U0126, Invivogen, No. tlr-u0126

### Detaillierte Versuchsbeschreibung:

**1. Tag:** Es werden HeLa-Luc Zellen in eine 48 Well Platte ausgesät. Dabei wird eine Zellzahl von 1,5 x 10⁴ Zellen in ein Well in 250 µl komplettem Medium (10% FCS) ausplattiert. Anschließend wird 24 h in einem CO₂-lnkubator (10%) inkubiert.

### 2.Tag:

Zuerst werden die Inhibitoren vorbereitet:
SB203580 (M = 377,43 g/mol): 1 mg wird in 20 µl DMSO gelöst. Die Stocklösung wird mit
PBS 1:105 verdünnt.
U0126 (M = 380,49 g/mol): 1 mg des Inhibitors wird in 100 µl DMSO gelöst. Die Stocklösung wird mit PBS 1:20 verdünnt.

Die Wells der 48 Well Platte werden mit folgenden Mengen Inhibitor versorgt:

| | **U0126** | | **SB203580** | |
|---|---|---|---|---|
| | **F** | **E** | **D** | **C** |
| **1** | 0µM(0µl) | 0µM(0µl) | 0µM(0µl) | 0µM(0µl) |
| **2** | 5µM(1µl) | 5µM(1µl) | 5µM(1µl) | 5µM(1µl) |
| **3** | 10µM(2µl) | 10µM(2µl) | 10µM(2µl) | 10µM(2µl) |
| **4** | 25µM(5µl) | 25µM(5µl) | 20µM(4µl) | 20µM(4µl) |
| **5** | 50µM(10µl) | 50µM(10µl) | 30µM(6µl)) | 30µM(6µl) |
| **6** | 75µM(15µl) | 75µM(15µl) | 40µM(8µl) | 40µM(8µl) |
| **7** | 0µM(0µl) | 0µM(0µl) | 0µM(0µl) | 0µM(0µl) |
| **8** | 0µM(0µl) | 0µM(0µl) | 0µM(0µl) | 0µM(0µl) |

### Anschließend wird im Inkubator für 2 h inkubiert. Danach werden die Lipoplexe hergestellt.

Dazu wird 1 µl anti-Luciferase-siRNA Stocklösung in 300 µl PBS einpipettiert und durch sanftes Auf- und Abpipettieren gemischt. Von dieser Lösung werden nur 240 µl weiterverwendet. 1 µl non-specific-control-siRNA wird in 300 µl PBS einpipettiert und durch sanftes Auf- und Abpipettieren gemischt. Von dieser Lösung werden nur 40 µl weiterverwendet.
3 µl Metafectene Pro werden in 225 µl PBS einpipettiert und ebenfalls durch sanftes Auf und Abpipettieren gemischt. Jetzt werden 190µl zur anti-Luciferase-siRNA Arbeitslösung und 35µl zur non-specific-control-siRNA Arbeitslösung pipettiert. Die Lösungen werden gemischt und 15 min inkubiert. Es resultieren Lipoplexe mit einem siRNA-Reagenzverhältnis von 1:5 µg/µl.

Am Ende werden jeweils 15 µl der anti-Luciferase-siRNA-Lipoplexlösung in die ersten 6 Reihen der Platte gegeben. 15 µl der non-specific-control-siRNA- Lipoplexlösung werden in die 7. Reihe der Platte gegeben. Es resultiert eine siRNA Menge von 1 pMot/Well. Die Reihe 8 bleibt untransfiziert. Anschließend wird 24 h in einem CO₂-Inkubator (10%) inkubiert.

### 3. Tag:

Am dritten Tag wird das Medium aller Wells erneuert und die in der Tabelle angegebenen Inhibitormengen ersetzt. Die Schritte Lipoplexherstellung und Transfektion werden nach der Inhibitorzugabe für die Spalten E und C wiederholt. Anschließend wird weitere 24 h in einem CO₂-lnkubator (10%) inkubiert.

### 4. Tag

Luciferase-Assay und BCA Protein Assay:
Beide Assays werden gemäß den Herstellerangaben durchgeführt.

### Auswertung:

Von der 48 Well Platte wir das Medium entfernt und die Zellen mit 100 µl PBS gewaschen. Anschließend werden 120 µl Luciferase-Lysispuffer hinzugegeben. Die Platte wird auf Eis ca. 30 Minuten inkubiert und 20-30 Sekunden durch vorsichtiges Schütteln (Klopfen) gemischt. Nun werden von dieser 48 Well Platte pro Well 25 µl entfernt und mit analoger Belegung in eine zweite 48 Well Platte gegeben. Mit der ersten Platte wird der Luciferase Assay mit der zweiten Platte der BCA Test durchgeführt.

### Luciferase Assay:

Parametereinstellungen des Luminometers:
- Messzeit: 15 sec
- Zeitintervall zwischen Lösungsinjektionen: 0,5 sec
- Intervall zwischen Lösungsinjektion und Messung: 0,1 sec

### BCA Protein Assay:

- Auswertung bei Wellenlänge 562 nm

Alle Messungen wurden dreifach durchgeführt. Im Fall des Luciferase Assays dreimal mit drei neuen Zellextrakten. Die erhaltenen Werte des Luciferase-Assays (RLU) wurden auf 1 µl Zellextrakt und 1 sec Messzeit normiert. Die erhaltenen Werte des BCA Protein Assays wurden ebenfalls auf 1 µl Zellextrakt normiert.

### Luciferase Assay:

RLU/µl Zellextrakt/sec

### Mittelwert von 3x3

### Messungen:

| | **F** | **E** | **D** | **C** |
|---|---|---|---|---|
| **1** | 1157 | 1001 | 1027 | 976 |
| **2** | 375 | 641 | 693 | 696 |
| **3** | 567 | 162 | 747 | 656 |
| **4** | 268 | 179 | 630 | 617 |
| **5** | 193 | 171 | 671 | 587 |
| **6** | 260 | 103 | 669 | 610 |
| **7** | 1829 | 2290 | 2029 | 2135 |
| **8** | 1674 | 2249 | 2504 | 2531 |

### BCA Assay:

ABS/µl Zellextrakt

### Mittelwert von 3 Messungen:

| | **F** | **E** | **D** | **C** |
|---|---|---|---|---|
| **1** | 0,0196 | 0,0196 | 0,0192 | 0,0192 |
| **2** | 0,0192 | 0,0204 | 0,0193 | 0,0189 |
| **3** | 0,0189 | 0,0196 | 0,0193 | 0,0187 |
| **4** | 0,0183 | 0,0188 | 0,0186 | 0,0186 |
| **5** | 0,0182 | 0,0191 | 0,0183 | 0,0186 |
| **6** | 0,0182 | 0,0190 | 0,0184 | 0,0192 |
| **7** | 0,0184 | 0,0186 | 0,0183 | 0,0184 |
| **8** | 0,0184 | 0,0178 | 0,0181 | 0,0187 |

### Figur 11

### Beispiel 9

siRNA Transfektion in Anwesenheit der TLR7/8 Agonisten:
- Imiquimod, InvivoGen, Kat.-Nr.: tlrl-imq
- ssRNA40/LyoVec, InvivoGen, Kat.-Nr.: tlrl-Irna-40

### Detaillierte Versuchsbeschreibung:

### Tag 1:

### Vorbereiten der Zellen:

Es soll eine Zellzahl von 2 x 10⁴ Zellen/cm² in ein Well in 250 µl komplettem Medium ausplattiert werden.

### Herstellung der Lipoplexe:

Zunächst werden die ersten 5 Wells der ersten 3 Reihen einer 48 Well Platte mit jeweils 30 µl PBS gefüllt.
Die Stocklösung der anti-Luciferase-siRNA und der non-specific-control-siRNA wird mit PBS auf eine Konzentration von 1 pmol/10 µl eingestellt. Anschließend werden jeweils 10 µl der Arbeitslösung der anti-Luciferase-siRNA in die ersten 3 Wells der der ersten drei Reihen der 48 Well Platte pipettiert. In die jeweils vierten Wells werden jeweils 10 µl der Arbeitslösung der non-specific-control-siRNA pipettiert.
Das Metafectene Pro Reagenz wird mit PBS 1:300 verdünnt. Von dieser Verdünnung werden jeweils 20 µl in jedes Well mit siRNA gegeben.
Anschließend werden die Lipoplexe für 20 Minuten bei Raumtemperatur inkubiert. Danach werden pro Well 250 µl Zellsuspension zugegeben.
Analog wird eine zweite Platte bestückt.

### Zugabe der Agonisten:

In die ersten 2 Spalten der beiden 48 Well Platte werden 2 verschiedene Agonisten hinzugegeben. Dabei werden 3 verschiedene Arbeitskonzentrationen der Agonisten verwendet. Die Agonisten liegen lyophilisiert vor. Sie werden mit endotoxin-freiem Wasser zu Stocklösungen mit der Konzentration 100µg/ml gelöst. Die Stocklösungen werden jeweils 1:10 mit Wasser zu Arbeitslösungen verdünnt.

Von der Imiquimod-Arbeitslösung werden in A1 0,78 µl (=0,25 µg/ml Endkonzentration), in A2 15,5 µl (=5µg/ml) und in A3 31 µl (=10µg/ml) pipettiert.
Von der ssRNA40/LyoVec-Arbeitslösung werden in B1 0,78 µl (=0,5 µg/ml Endkonzentration ssRNA40), in B2 15,5 µl (=5µg/ml) und in B3 31µl (=10µg/ml) pipettiert.
Nach der Zugabe der Agonisten werden die beiden 48 Well Platten für 48 Stunden im CO₂-Inkubator inkubiert.

### Tag 3:

### Vitalitätsbestimmung:

Nach einer Inkubationszeit von 48 Stunden werden die Zellen einer Platte trypsiniert und mittels einer Neubauer-Zählkammer eine Vitalitätbestimmung mit Trypanblau für jedes Well durchgeführt.

### Luciferase-Assay und BCA Protein Assay: Analog Beispiel 13

### Ergebnisse

### Trypanblaufärbung:

Es wurden zwischen 40 und 70 Zellen ausgewertet.

| | Vitalität [%] |
|---|---|
| Imiquimod 0,5µg/ml + anti-Luciferase-siRNA/Metafectene Pro | 98,6 |
| Imiquimod 5µg/ml + anti-Luciferase-siRNA/Metafectene Pro | 88,7 |
| Imiquimod 10µg/ml + anti-Luciferase-siRNA/Metafectene Pro | 88,6 |
| ssRNA40/LyoVec 0,5µg/ml + anti-Luciferase-siRNA/Metafectene Pro | 97,8 |
| ssRNA40/LyoVec 5µg/ml + anti-Luciferase-siRNA/Metafectene Pro | 81,5 |
| ssRNA40/LyoVec 10µg/ml + anti-Luciferase-siRNA/Metafectene Pro | 83,0 |
| anti-Luciferase-siRNA/Metafectene Pro | 87,7 |
| non-specific-control-siRNA/Metafectene Pro | 92,6 |
| Blindprobe | 95,9 |

### Luciferase Assay:

### RLU/µl Zellextrakt/sec

### Mittelwert von 3x3

| | **1** | **2** | **3** | **4** | **5** |
|---|---|---|---|---|---|
| **A** | 676 | 1023 | 1604 | 3016 | 2936 |
| **B** | 349 | 223 | 1302 | 2753 | 2673 |
| **C** | 116 | 98 | 1481 | 2822 | 2476 |

### BCA Assay ABS/µl Zellextrakt

### Mittelwert von 3x3

### Messungen:

| | **1** | **2** | **3** | **4** | **5** |
|---|---|---|---|---|---|
| **A** | 0,0086 | 0,0080 | 0,0080 | 0,0087 | 0,0093 |
| **B** | 0,0069 | 0,0068 | 0,0078 | 0,0080 | 0,0092 |
| **C** | 0,0073 | 0,0069 | 0,0078 | 0,0080 | 0,0088 |

### Figur 12

Die Ergebnisse der erfindungsgemäßen Beispiele lassen auch den Schluss zu, dass nicht nur das genetische Material, sondern auch die chemischen Agentien wie zb. kationische Lipide und kationische Polymere von den TLRs detektiert werden. Nach den vorliegenden Ergebnissen muss man davon ausgehen, dass im Fall von Hela Zellen, Metafectene Pro als Transfektionsreagenz und DNA (Plasmid) hoher Qualität folgende TLRs involviert sind. TLR 4 detektiert Spuren von LPS in der Plasmidlösung. TLR9 und TLR3 detektieren genetisches Material, also in diesem Fall Plasmide oder entsprechende Verunreinigungen. TLR1 und TLR2 detektieren das Transfektionsreagenz.

Zwanglos lässt sich damit möglicherweise auch erklären, dass unterschiedliche Zellen bei unterschiedlichen Verhältnissen von genetischem Material und Transfektionsreagenz optimale Ergebnisse zeigen. Zellen die zB. sehr viele TLR9 und wenige TLRs für die Reagentien exprimieren, sollten demnach bei einem Verhältniss optimale Ergebnisse zeigen, bei dem wenig DNA auf viel Reagenz trifft.

Es ist einleuchtend, das die Aktivierung der TLRs vor und während der Transfektion von vielen Faktoren abhängt. Je nach Expressionsprofil der Zielzelle kann dabei zB. eine besondere Empfindlichkeit der Zellen gegenüber bestimmten PAMPs gegeben sein. Weiter können unterschiedliche Reagentien und unterschiedliches genetisches Material unterschiedliche TLRs aktivieren. Selbst unterschiedliche DNA Sequenzen können je nach CpG Gehalt Einfluss auf die Transfektionsergebnisse nehmen. Nicht zuletzt spielen auch Verunreinigungen eine nicht zu unterschätzende Rolle. So kann DNA bakteriellen Ursprungs beispielsweise mit LPS oder Flagellin oder RNA verunreinigt sein. ssRNA kann mit dsRNA verunreinigt sein und damit ausser TLR7/8 auch TLR3 ansprechen und umgekehrt. Unterschiedliche Behandlung der Zellen kann durch Stress-Signaltransduktionkaskaden zu unterschiedlichen Ergebnissen führen. Zusammengefasst kann gesagt werden, dass für unterschiedliche Transfektionsexperimente unterschiedliche Blockierungsstrategien erforderlich sind.

Die unterschiedlichen Transfektioneergebnisse zur Blockierung des angeborenen Immunsystems bezüglich einfacher und repetitiver Transfektion lassen sich ebenfalls deuten. Es ist bekannt, dass die Endozytose, also der äußere Membrantransport, stark durch Kinasen reguliert ist. Über den schnellen Weg der Phosphorylierung kann die die Zelle bei der Detektion pathogen assoziierter Muster die Endozytose herunterregulieren und damit ein Enfallstor für Pathogene schliessen. Da die Signaltransdukionskaskaden auch über Kinasen das Signal weiterleiten, ist hier ein Zusammenhang zu vermuten. Deutliche Zuwächse der Transfektionseffizienz schon bei der ersten Transfektion deuten darauf hin, dass schnelle Abwehrprozesse unterbunden wurden, also vermutlich auch der Memrantransport. Abwehrprozesse, die über die Expression spezielle Proteine oder Zytokine laufen, dauern erheblich länger und sind dabei in einem Zeitschema erst bei einer Doppeltransfektion wirksam. Eine Steigerung der Transfektionseffizienz nach einem Tag durch eine repetitive Transfektion deutet auf eine reduzierte Expression eines oder mehrerer für den Abwehrprozess wichtigen Proteine hin.

Nur im Falle der Transfektion von siRNA lässt sich mit einer Aktivierung von TLRs eine Verbesserung des Transfektionesergebnisses erzielen, da hier die benötigte RNAi-Maschinerie Bestandteil des angeborenen Immunsystems ist. In diesem Fall kann allerdings auch eine Blockierung verschiedener Signaltransduktionswege (Beispiel U0126) vorteilhaft sein.

Wie bereits ausgeführt, kann die Erfindung zu Therapiezwecken eingesetzt werden. Insbesondere kann die Erfindung für die Gentherapie von zum Beispiel Cystischer Fibrose, Muskeldystrophie, Phenylketonurie, Ahornsirupkrankheit, Propionazidämie, Methylmalonazidämie, Adenosindeaminasemangel, Hypercholesterinämie, Hämophilie, β-Thalassämie und Krebs genutzt werden. Gentherapeutische Behandlungsmethoden sind weiters interessant, wenn Hormone, Wachstumsfaktoren, Cytotoxine oder immunomodulierend wirkende Proteine im Organismus synthetisiert werden sollen. Für die oben genannten Zwecke können DNA-Fragmente mittels der Erfindung effektiv in Zellen gebracht werden, in denen diese DNA die gewünschte Wirkung entfalten kann ohne dabei zu unerwünschten Nebenwirkungen zu führen. Die gewünschte Wirkung kann der Ersatz fehlender oder defekter DNA-Bereiche oder die Inhibition von DNA-Bereichen (zum Beispiel durch Antisense-DNA / RNA oder siRNA), die die Erkrankung auslösen, im erkrankten Zelltyp sein. Auf diese Weise können z.B. tumorunterdrückende Gene in der Krebs-Therapie eingesetzt werden oder durch die Einführung cholesterolregulierender Gene ein Beitrag zur Vorbeugung von Herz- und Blutgefäßkrankheiten geleistet werden. Weiters kann DNA, welche Ribozyme, siRNA oder shRNA kodiert, oder Ribozyme oder siRNA selbst in erkrankte Zellen eingeschleust werden. Die Translation der DNA erzeugt aktive Ribozyme oder siRNA, die an spezifischen Stellen m-RNA katalytisch spalten und auf diese Weise die Transkription verhindern. Auf diese Weise kann zum Beispiel virale m-RNA gespalten werden, ohne eine andere zelluläre m-RNA in Mitleidenschaft zu ziehen. Der Vermehrungszyklus von Viren (HIV, Herpes, Hepatitis B und C, respiratorisches Syncytial Virus) kann auf diesem Wege unterbrochen werden. Weitere Erkrankungen, die speziell über die Behandlung mit siRNA geheilt werden sollen sind die altersbedingte Degeneration der Macula (Augenerkrankung), Leberkrebs, solide Tumoren, Amyotropische Lateral-Sklerose und Entzündungserkrankungen. Auch in der Krebstherapie spielt Transfektion beispielsweise zur Herstellung von Krebsvakzinen eine immer größer werdende Rolle. Damit ist jene auch mögliches Anwendungsgebiet für die Erfindung.
Eine weitere Anwendung kann die Erfindung zum Beispiel in Impfverfahren finden, die auf der Basis der Expression von DNA, welche immunogene Peptide kodiert, im Körper von Mensch und Tier funktionieren. Dazu werden z.B. Lipid / DNA-Komplexe als Impfstoffe benutzt. Die Einschleusung der DNA in die Körperzellen führt zur Expression des immunogenen Peptids und löst somit die adaptive Immunantwort aus.

Im folgenden werden erfindungsgemäß beispielhafte, aber keinesfalls beschränkende Definitionen aufgeführt:

### Transfektion:

Einschleusen von genetischem Material in eine eukariotische Zelle.

### Transfektionsergebnis/Transfektionseffizienz

Menge einer Proteinexpression einer Zellpopulation in Folge von Transfektionsprozessen mit genetischem Material, welches unter anderem dieses exprimierte Protein codiert oder Ausmaß eines Knock-downs einer Proteinexpression einer Zellpopulation in Folge von Transfektionsprozessen mit genetischem Material, welches einen solchen Knock-Down auslösen kann, insbesondere siRNA oder Ribozyme oder DNA, die für shRNA oder Ribozyme codiert oder Anteil der Zellen einer Gesamtpopulation von Zellen, die die biologische Wirksamkeit des eingeschleusten genetischen Materials in Folge von Transfektionsprozessen zeigt. Gleichzeitig soll der physiologische Status der Zellpopulation so wenig wie möglich beeinflusst werden, das heisst das Protein-Expressionsprofil der Zellpopulation soll sich idealerweise nur bezüglich der Proteine ändern, deren Gene in die Zelle eingeschleust wurden oder deren Expression durch das eingeschleuste genetisches Material herabgesetzt oder verhindert werden soll.

### Nicht-virales Genliefersystem:

Nicht-virale Genliefersysteme werden nicht durch Rekombination genetischen Materials von natürlich vorkommenden Viren erzeugt. Sie sind in der Lage genetisches Material in eukariotische Zellen einzuschleusen. Insbesondere handelt es sich bei den nicht-viralen Genliefersystemen um physikalische Methoden und chemische Methoden. Physikalische Methoden lokalisieren mindestens das genetische Material in der Nähe der Zelle, insbesondere nutzen physikalische Verfahren jedoch Energiezufuhr insbesondere in Form von thermischer, kinetischer, elektrischer oder sonstiger Energie um einen Transport des genetischen Materials durch die Zellmembran zu vermitteln. Chemische Methoden beruhen entweder auf einer chemischen Veränderung oder Derivatisierung der Nukleinsäuren, die sie insbesondere zellgängig machen oder bestehen insbesondere aus Stoffen, die DNA binden und einen Transport durch die Zellmembran vermitteln können. Insbesondere nutzen diese zur Bindung der Nukleinsäuren elektrostatische Kräfte oder Wasserstoffbrückenbindungen. Wiederum insbesondere geschieht der Transport der DNA durch die Zellmembran durch einen aktiven Transportmechanismus der Zelle, der Endozytose. Stoffe, die diese Eigenschaften aufweisen enthalten insbesondere kationische Lipide, kationische Polymere, kationische Peptide oder auch Moleküle die eine Domäne haben, die DNA oder RNA binden kann und zugleich eine zweite Domäne haben, die eine Liganden enthält, der von einem Rezeptor auch der Zelloberfläche erkannt wird und durch diesen Erkennungsprozess Endozytose auslöst. Eine zweite Möglichkeit besteht darin, daß dieser Ligand in der Lage ist einen Membrantransfer auszulösen, d.h. einen Transport auf die andere Seite der Membran vermitteln. Die Stoffe können auch besonders formuliert sein, insbesondere als Micellen oder Liposomen und auch aus mehreren Komponenten insbesondere mit unterschiedlicher Funktion bestehen.

### Gentherapie

Therapie zur Heilung oder Linderung von Krankheiten bei der als Wirkstoff modifizierte oder unmodifizierte Nukleinsäuren eingesetzt werden.

### Angeborene Immunabwehr

Die angeborene Immunabwehr grenzt sich von der erworbenen oder adaptiven Immunabwehr dahingehen ab, dass sie einen Erreger abwehrt, ohne dass es vorher jemals zu einem Kontakt mit dem Erreger gekommen sein muss, um das Immunsystem zu schulen. Die angeborene Immunabwehr ist den meisten Zelltypen zu eigen und besteht aus zwei Teilen, dem intrazellulären und dem interzellulären Anteil. Der intrazelluläre Anteil nutzt die Erkennung von Pathogenen zuzuordnenden molekularen Strukturen durch Rezeptoren. Diese Rezeptoren stoßen insbesondere Signaltransduktionskaskaden an, die insbesondere durch Expression vieler zelleigener Gene und Phosphorylierung wichtiger Proteine in einem geänderten physiologischen Status (zB. "antiviralen Status") der direkt betroffenen Zellen münden. Zusätzlich werden Zytokine ausgeschüttet.

Über den interzellulären Anteil des angeborenen Immunsystems benachrichtigen betroffene Zellen über diese Botenstoffe (Zytokine) nichtbetroffene Zellen und lösen auch dort einen geänderten physiologischen Status (zB. "Antiviralen Status") aus. Dabei docken die Zytokine an Zytokin-Rezeptoren der anderen Zellen an und lösen wiederum eine Signaltransduktionskaskade aus. Der interzelluläre Anteil grenzt sich also vom intrazellulären Anteil durch die unterschiedlichen Rezeptoren (Zytokinrezeptoren statt PRRs (pattern recognition receptors) und Agonisten (Zytokine statt pathogene Muster) ab.

### Antiviraler Status

Status von Zellen, der sich dadurch auszeichnet, dass die Zelle versucht die mögliche biologische Wirksamkeit von fremdem genetischen Material durch Gegenmaßnahmen zu unterbinden.

### Transfektion in vivo

Das Einschleusen von genetischem Material in eukariotische Zellen findet in einem lebenden Organismus statt.

### Transfektion in vitro

Das Einschleusen von genetischem Material in eukariotische Zellen findet außerhalb eines lebenden Organismus statt, insbesondere in Gefäßen, die sich zur Kultivierung von eukariotischen Zellen eignen.

### Genetisches Material

Nukleinsäuren, insbesondere Ribonukleinsäuren oder Desoxyribonukleinsäuren, die insbesondere aus zwei wenigstens teilweise komplementären Strängen (doppelsträngig = ds) bestehen z.B. dsDNA und dsRNA, oder die insbesondere aus einem Strang (einzelsträngig = ss) besteht, z.B. ssDNA und ssRNA, der teilweise komplentäre Bereiche aufweisen kann, die über Wasserstoffbrückenbindung miteinander verbunden sein können. Das genetische Material dient im Falle von DNA zur Erzeugung von RNA und/oder Proteinen. Im Falle von ssRNA dient es zur Erzeugung von Proteinen. Im Falle von dsRNA dient das genetische Material dazu einen Knockdown eines Gens durch RNA-Interferrenz zu erreichen.

### Modifiziertes genetisches Material

Natürliche Nukleinsäuren, die durch Modifikation in ihren Eigenschaften verändert wurden. Dabei können diese Modifikationen insbesondere chemische Veränderungen sein, die insbesondere das Phosphatgerüst, die Zucker oder Basen betreffen, was insbesondere die Stabilität der Nukleinsäuren gegen Nukleasen und Ribonukleasen erhöhen soll. Weiters können Moleküle (Labels) an die Nukleinsäuren kovalent oder nicht-kovalent angeheftet werden, die zu neuen Eigenschaften der Nukleinsäuren führen, insbesondere zu optischer Verfolgbarkeit durch Fluoreszenzlabels oder Labels, die die Nukleinsäuren zu einen bestimmen Ort in der Zelle dirigieren (Lokalisationselemente) oder Labels, die den Durchtritt von Nukleinsäuren durch Membranen vermitteln und so Nukleinsäuren beispielsweise zellgängig machen.
Ein Beispiel für eine Modifikation ist der Austausch von Sauerstoff gegen Schwefel im Phosphatgerüst, die Methylierung von 2'-OH Gruppen der Ribose im Falle von RNA, oder die vollständige Subtitition von 2'OH Gruppen durch Fluor, um die Stabilität gegen Nukleasen zu erhöhen. Ein weiteres Beipiel ist das Anheften von FITC (fluorescein isothiocyanate) als Fluoreszenzlabel, um den Weg des genetischen Materials in der Zelle mikroskopisch verfolgen zu können oder das Anheften von sogenannten NLS (Nuclear localisation signals, zB. PKKKRKVG) um einen Transport in den Zellkern zu erreichen.

### siRNA (short interfering RNA):

Kurze dsRNA (bis 28 bp) die durch RNA-Interferenz den Knock-Down eines Proteins bewirken kann.

### shRNA (short hairpin RNA):

Kurze ssRNA, die am 3'-Ende und am 5'-Ende komplementäre Bereiche besitzt und dadurch über Wasserstoffbrückenbindung hybridisieren und eine Haarnadelstruktur ausbilden kann. shRNA kann durch RNA-Interferenz den Knock-Down eines Proteins bewirken.

### Rezeptor:

Molekül, das in der Lage ist einen Stoff (Agonist) zu detektieren und damit eine biologische Reaktion auslöst, Insbesondere handelt es sich bei Rezeptoren um Proteine.

### Blockierung:

Verhinderung der biologischen Funktion, insbesondere von Proteinen.

### Unterdrückung:

Unterbrechung des Kommunikationsnetzes der Immunabwehr, d.h. von einer oder mehreren eine Immunantwort auslösenden Signaltransduktionskaskade(n), insbesondere der angeborenen intra- und/oder interzellulären Immunabwehr. Durch diese Unterbrechung wird die Immunabwehr geschwächt, wodurch sich die Immunantwort verringert.

### DNA/RNA-bindende Domäne:

Bereich in einem Molekül das DNA kovalent oder über nicht kovalente Wechselwirkungen gebunden trägt, insbesondere sind die nicht kovalenten Wechselwirkungen elektrostatische Kräfte und Wasserstoffbrückenbindungen.

### Signaltransduktionskaskade

Ausgehend von einem Rezeptor, der die Anwesenheit eines Stoffes durch Anlagerung dieses Stoffes an den Rezeptor detektiert, wird die Information über die Anwesenheit dieses Stoffes in ein Signal umgewandelt und über eine Kette von Molekülen durch Signaltransduktion weitergetragen. Am Ende steht eine biologische Reaktion. Insbesondere wird das durch den Rezeptor erzeugte Signal von Adaptermolekülen aufgenommen und insbesondere über Kinasen insbesondere an Transkriptionsfaktoren weitergetragen. Die Transkriptionsfaktoren stimulieren die Expression von Genen, die die biologische Reaktion vermitteln.

### Knock-down

Abschwächung oder Ausschaltung der Translation einer mRNA zu einem Protein bei der Proteinbiosynthese.

### Antikörper

Moleküle, insbesondere Proteine, die in der Lage sind molekulare Strukturen, insbesondere andere Proteine, zu erkennen und durch Bindung dessen biologische Wirkung beeinflussen. Im erfindungsgemäßen Zusammenhang handelt es sich um blockierende Antikörper, d.h. im Falle der Rezeptoren des angeborenen Immunsystems wird die Signalweiterleitung verringert oder unterbunden. Die Antikörper können polyklonal oder monoklonal sein. Die Antikörper können humanisiert sein. In der Regel müssen Sie gegen den Rezeptor einer Spezies gerichtet sein, dessen Zellen transfioziert werden sollen. Aufgrund der Ähnlichkeit der der Rezeptoren, kann es aber auch vorkommen, daß ein Antikörper kreuzreaktiv ist. Weiters können die Antikörper modifiziert sein, d.h. es können zb. Molekülteile (zB. Fc-Fragmente, sodaß nur noch Fab Fragmente verbleiben) abgespalten sein, sofern damit die biologische Wirkung nicht wesentlich verringert wird.
Weiters können zusätzlich Molekülteile an dem Antikörper angebracht werden, die dem Antikörper zusätzliche Eigenschaften verleihen, sofern damit die biologische Wirkung nicht wesentlich verringert wird.
Ein Beispiel ist das Anbringen von Fluoreszenzlabels oder das Anbringen von hydrophoben Resten (zB. Phospholipide, A. Schnyder et al.; J. Am. Soc. for Exp. NeuroTherapeutics; 2005; 2; 99-107) um eine Verankerung in Membranen und/oder Liposomen zu erleichtern.

### Intrabodies

Intrazellular exprimierte Antikörper.

### Aptamere

RNA-Moleküle, die durch Ausbildung einer Tertiärstruktur in der Lage sind molekulare Strukturen, insbesondere Proteine, ähnlich einem Antikörper zu erkennen und durch Bindung dessen biologische Wirkung beeinflussen. Aptamere können aus modifizierter oder unmodifizierter RNA oder DNA bestehen.

### Antagonist

Substanz, die einen Agonisten durch Blockierung eines entsprechenden Rezeptors in seiner Wirkung hemmt, ohne selbst einen Effekt auszulösen.

### Agonist

Ein Agonist ist in der Lage durch Bindung an einen Rezeptor eine biologische Wirkung zu erzielen.

### Antivirale Zytokine

Zytokine, die bei einer Infektion einer eukariotischen Zelle mit Viren exprimiert werden. Beispiele sind Interferon alpha, Interferon beta, Interferon gamma, TNF alpha, TFN beta, Interleukin 6,8,15,28 und 29.

### Inhibitoren

Moleküle, die die biologische Wirkung eines anderen Moleküls, insbesondere von Proteinen inhibieren können. Insbesondere sind die Inhibitoren selbst Proteine, modifizierte oder unmodifizierte Nukleinsäuren oder kleine organische Moleküle.

### TLRs, RIG-I-Helikase, RIG-I-like Helikase

Rezeptoren die speziesübergreifend in Gruppen nach ihrer Funktion zusammengefasst werden.

### Adaptermoleküle

Moleküle die ein Signal von Rezeptoren übernehmen und die speziesübergreifend in Gruppen nach ihrer Funktion zusammengefasst werden.

### Abkürzungen

AP-1 = Activated protein-1
ERK = Extracellular-signal Regulated Kinase
IkB = Inhibitory-binding protein kB
IKK = IkappaBKinase = inhibitory-binding protein κB kinase
IKKa = Ikkalpha= I Kappa Kalpha = IKK1
IKKb = IKKbeta = I Kappa Kbeta = IKK2
IKKd = IKKdelta = I Kappa Kdelta
IKKe = IKKepsilon = I Kappa Kepsilon
IKKg = IKK gamma = I Kappa Kgamma= IKK3
IKKi = IKK epsilon
IRAK1 = Interleukin 1 Receptor-Associated Kinase 1
IRAK4 = interleukin-1 receptor-associated kinase 4
IRF = Interferon regulating Factor
JNK = c-Jun N-terminal Kinase
JAK = Janus activated Kinaste
Mal = TIRAP= MyD88-adapter-like
MAPK = Mitogen activated Protein Kinase
MEK = MAPK/ERK kinase
MKK = MAP Kinase Kinase
MKK = Mitogen-activated protein kinase kinase
MSK = Mitogen and stress activated kinase
MyD88 = Myeloid differentiation factor 88
NAP1 = Nck-associated protein 1
NEMO = IKK gamma
NF-kB = Nuclear Factor kappaB
P1 3K = Phosphoinositol-3-Kinase
PKR = Protein Kinase R = Protein Kinase RNA-activated
PKB = Protein Kinase B
PDK1 = Phosphoinositide-dependend Protein Kinase 1
PDK2 = Phosphoinositide-dependend Protein Kinase 1
Rac1 = Ras-related C3 botulinum toxin substrate 1
RIP1 = Receptor-interacting protein 1
STAT = Signal Transducers and Activators of Transcription
TAK1= Transforming growth factor-β-activated kinase
TBK1 = IKKd= TANK-binding Kinase
TIRAP = Mal =Toll-interleukin 1 receptor (TIR) domain-containing adapter protein
TRAF3 = TNF receptor-associated factor 3
TRAF6 = TNF receptor-associated factor 6
TRAM = TRIF-related adaptor molecule
TRIF = Toll/IL-1 receptor domain-containing adaptor inducing interferon-b adaptor protein

Bevorzugte Ausführungsformen
1. Zusammensetzung für eine Transfektion, umfassend:
   a) ein nicht-virales Genliefersystem, wobei das nicht-virale Genliefersystem
      (i) ein kationisches Lipid, ein kationisches Polymer, oder ein kationisches Protein umfasst; und/oder
      (ii) eine Verbindung umfasst, die eine DNA und/oder RNA-bindende Domäne aufweist und rezeptorvermittelte Endozytose oder einen Membrantransfer auslösen kann; und/oder
      (iii) eine Verbindung umfasst, die kovalent an DNA und/oder RNA gebunden ist und rezeptorvermittelte Endozytose oder einen Membrantransfer auslösen kann; und
   b) ein Mittel zur zumindest teilweisen Unterdrückung und/oder Aktivierung der angeborenen intrazellulären und/oder interzellulären lmmunabwehr, ausgewählt aus:
      (i) einem Antikörper gegen TLR 1, TLR 2, TLR 3, TLR 4, TLR 5, TLR 6, TLR 7, TLR 8, TLR 9, TLR 10, TLR 11,TLR 12, oder TLR 13;
      (ii) einem Antikörper gegen einen Zytokinrezeptor oder einem Bevorzupte Ausfulsungsformen Zytokinrezeptor-Antagonisten;
      (iii) einem Inhibitor der Kinase MEK1 und/oder MEK2;
      (iv) einem Agonisten für TLR7 und/oder TLR8, ausgewählt aus der Gruppe, umfassend Bropirimine (2-amino-5-bromo-6-phenyl-4-pyrimidinone), Imidazochinoline, Thiazolochinoline, und Guanosinanaloga; und
      (v) einer Kombination derselben.
2. Kit of parts zur Transfektion, umfassend:
   a) ein nicht-virales Genliefersystem, wobei das nicht-virale Genliefersystem
      (i) ein kationisches Lipid, ein kationisches Polymer, oder ein kationisches Protein umfasst; und/oder
      (ii) eine Verbindung umfasst, die eine DNA und/oder RNA-bindende Domäne aufweist und rezeptorvermittelte Endozytose oder einen Membrantransfer auslösen kann; und/oder
      (iii) eine Verbindung umfasst, die kovalent an DNA und/oder RNA gebunden ist und rezeptorvermittelte Endozytose oder einen Membrantransfer auslösen kann; und
   b) ein Mittel zur zumindest teilweisen Unterdrückung und/oder Aktivierung der angeborenen intrazellulären und/oder interzellulären lmmunabwehr, ausgewählt aus:
      (i) einem Antikörper gegen TLR 1, TLR 2, TLR 3, TLR 4, TLR 5, TLR 6, TLR 7, TLR 8, TLR 9, TLR 10, TLR 11,TLR 12, oder TLR 13;
      (ii) einem Antikörper gegen einen Zytokinrezeptor oder einem Zytokinrezeptor-Antagonisten;
      (iii) einem Inhibitor der Kinase MEK1 und/oder MEK2;
      (iv) einem Agonisten für TLR7 und/oder TLR8, ausgewählt aus der Gruppe, umfassend Bropirimine (2-amino-5-bromo-6-phenyl-4-pyrimidinone), Imidazochinoline, Thiazolochinoline, und Guanosinanaloga; und
      (v) einer Kombination derselben.
3. Zusammensetzung oder Kit of parts nach Ausführungsform 1 oder Ausführungsform 2, dadurch gekennzeichnet, dass das nicht-virale Genliefersystem ein kationisches Lipid umfasst.
4. Zusammensetzung oder Kit of parts nach einer der Ausführungsform en bis 3, dadurch gekennzeichnet, dass das in a) definierte nicht-virale Genliefersystem ein kationisches Lipid mit folgender Formel umfasst: 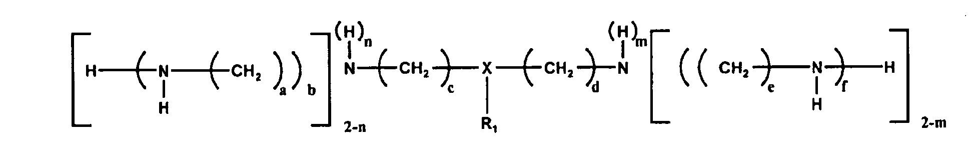 wobei 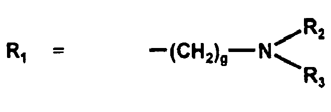 ist, wobei
   R₂ und R₃ unabhängig voneinander Dodecyl, Dodecenyl, Tetradecyl, Tetradecenyl, Hexadecyl, Hexadecenyl, Octadecyl, Octadecenyl oder andere Alkylreste sind, die in allen möglichen Kombinationen gesättigt, ungesättigt, verzweigt, unverzweigt, fluoriert oder nicht fluoriert sind, und aus 5 bis 30 Kohlenstoffatomen aufgebaut sind; 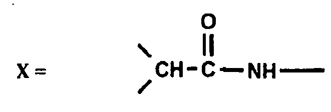 ist; und wobei
   m = 0 und n = 0 ist; oder
   m = 0 und n = 1 ist; oder
   m = 0 und n = 2 ist; oder
   m = 1 und n = 1 ist; oder
   m = 1 und n = 2 ist; oder
   m = 2 und n = 2 ist; und
   g = 1,2,3,4,5,6,7 oder 8 ist;
   a = 0,1,2,3,4,5 oder 6 ist;
   b = 0,1,2,3,4,5 oder 6 ist;
   c = 0,1,2,3,4,5 oder 6 ist;
   d = 0,1,2,3,4,5 oder 6 ist;
   e = 0,1,2,3,4,5 oder 6 ist; und
   f = 0,1,2,3,4,5 oder 6 ist.
5. Zusammensetzung oder Kit of parts nach Ausführungsform 4, dadurch gekennzeichnet, dass R₂ und R₃ unabhängig voneinander Dodecyl, Dodecenyl, Tetradecyl, Tetradecenyl, Hexadecyl, Hexadecenyl, Octadecyl, Octadecenyl sind;
   m und n = 1 sind;
   g = 1,2,3,4,5,6,7 oder 8 ist;
   a = 0,1,2,3,4,5 oder 6 ist;
   b = 0,1,2,3,4, 5 oder 6 ist;
   c = 0,1,2,3,4,5 oder 6 ist;
   d = 0,1,2,3,4,5 oder 6 ist;
   e = 0,1,2,3,4,5 oder 6 ist; und
   f = 0,1,2,3,4,5 oder 6 ist.
6. Zusammensetzung oder Kit of parts nach einer der Ausführungsform en 1 bis 5, enthaltend modifiziertes oder unmodifiziertes genetisches Material, insbesondere modifizierte oder unmodifizierte ssDNA, modifizierte oder unmodifizierte dsDNA, modifizierte oder unmodifizierte ssRNA, modifizierte oder unmodifizierte dsRNS und/oder modifizierte oder unmodifizierte siRNA.
7. Zusammensetzung oder Kit of parts nach mindestens einer der Ausführungsform en bis 6, dadurch gekennzeichnet, dass das Mittel zur zumindest teilweisen Unterdrückung und/oder Aktivierung der angeborenen intrazellulären und/oder interzellulären Immunabwehr 1,4-Diamino-2,3-dicyano-1,4-bis(o-amionophenylmercapto)butadien (U0126); Imiquimod (R837, 1-(2-methylpropyl)-1H-imidazol[4,5-c]quinolin-4-amine); Resiquimod (R848, 4-amino-2-(ethoxymethyl)-a,a-dimethyl-1H-imidazo[4,5-c]quinoline-1-ethanol); Gardiquimod (1-(4-amino-2-ethylamino methylimidazo-[4,5-c]quinolin-1-yl)-2-methylpropan-2-ol); CL075; CL097; Loxoribine (7-allyl-7,8-dihydro-8-oxo-guanosin); Isatoribine (7-Thia-8-oxoguanosin); Bropirimine (2-amino-5-bromo-6-phenyl-4-pyrimidinone); oder irgendeine Kombination derselben ist.
8. Zusammensetzung oder Kit of parts nach mindestens einer der Ausführungsform en bis 7, dadurch gekennzeichnet, dass das Mittel zur zumindest teilweisen Unterdrückung und/oder Aktivierung der angeborenen intrazellulären und/oder interzellulären Immunabwehr ein Antikörper gegen TLR 3, TLR 7, TLR 8, oder TLR 9 ist.
9. Zusammensetzung oder Kit of parts nach mindestens einer der Ausführungsform en1 bis 8, dadurch gekennzeichnet, dass das Mittel zur zumindest teilweisen Unterdrückung und/oder Aktivierung der angeborenen intrazellulären und/oder interzellulären Immunabwehr ein Antikörper oder Antagonist gegen Interleukin-1-Rezeptor, insbesondere IL-ra; lnterferon-Typ-I-Rezeptor; Interferon-gamma-Rezeptor; oder Tumornekrosefaktor-Rezeptor ist.
10. Kit of parts nach einer der Ausführungsform en 2 bis 9, wobei
   (i) alle Komponenten vollständig getrennt voneinander vorliegen;
   (ii) die Komponenten a) und b) getrennt voneinander vorliegen; oder
   (iii) die Komponenten a) und b) gemeinsam vorliegen.
11. Pharmazeutische Zusammensetzung, die eine Zusammensetzung nach einer der Ausführungsform en 1 bis 9 umfasst.
12. Pharmazeutischer Kit of parts, der einen Kit of parts nach einer der Ausführungsform en 2 bis 10 umfasst.
13. Verwendung einer Zusammensetzung oder eines Kit of parts nach einer der Ausführungsform en 1 bis 12 zur Behandlung einer Krankheit durch Gentherapie.
14. Verwendung nach Ausführungsform 13, wobei es sich bei der Krankheit um cystische Fibrose, Muskeldystrophie, Phenylketonurie, Ahornsirupkrankheit, Propionazidämie, Methylmalonazidämie, Adenosindeaminasemangel, Hypercholesterinämie, Hämophilie, β-Thalassämie, Krebs, eine Viruserkrankung, eine Degeneration der Macula, Amyotropische Lateral-Sklerose und/oder eine Entzündungserkrankung handelt.
15. Verfahren zur Verbesserung des Transfektionsergebnisses von nicht-viralen Genliefersystemen, dadurch gekennzeichnet, dass man
   a) die Zellen vor und/oder bei der Transfektion mit mindestens einem Mittel zur zumindest teilweisen Unterdrückung der angeborenen intrazellulären und/oder interzellulären Immunabwehr behandelt und bei der Transfektion genetisches Material, insbesondere modifizierte und/oder unmodifizierte ssDNA, modifizierte und/oder unmodifizierte dsDNA, modifizierte und/oder unmodifizierte ssRNA, modifizierte und/oder unmodifizierte dsRNA und/oder modifizierte und/oder unmodifizierte siRNA, in die Zellen einbringt; oder
   b) die Zellen vor und/oder bei und/oder nach der Transfektion mit mindestens einem Mittel zur zumindest teilweisen Aktivierung der angeborenen intrazellulären und/oder interzellulären Immunabwehr behandelt und bei der Transfektion modifizierte und/oder unmodifizierte siRNA in die Zellen einbringt.
16. Verfahren nach Ausführungsform 15, dadurch gekennzeichnet, dass das nicht-virale Genliefersystem
   (i) ein kationisches Lipid, ein kationisches Polymer, oder ein kationisches Protein umfasst; und/oder
   (ii) eine Verbindung umfasst, die eine DNA und/oder RNA-bindende Domäne aufweist und rezeptorvermittelte Endozytose oder einen Membrantransfer auslösen kann; und/oder
   (iii) eine Verbindung umfasst, die kovalent an DNA und/oder RNA gebunden ist und rezeptorvermittelte Endozytose oder einen Membrantransfer auslösen kann; und/oder
   (iv) auf einer physikalischen Methode wie Elektroporation, Mikroinjektion, Magnetofektion, Ultraschall oder einer ballistischen oder hydrodynamischen Methode beruht.
17. Verfahren nach Ausführungsform 15 oder 16, dadurch gekennzeichnet, dass die Zellen bis zu 4 Tage, vorzugsweise bis zu 18 Stunden, insbesondere bis zu 6 Stunden vor der Transfektion mit mindestens einem Mittel zur zumindest teilweisen Unterdrückung oder Aktivierung der angeborenen intrazellulären und/oder interzellulären Immunabwehr behandelt werden.
18. Verfahren nach einer der Ausführungsformen 15 bis 17, dadurch gekennzeichnet, dass die Zellen gleichzeitig mit dem mindestens einen Mittel zur zumindest teilweisen Unterdrückung oder Aktivierung der angeborenen intrazellulären und/oder interzellulären Immunabwehr behandelt werden und mit dem nicht-viralen Genliefersystem in Kontakt gebracht werden.
19. Verfahren nach einer der Ausführungsform en 15 bis 18, dadurch gekennzeichnet, dass das Mittel zur zumindest teilweisen Unterdrückung der angeborenen intrazellulären und/oder interzellulären Immunabwehr einen Antikörper, Intrabody, Aptamer, Antagonisten, Inhibitor und/oder eine siRNA umfasst.
20. Verfahren nach mindestens einer der Ausführungsform en 5 bis 19, dadurch gekennzeichnet, dass durch Knock-down mit siRNA zumindest ein Gen ausgeschaltet wird, das für ein zur Signaltransduktion über TLR notwendiges Protein codiert.
21. Verfahren nach mindestens einer der Ausführungsform en 15 bis 20, dadurch gekennzeichnet, dass die angeborene intrazelluläre und/oder interzelluläre Immunabwehr durch die Blockierung von mindestens einem aus der Gruppe TLR 1, TLR 2, TLR 3, TLR 4, TLR 5, TLR 6, TLR 7, TLR 8, TLR 9, TLR 10, TLR 11,TLR 12, TLR 13, CD14, CD38, RIG-1 Helikase und RIG-I-like Helikase, insbesondere durch die Blockierung von mindestens einem aus der Gruppe TLR 1, TLR 2, TLR 4 und TLR 9, zumindest teilweise unterdrückt wird.
22. Verfahren nach mindestens einer der Ausführungsform en 15 bis 21, dadurch gekennzeichnet, dass die angeborene intrazelluläre und/oder interzelluläre Immunabwehr durch die Blockierung von mindestens einer Kinase aus der Gruppe MEK1 und MEK2 zumindest teilweise unterdrückt wird.
23. Verfahren nach Ausführungsform 22, dadurch gekennzeichnet, dass die Kinase(n) MEK1 und/oder MEK2 durch 1,4-Diamino-2,3-dicyano-1,4-bis(o-amionophenylmercapto)butadien (U0126) blockiert wird/werden.
24. Verfahren nach mindestens einer der Ausführungsform en 15 bis 23, dadurch gekennzeichnet, dass die angeborene interzelluläre Immunabwehr durch die Blockierung von mindestens einem Zytokin, einem Tumornekrosefaktor, einem Interleukin, oder einem Interferon zumindest teilweise unterdrückt wird.
25. Verfahren nach Ausführungsform 24. dadurch gekennzeichnet, dass ein Interferon des Typs I, insbesondere ein Interferon aus der Gruppe Interferon-alpha, Interferon-beta, Interferon-gamma und Interferon-omega, blockiert wird.
26. Verfahren nach mindestens einer der Ausführungsform en 15 bis 25, dadurch gekennzeichnet, dass die angeborene interzelluläre lmmunabwehr durch die Blockierung von mindestens einem Rezeptor aus der Gruppe der Zytokinrezeptoren, Interferonrezeptoren, insbesondere Rezeptoren für Interferone des Typs I, Interleukinrezeptoren und Tumornekrosefaktorrezeptoren zumindest teilweise unterdrückt wird.
27. Verfahren nach mindestens einer der Ausführungsform en 15 bis 18, dadurch gekennzeichnet, dass das Mittel zur Aktivierung der angeborenen Immunabwehr ein Agonist ist.
28. Verfahren nach mindestens einer der Ausführungsform en bis 18 und 27, dadurch gekennzeichnet, dass die angeborene intrazelluläre und/oder interzelluläre Immunabwehr durch mindestens einen Agonisten für einen TL-Rezeptor, insbesondere durch mindestens einen Agonisten für TLR7 und/oder TLR8, zumindest teilweise aktiviert wird.
29. Verfahren nach Ausführungsform 28, dadurch gekennzeichnet, dass der mindestens eine Agonist für TLR7 und/oder TLR8 ausgewählt wird aus der Gruppe, umfassend Bropirimine (2-amino-5-bromo-6-phenyl-4-pyrimidinone), Imidazochinoline, Thiazolochinoline, Guanosinanaloga und ssRNA.
30. Verfahren nach Ausführungsform 29, dadurch gekennzeichnet, dass der mindestens eine Agonist
   (i) Imiquimod (R837, 1-(2-methylpropyl)-1H-imidazol[4,5-c]quinolin-4-amine), Resiquimod (R848, 4-amino-2-(ethoxymethyl)-a,a-dimethyl-1H-imidazo[4,5-c]quinoline-1-ethanol) oder Gardiquimod (1-(4-amino-2-ethylamino methylimidazo-[4,5-c]quinolin-1-yl)-2-methylpropan-2-ol) ist; oder
   (ii) CL075 oder CL097 ist; oder
   (iii) Loxoribine (7-allyl-7,8-dihydro-8-oxo-guanosin) oder Isatoribine (7-Thia-8-oxoguanosin) ist; oder
   (iv) ssRNA mit U und/oder GU reichen Sequenzen ist, insbesondere ssRNA mit den Sequenzmotiven UGUGU und/oder GUCCUUCAA.
31. Verfahren nach mindestens einer der Ausführungsform en 15 bis 18 sowie 27 bis 30, dadurch gekennzeichnet, dass die angeborene interzelluläre Immunabwehr durch mindestens einen Agonisten für Rezeptoren von antiviralen Zytokinen, insbesondere durch Interferon-beta oder Interferon gamma, zumindest teilweise aktiviert wird.
32. Verfahren nach mindestens einer der Ausführungsform en 15, 16 sowie 27 bis 31, dadurch gekennzeichnet, dass die Zellen bis zu 2 Tage, vorzugsweise bis zu 12 Stunden, insbesondere bis zu 6 Stunden nach der Transfektion mit dem mindestens einen Mittel zur zumindest teilweisen Aktivierung der angeborenen intrazellulären und/oder interzellulären Immunabwehr behandelt werden.
33. Verfahren nach mindestens einer der Ausführungsform en 15 bis 32, dadurch gekennzeichnet, dass das nicht-virale Genliefersystem ein kationisches Lipid gemäß einer der Ausführungsform en 4 oder 5 umfasst

## Patentansprüche

1. Verfahren zur Verbesserung des Transfektionsergebnisses von nicht-viralen Genliefersystemen, **dadurch gekennzeichnet, dass** man
die Zellen vor und/oder bei der Transfektion mit mindestens einem Mittel zur zumindest teilweisen Unterdrückung der angeborenen intrazellulären und/oder interzellulären Immunabwehr behandelt und bei der Transfektion genetisches Material, insbesondere modifizierte und/oder unmodifizierte ssDNA, modifizierte und/oder unmodifizierte dsDNA, modifizierte und/oder unmodifizierte ssRNA, modifizierte und/oder unmodifizierte dsRNA, in die Zellen einbringt, wobei
die angeborene intrazelluläre und/oder interzelluläre Immunabwehr der zu transfizierenden Zellen durch die Blockierung von mindestens einem Zytokin, einem Tumornekrosefaktor, einem Interleukin, oder einem Interferon zumindest teilweise unterdrückt wird.

2. Verfahren nach Anspruch 1, **dadurch gekennzeichnet, dass** ein Interferon des Typs I, insbesondere ein Interferon aus der Gruppe Interferon-alpha, Interferon-beta, Interferon-gamma und Interferon-omega, blockiert wird.

3. Verfahren nach Anspruch 1 oder Anspruch 2, **dadurch gekennzeichnet, dass** die angeborene interzelluläre Immunabwehr durch die Blockierung von mindestens einem Rezeptor aus der Gruppe der Zytokinrezeptoren, Interferonrezeptoren, insbesondere Rezeptoren für Interferone des Typs I, Interleukinrezeptoren und Tumornekrosefaktorrezeptoren zumindest teilweise unterdrückt wird.

4. Verfahren nach einem der Ansprüche 1 bis 3, **dadurch gekennzeichnet, dass** das nicht-virale Genliefersystem
(i) ein kationisches Lipid, ein kationisches Polymer, oder ein kationisches Protein umfasst; und/oder
(ii) eine Verbindung umfasst, die eine DNA und/oder RNA-bindende Domäne aufweist und rezeptorvermittelte Endozytose oder einen Membrantransfer auslösen kann; und/oder
(iii) eine Verbindung umfasst, die kovalent an DNA und/oder RNA gebunden ist und rezeptorvermittelte Endozytose oder einen Membrantransfer auslösen kann; und/oder
(iv) auf einer physikalischen Methode wie Elektroporation, Mikroinjektion, Magnetofektion, Ultraschall oder einer ballistischen oder hydrodynamischen Methode beruht.

5. Verfahren nach einem der Ansprüche 1 bis 4, **dadurch gekennzeichnet, dass** das Mittel zur zumindest teilweisen Unterdrückung der angeborenen intrazellulären und/oder interzellulären Immunabwehr einen Antikörper, Intrabody, Aptamer, Antagonisten, Inhibitor und/oder eine siRNA umfasst.

6. Verfahren nach einem der Ansprüche 1 bis 5, **dadurch gekennzeichnet, dass** das nicht-virale Genliefersystem ein kationisches Lipid mit folgender Formel umfasst: 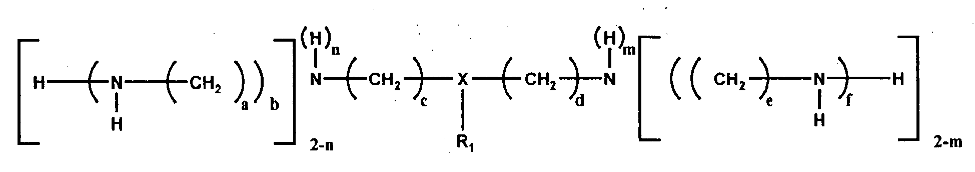 wobei 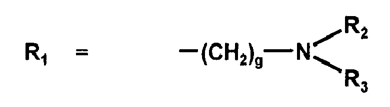 ist, wobei
R₂ und R₃ unabhängig voneinander Dodecyl, Dodecenyl, Tetradecyl, Tetradecenyl, Hexadecyl, Hexadecenyl, Octadecyl, Octadecenyl oder andere Alkylreste sind, die in allen möglichen Kombinationen gesättigt, ungesättigt, verzweigt, unverzweigt, fluoriert oder nicht fluoriert sind, und aus 5 bis 30 Kohlenstoffatomen aufgebaut sind; 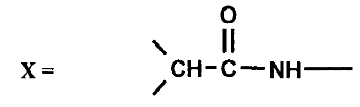 ist; und wobei
m = 0 und n = 0 ist; oder
m = 0 und n = 1 ist; oder
m = 0 und n = 2 ist; oder
m = 1 und n = 1 ist; oder
m = 1 und n = 2 ist; oder
m = 2 und n = 2 ist; und
g = 1,2,3,4,5,6,7 oder 8 ist;
a = 0,1,2,3,4,5 oder 6 ist;
b = 0,1,2,3,4,5 oder 6 ist;
c = 0,1,2,3,4,5 oder 6 ist;
d = 0,1,2,3,4,5 oder 6 ist;
e = 0,1,2,3,4,5 oder 6 ist; und
f = 0,1,2,3,4,5 oder 6 ist.

7. Verfahren nach Anspruch 6, **dadurch gekennzeichnet, dass** R₂ und R₃ unabhängig voneinander Dodecyl, Dodecenyl, Tetradecyl, Tetradecenyl, Hexadecyl, Hexadecenyl, Octadecyl, Octadecenyl sind;
m und n = 1 sind;
g = 1,2,3,4,5,6,7 oder 8 ist;
a = 0,1,2,3,4,5 oder 6 ist;
b = 0,1,2,3,4,5 oder 6 ist;
c = 0,1,2,3,4,5 oder 6 ist;
d = 0,1,2,3,4,5 oder 6 ist;
e = 0,1,2,3,4,5 oder 6 ist; und
f = 0,1,2,3,4,5 oder 6 ist.
